# EUROPEAN PATENT APPLICATION

(11) **EP 4 389 251 A1**
(43) Date of publication of application: **26.06.2024**
(21) Application number: 22216598.7
(22) Date of filing: 23.12.2022
(51) Int. Cl.: B01D 15/20, B01D 15/36, C07H 1/00, C12N 1/00, G01N 30/96, C08B 37/00

(54) **MEANS AND METHODS TO PURIFY AND/OR QUANTIFY FUCOIDAN**

(71) Applicant: Max-Planck-Gesellschaft zur Förderung der Wissenschaften e.V., 80539 München (DE); Universität Bremen, 28359 Bremen (DE)
(72) Inventor: The designation of the inventor has not yet been filed
(74) Representative: Vossius & Partner Patentanwälte Rechtsanwälte mbB

(57) **Abstract**

The present invention relates to methods for purifying and/or quantifying fucoidan from a sample, e.g. a sample of seawater. In particular, the invention provides a method for purifying fucoidan via an anion exchange chromatography column and/or via precipitation of fucoidan. In addition, the invention provides methods for quantifying fucoidan in a sample, e.g. a sample of seawater or a purified sample by correlating fucoidan specific monosaccharides, such as fucose, obtained via a highly specific enzymatic digestion and/or acid hydrolysis, to the fucoidan concentration in the initial/input sample, e.g. sample of seawater or purified sample.

## Description

The present invention relates to methods for purifying and/or quantifying fucoidan from a sample, e.g. a sample of seawater. In particular, the invention provides a method for purifying fucoidan via an anion exchange chromatography column and/or via precipitation of fucoidan. In addition, the invention provides methods for quantifying fucoidan in a sample, e.g. a sample of seawater or a purified sample by correlating fucoidan specific monosaccharides, such as fucose, obtained via a highly specific enzymatic digestion and/or acid hydrolysis, to the fucoidan concentration in the initial/input sample, e.g. sample of seawater or purified sample.

Brown algae annually convert gigatons of carbon dioxide into carbohydrates, including the complex extracellular matrix polysaccharide fucoidan. Due to its persistence in the environment, fucoidan is potentially a pathway for marine carbon sequestration.

Numerous publications describe the extraction of fucoidan from brown algae and animal tissue (e.g. 96), and two publications and one patent application the extraction of fucoidan from microalgae (101, 99, WO 2019/211442 A1). Various patents claim specific techniques for the purification of fucoidan from biomass (e.g. EP2121767B1, EP2532686B1, US8748595B2, US9234051B2). The described state-of-the-art aims at maximizing yields of purified fucoidan obtained from biomass. Therefore, biomass is homogenized and either brought into suspension to solubilize fucoidan for subsequent purification or fucoidan extracted from dry, homogenized biomass.

Compounds in seawater disperse and thereby often become too dilute for analytical quantification. Seawater typically contains only 40-100 µM organic carbon in millions of different compounds (98). Low concentrations and the complex matrix of salts and other organic compounds complicate the quantification of specific compounds (100, 97). Following organic compounds starting at the site of release from their source through dispersal by advection and diffusion, however, requires specific quantification. Established techniques for fucoidan extraction from biomass are not feasible to quantify fucoidan dissolved in seawater. Measurements of bulk organic carbon for example can only provide a cumulative account of particulate or dissolved organic carbon, masking any molecular differences. Quantification of bulk carbohydrates or quantification of individual free monosaccharides and monosaccharides contained in oligo- and polysaccharides lacks molecular resolution and structural identification of actually present biomolecules, severely impacting the ability to determine sources and trajectories of said monosaccharides. Fluorescence has been used to detect seaweed phenols but is unable to establish an unequivocal fluorescent signature for seaweed compounds. Without available methods for dissolved polysaccharide quantification, fucoidan concentrations in seawater and the rates or total amount of fucoidan secretion by brown algae remain unknown (35).

Thus, there is a need to provide means and methods for the purification and/or quantification of fucoidan from seawater. The technical problem underlying the present invention is the purification and/or quantification of fucoidan from seawater.

The technical problem is solved by the provision of the embodiments characterized in the claims and as provided herein below. Specifically, the technical problem is solved, and the above-mentioned difficulties are overcome by the provision of a method for purifying and/or quantifying fucoidan.

The inventors have developed methods using anion exchange chromatography, enzyme-linked immunosorbent assay and biocatalytic enzyme-based assay for purification and quantification of fucoidan from a seawater sample. These methods are particularly advantageous as they result in increased specificity and sensitivity (see Table 8). The advantages of the presented invention comprise specific quantification compared to bulk organic carbon measurements and focus on fucoidan while discriminating against possible false positives e.g. free monosaccharides and protein-glycans. Moreover, the invention is scalable from milliliters to liters depending on desired concentration factor.

In addition, the invention, inter alia, provides the following advantages:
- The invention provides for the first time methods to reliably purify and quantify fucoidan from a seawater sample
- Fucoidan purification via an anion exchange chromatography column results in a high purity fucoidan eluate and can be performed directly with seawater, i.e. without dilution or dialysis.
- Quantification of fucoidan-specific monosaccharides, such as fucose, via a highly specific enzyme digestion of fucoidan allow for robust quantification of fucoidan concentration in the initial/input sample.
- Quantification of fucoidan-specific monosaccharides, such as fucose, via a highly specific enzyme digestion of fucoidan can be performed on samples of seawater directly or after purifying fucoidan from a sample.
- Selective purification and/or quantification of fucoidan from seawater using anion exchange chromatography (AEX) prior to, e.g. hydrolysis, provides previously unachieved specificity.
- Purification from seawater using AEX enables highly quantitative concentration of fucoidan lowering the detection limit of quantitative analyses by orders of magnitude and thereby allowing the quantification of dissolved, environmental fucoidan

In the following the invention is described in more detail.

In particular, the invention relates to the following items:
1. A method for purifying fucoidan, comprising:
   (i) obtaining a sample of seawater, and
   (ii) purifying fucoidan from the sample.
2. The method of item 1, further comprising a prefiltration step, optionally wherein the prefiltration step comprises filtrating the sample of seawater through a filter, e.g. a combusted glass fiber filter.
3. The method of item 1 or 2, wherein step (ii) comprises purification of fucoidan from the sample of seawater via an anion exchange chromatography.
4. The method of item 3, comprising purifying fucoidan via an anion exchange chromatography column.
5. The method of item 4, wherein the column is conditioned with at least one buffer that resembles the salinity/salt concentration of the sample.
6. The method of item 5, wherein the column is conditioned with a buffer A and/or a buffer B.
7. The method of item 6, wherein buffer A comprises a same or similar salinity/salt concentration as the sample of seawater.
8. The method of any one of items 5-7, wherein the method further comprises measuring the salinity/salt concentration in the sample of seawater and adjusting the salinity/salt concentration of buffer A to correspond to that of the sample of seawater.
9. The method of any one of items 6-8, further comprising conditioning the column with buffer A, optionally followed by buffer B, optionally followed by buffer A.
10. The method of any one of items 6-10, further comprising conditioning the column with about 1-5 column volumes of buffer A, optionally followed by about 1-5 column volumes of buffer B, optionally followed by about 1-5 column volumes of buffer A.
11. The method of any one of items 4-10, wherein the column has a volume of about 1-200 ml.
12. The method of any one of items 6-11, wherein buffer A comprises about 20 mM Tris-HCl, about 0.4-0.7 M NaCl at about pH 8, and wherein buffer B comprises about 20 mM Tris-HCl, about 4-5 M NaCl at about pH 8.
13. The method of any one of items 6-12, wherein buffer A consists of 20 mM Tris-HCl, 0.5 M NaCl at pH 8 in an aqueous solution, and wherein buffer B consists of 20 mM Tris-HCl, 4 M NaCl at pH 8 in an aqueous solution.
14. The method of any one of items 5-13, further comprising applying the sample of seawater onto the conditioned column.
15. The method of any one of items 1-14, wherein the sample of seawater is undiluted or, wherein the sample of seawater is not diluted.
16. The method of any one of items 1-15, wherein the salt concentration in the sample of seawater is about 0.085-0.7 M NaCl or about 5-40 grams salt per kilogram.
17. The method of any one of items 1-16, wherein the sample of seawater comprises about 1 ml to about 101 of seawater, preferably about 100 ml to 1 l of seawater.
18. The method of any one of items 14-17, further comprising a washing step which comprises applying a volume of wash buffer to the column.
19. The method of item 18, wherein the salinity/salt concentration of the wash buffer resembles the salinity/salt concentration of the sample of seawater.
20. The method of item 19, wherein the wash buffer comprises a same or similar salinity/salt concentration as the sample of seawater.
21. The method of any one of items 18-20, wherein the method further comprises measuring the salinity/salt concentration in the sample of seawater and adjusting the salinity/salt concentration of the wash buffer to correspond to that of the sample of seawater.
22. The method of any one of items 18-21, wherein the washing step removes undesired contaminants from the sample of seawater, optionally wherein contaminants are eluted by the wash buffer.
23. The method of any one of items 18-22, wherein the washing step comprises washing the column with about 1-5 column volumes of washing buffer, optionally wherein the washing step is repeated 1-10 times.
24. The method of any one of items 18-23, wherein the wash buffer comprises about 20 mM Tris-HCl, about 0.4-0.7 M NaCl at about pH 8.
25. The method of any one of items 18-24, wherein the wash buffer consists of 20 mM Tris-HCl, 0.5 M NaCl at pH 8 in an aqueous solution.
26. The method of any one of items 1-25, further comprising an elution step which comprises applying a volume of elution buffer to the column.
27. The method of item 26, wherein the elution buffer comprises about 20 mM Tris-HCl, about 4-5 M NaCl at about pH 8.
28. The method of item 26 or 27, wherein the elution step comprises eluting the fucoidan with about 1-5 column volumes of elution buffer, optionally wherein the elution step is repeated 1-10 times, optionally using only one volume of elution buffer.
29. The method of any one of items 26-28, further comprising a reconditioning step, comprising rinsing the column with about 1-5 column volumes of buffer B comprising about 20 mM Tris-HCl, about 0.5 M NaCl, at about pH 8, followed by about 1-5 column volumes of ultrapure water, followed by about 1-5 column volumes of about 1 M NaOH at a reduced flow rate, followed by about 1-5 column volumes of ultrapure water.
30. The method of item 1 or 2, further comprising a precipitation step, wherein fucoidan in the sample of seawater is precipitated.
31. The method of item 30, comprising reducing the salinity/salt concentration in the sample of seawater.
32. The method of item 30 or 31, wherein the sample of seawater is diluted with ultrapure water.
33. The method of item 32, wherein the precipitation step comprises adding metal ions, alcohols, low pH acids, or an amphiphilic compound to the sample of seawater.
34. The method of item 33, wherein the amphiphilic compound is a quaternary ammonium surfactant.
35. The method of item 33 or 34, wherein the concentration of the amphiphilic compound in the sample of seawater is about 0.1 g/l.
36. The method of any one of items 32-35, further comprising a filtration step, wherein the filtration step comprises filtrating the sample of seawater through a filter, e.g. a combusted glass fiber filter.
37. The method of item 36 further comprising an elution or resuspension step, wherein the precipitated fucoidan is eluted or resuspended from the filter.
38. The method of any one of items 1-37, further comprising step (iii) quantifying fucoidan in the sample.
39. The method of item 38, wherein the quantifying step comprises processing of fucoidan into monosaccharides.
40. The method of item 38 or 39, wherein the quantifying step comprises acid hydrolysis of the sample of seawater or fucoidan in the sample of seawater, or the eluted sample of seawater or the precipitated sample of seawater.
41. The method of item 38, wherein the quantifying step comprises quantifying the binding of fucoidan in the sample to an anti-fucoidan antibody.
42. The method of any one of items 1-29 and 37-41, comprising:
   (a) a step of obtaining a sample of seawater,
   (b) a prefiltration step, comprising filtrating the sample of seawater through a combusted glass fiber filter,
   (c) a conditioning step, comprising conditioning an anion exchange chromatography column with about 1-5 column volumes of buffer A comprising about 20 mM Tris-HCl, about 0.5 M NaCl at about pH 8, followed by about 1-5 column volumes of buffer B comprising about 20 mM Tris-HCl, about 4 M NaCl at about pH 8, followed by about 1-5 column volumes of buffer A comprising about 20 mM Tris-HCl, about 0.5 M NaCl at about pH 8,
   (d) an application step, comprising applying the sample of seawater onto the column
   (e) a washing step, washing the column with about 1-5 column volumes of wash buffer comprising about 20 mM Tris-HCl, about 0.5 M NaCl at about pH 8, optionally repeated the washing step 1-10 times,
   (f) an elution step, comprising eluting fucoidan with about 1-5 column volumes of elution buffer comprising about 20 mM Tris-HCl, about 4 M NaCl at about pH 8,
   (g) an optional reconditioning step, comprising rinsing the column with about 1-5 column volumes of buffer B comprising about 20 mM Tris-HCl, about 0.5 M NaCl, at about pH 8, followed by about 1-5 column volumes of ultrapure water, followed by about 1-5 column volumes of about 1 M NaOH at a reduced flow rate, followed by about 1-5 column volumes of ultrapure water,
   (h) a quantification step,
   preferably wherein steps (a)-(h) are performed in chronological/alphabetical order.
43. The method of item 38, further comprising
   (a) contacting a sample comprising a known amount/quantity of fucoidan (fucoidan standard/reference sample) with enzymes capable of digesting fucoidan,
   (b) quantifying monosaccharides obtained in (a), and
   (c) generating a fucoidan standard curve by correlating the yield of monosaccharides in (b) with the known amount/quantity of fucoidan in the sample of (a).
44. The method of item 38 or 43, further comprising contacting the eluted or precipitated sample with enzymes capable of digesting fucoidan.
45. The method of item 44, further comprising comparing the yield of monosaccharides, such as fucose, obtained from the fucoidan digestion in the eluted or precipitated sample with a fucoidan standard curve, thereby quantifying the amount of fucoidan in the sample of seawater.
46. A method for quantifying fucoidan, comprising:
   (i) obtaining a sample of seawater, and
   (ii) quantifying fucoidan in the sample.
47. The method of item 46, wherein the quantifying step comprises processing of fucoidan into monosaccharides.
48. The method of item 46 or 47, wherein the quantifying step comprises acid hydrolysis of the sample of seawater.
49. The method of item 46, comprising contacting a sample with enzymes capable of digesting fucoidan, e.g. fucoidanases, sulfatases and/or fucosidases.
50. The method of item 46 or 49, further comprising
   (a) contacting a sample comprising a known amount/quantity of fucoidan (fucoidan standard/reference sample) with enzymes capable of digesting fucoidan,
   (b) quantifying monosaccharides obtained in (a), and
   (c) generating a fucoidan standard curve by correlating the yield of monosaccharides in (b) with the known amount/quantity of fucoidan in the sample of (a).
51. The method of any one of items 46, 49-50, comprising contacting the sample of seawater with enzymes capable of digesting fucoidan, e.g. fucoidanases, sulfatases and/or fucosidases.
52. The method of item 51, comprising comparing the yield of monosaccharides, such as fucose, obtained from the fucoidan digestion in the sample of seawater with a fucoidan standard curve, thereby quantifying the amount of fucoidan in the sample of seawater.
53. The method of any one of items 46, 49-52, further comprising culturing fucoidan-degrading microorganisms, preferably bacteria belonging to the phyla Verrucomicrobiota, Planktomycetota or Bacteroidota, more preferably *Lentimonas sp.* CC4.
54. The method of item 53, further comprising isolating fucoidan-degrading enzymes, e.g. fucoidanases, sulfatases and/or fucosidases, from the culture of fucoidan-degrading microorganisms, optionally wherein the fucoidan degrading enzymes are obtained by a lysis step of the fucoidan-degrading microorganisms followed by a separation step that separates the culture into a cellular fraction and a fraction comprising enzymes capable of digesting fucoidan, e.g. in the supernatant.
55. The method of any one of items 46, 49-54, comprising the steps of:
   (a) a step of obtaining a sample of seawater or a fucoidan standard/reference sample,
   (b) an optional culturing step, comprising culturing a fucoidan-degrading microorganism, e.g. bacteria belonging to the phyla Verrucomicrobiota, Planktomycetota or Bacteroidota,
   (c) an optional lysis step, comprising lysing the fucoidan-degrading microorganism,
   (d) an optional isolation step, comprising separating the lysed fucoidan-degrading microorganism into a cellular fraction and a fraction comprising enzymes capable of digesting fucoidan, e.g. fucoidanases, sulfatases and/or fucosidases, thereby isolating the enzymes capable of digesting fucoidan,
   (e) a contacting step, comprising contacting a sample with enzymes capable of digesting fucoidan, e.g. fucoidanases, sulfatases and/or fucosidases,
   (f) a quantification step, comprising quantifying monosaccharides obtained in (e), preferably wherein the monosaccharide is fucose,
   (g) an optional step generating a fucoidan standard curve, comprising correlating the yield of monosaccharides, such as fucose, in (f) with the known amount/quantity of the fucoidan standard/reference sample of (a)
   (h) an evaluation step, comprising comparing the yield of monosaccharides, preferably fucose, in step (f) with a fucoidan standard curve, e.g. of (g), thereby quantifying the initial amount/quantity of fucoidan in the sample of seawater,
   preferably wherein steps (a)-(h) are performed in chronological/alphabetical order.
56. The method of any one of items 1-55, wherein the salt concentration in the sample of seawater is about 0.085-0.7 M NaCl or about 5-40 grams salt per kilogram.
57. The method of any one of items 1-56, wherein the sample of seawater comprises about 1 ml to about 101, preferably about 100 ml to 11 of seawater.
58. The method of any one of items 1-57, wherein the sample of seawater is obtained from an area where brown algae grow.
59. The method of any one of items 1-58, wherein the sample of seawater is obtained from an ocean, such as the Atlantic Ocean, the Pacific Ocean, the Indian Ocean, the Arctic Ocean, and/or the Southern Ocean.
60. The method of any one of items 1-59, wherein the sample of seawater is obtained from, the Mediterranean Sea, the Baltic Sea, Red Sea, and/or Black Sea.
61. The method of any one of items 1-60, or the method of any one of items 1-58, wherein the sample of seawater is obtained from an aquafarm, aquaculture or aquafarming, optionally wherein the aquafarm, aquaculture or aquafarming is on land.
62. The method of any one of items 1-61, wherein the sample of seawater is obtained from a brown algae aquaculture.
63. The method of any one of items 58-62, wherein the brown alga is a brown alga selected from the class of Phaeophyceae.
64. The method of any one of items 58-63, wherein the brown algae are one or more of *Lessonia trabeculata, Durvillaea potatorum, Laminaria digitata, Ecklonia maxima, Ecklonia radiata, Fucus vesiculosus, Macrocystis pyrifera, Undaria pinnatifida, Saccharina latissima, Saccharina japonica, Sargassum fluitans, Sargassum natans, Laminaria hyperborea, Ecklonia cava* or *Fucus serratus.*
65. A composition comprising fucoidan produced by the method of any one of items 1-37.

The present invention provides in the broadest sense a method for purifying and/or quantifying fucoidan. Fucoidan is a complex, heterogeneous polysaccharide that is found in many eukaryotic taxa, including brown algae. Fucoidan is also known as fucose-containing sulphated polysaccharide or fucan. Two broader types of fucoidan are frequently distinguished: Homofucans typically consists of an alpha-L-fucose chain with alpha-1,2, alpha-1,3- and/or alpha-1,4-glycosidic linkages, 0-3 sulphate groups on each fucose monosaccharide, additional acetyl-groups, and branches next to the main saccharide chain that incorporate a range of monosaccharides. Next to fucose, fucoidan often contains galactose, xylose, mannose, glucose, rhamnose, uronic acids and amino sugars. In another type of fucoidan, heterofucans, the main saccharide chain comprises a range of monosaccharides including the aforementioned examples and fucose is mostly found in branches. Heterofucans can carry the same kind of decorations as homofucans. As used herein, fucoidan (to be purified and/or to be quantified as described herein) or fucoidan as comprised in a herein described sample of seawater is typically produced by brown algae/ brown alga as defined herein. Brown algae/ brown alga as defined herein can secrete fucoidan into seawater. Thus, a sample of seawater to be used herein which is collected/obtained from an area where brown algae grow or are cultivated comprises such secreted fucoidan. Preferably, the brown alga is a brown alga from the class of Phaeophyceae. More preferably, the brown algae are one or more of *Lessonia trabeculata, Durvillaea potatorum, Laminaria digitata, Ecklonia maxima, Ecklonia radiata, Fucus vesiculosus, Macrocystis pyrifera, Undaria pinnatifida, Saccharina latissima, Saccharina japonica, Sargassum fluitans, Sargassum natans, Laminaria hyperborea, Ecklonia cava* or *Fucus serratus.* It is understood that the sample of seawater (short seawater) as used herein typically comprises fucoidan, which is preferably produced by brown algae/ brown alga as explained and defined herein.

Brown algae annually convert gigatons of carbon dioxide into carbohydrates, including the complex extracellular matrix polysaccharide fucoidan. Due to its persistence in the environment, fucoidan is potentially a pathway for marine carbon sequestration. Rates of fucoidan secretion by brown algae is up to now unknown due to the challenge of identifying and quantifying complex polysaccharides which is addressed by the herein provided invention. In general, the term "purifying" relates to the enrichment of a target compound, e.g. the enrichment of fucoidan from a sample, such as a sample of seawater. In the sense of the present invention "purifying" or "purification" may also relate to an enrichment of a compound of fucose, e.g. fucose, galactose, xylose, mannose, glucose, rhamnose, uronic acids and amino sugars. In particular, the invention provides the purification of fucoidan from a seawater sample which allows for efficient quantification of fucoidan without quantifying other sugars that may be present in the sample. In some embodiments, a method for purifying fucoidan can further comprise quantification of fucoidan.

The method for purifying fucoidan can comprise a step of obtaining a sample. In some embodiments the method for purifying fucoidan does not comprise a step of obtaining a sample. In some embodiments the method for purifying fucoidan optionally comprises a step of obtaining a sample. For example, the method can be performed on a sample that has already been obtained. In a preferred embodiment, the method for purifying fucoidan comprises obtaining a sample of seawater. It is particularly desired to use a sample of seawater in the sense of the present invention, since fucoidan is produced by aquatic organisms in saltwater. Accordingly, it is desired to purify and/or quantify fucoidan in a sample of seawater. In general, the method for purifying fucoidan of the present invention is not limited to any particular form of sample, rather any sample that comprises fucoidan and that is desired to be purified and/or quantified can be used in the sense of the invention. In some embodiments, a sample comprises organic compounds. In some embodiments, a sample comprises polysaccharides. In some embodiments, a sample comprises fucoidan. In some embodiments a sample comprises one or more of laminarin, cellulose, alginate and fucoidan. In some embodiments a sample comprises monosaccharides. In some embodiments a sample comprises one or more of arabinose, fucose, galactosamine, galactose, glucose, mannose, rhamnose, and xylose.

In some embodiments, a sample is a sample comprising dissolved fucoidan. In some embodiments a sample comprises fucoidan dissolved in water. In some embodiments, a sample is seawater. In some embodiments, a sample is a sample of seawater. In some embodiments, a sample of seawater is seawater. In general, a sample in the sense of the present invention, e.g. sample of seawater is not particularly limited in the context of salinity/salt concentration in the sample. Accordingly, a sample, e.g. a sample of seawater can have any salinity/salt concentration. For example, a sample, e.g. a sample of seawater can have a salinity/salt concentration corresponding to the salinity/salt concentration of the water body whereof the sample has been obtained/is obtained. Thus, the salinity/salt concentration of a sample in the sense of the present invention, e.g. sample of seawater is not limited. Salinity/salt concentration of a sample can e.g. be defined by grams of salt per kilogram water, or molarity of specific salts, such as NaCl. Salinity/salt concentration can also be defined by practical salinity unit (psu) corresponding to grams of salt per kilogram water. In general, "salt" as used herein refers to sea salt, i.e. the entirety of salts present in seawater, e.g. a sample of seawater. Exemplary salts present in seawater, e.g. a sample of seawater are NaCl, MgCl₂, Na2SO₄, CaCl₂, KCI, NaHCO₃, KBr, H₃BO₃, SrCl₂ or NaF. In some embodiments a sample comprises fucoidan dissolved in water with a salinity/salt concentration of at least 0.085 M NaCl or at least 5 grams salt per kilogram. In some embodiments, a sample comprises fucoidan dissolved in seawater. In some embodiments a sample comprises fucoidan dissolved in seawater, wherein the seawater has a salinity/salt concentration of about 0.085-0.7 M NaCl or about 30-40 grams salt per kilogram. In some embodiments a sample comprises fucoidan dissolved in seawater, wherein the seawater has a salinity/salt concentration of about 0.085, 0.09, 0.095, 0.1, 0.15, 0.2, 0.25, 0.3, 0.31, 0.32, 0.33, 0.34, 0.35, 0.36, 0.37, 0.38, 0.39, 0.4, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.66, 0.67, 0.68, 0.69, or 0.7 M NaCl or about 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, 30, 30.5, 31, 31.5, 32, 32.5, 33, 33.5, 34, 34.5, 35, 35.5, 36, 36.5, 37, 37.5, 38, 38.5, 39, 39.5, or 40 grams salt per kilogram.

As used herein, the term "grams salt per kilogram" refers preferably to "grams sea salt per kilogram". As explained above, the term "sea salt" refers to the entirety of salts present in seawater, i.e. all salts (the entire amount of all salts), e.g. all of NaCl, MgCl₂, Na2SO₄, CaCl₂, KCl, NaHCO₃, KBr, H₃BO₃, SrCl₂ and/or NaF). It is understood that "per kilogram" refers to "per kilogram water".

In some embodiments, the sample comprises a sample of seawater. In some embodiments a sample of seawater has a salinity/salt concentration of about 5-40 grams salt per kilogram. In some embodiments a sample of seawater has a salinity/salt concentration of about 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, 30, 30.5, 31, 31.5, 32, 32.5, 33, 33.5, 34, 34.5, 35, 35.5, 36, 36.5, 37, 37.5, 38, 38.5, 39, 39.5, or 40 grams salt per kilogram. Salinity is the mass of salts in grams contained in 1 kilogram of seawater. The salinity of seawater is on average about 35 g/kg. In some embodiments a sample of seawater has a salinity/salt concentration of about 0.085-0.7 M NaCl or about 5-40 grams salt per kilogram. In some embodiments a sample of seawater has a salinity/salt concentration of about 0.3-0.7 M NaCl or about 30-38 grams salt per kilogram. In some embodiments a sample of seawater has a salinity/salt concentration of about 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, 30, 30.5, 31, 31.5, 32, 32.5, 33, 33.5, 34, 34.5, 35, 35.5, 36, 36.5, 37, 37.5, 38, 38.5, 39, 39.5, or 40 grams salt per kilogram. In some embodiments a sample of seawater has a salinity/salt concentration of about 0.085, 0.09, 0.095, 0.1, 0.15, 0.2, 0.25, 0.3, 0.31, 0.32, 0.33, 0.34, 0.35, 0.36, 0.37, 0.38, 0.39, 0.4, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.66, 0.67, 0.68, 0.69, or 0.7 M NaCl or about 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, 30, 30.5, 31, 31.5, 32, 32.5, 33, 33.5, 34, 34.5, 35, 35.5, 36, 36.5, 37, 37.5, 38, 38.5, 39, 39.5, or 40 grams salt per kilogram.

In some embodiments a sample of seawater comprises about 1 ml to about 10 1 of seawater. In some embodiments a sample of seawater comprises about 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000 ml of seawater or about 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, or 10 l of seawater. In a preferred embodiment, a sample of seawater comprises about 100-1000 ml of seawater. In a preferred embodiment, a sample of seawater comprises about 100, 200, 300, 400, 500, 600, 700, 800, 900, or 1000 ml of seawater. In another preferred embodiment, a sample of seawater comprises about 400 ml of seawater. In another preferred embodiment, a sample of seawater comprises about 100 ml of seawater.

A sample of seawater is preferably obtained or collected from (an area) where brown algae grow or are cultivated, e.g. obtained from within close vicinity to brown algae, such as obtained/collected at a distance of between about 1 cm to 100 m from the surface of a brown alga/algae, e.g. between about 1 cm to 90 m, 80 m, 70 m, 60 m, 50 m, 40 m , 30 m, 20 m, 10 m, 9 m, 8 m, 7 m, 6 m, 5 m, 4 m, 3 m, 2 m, 1 m, 90 cm, 80 cm, 70 cm, 60 cm, 50 cm, 40 cm, 30 cm, 20 cm or 10 cm. The sample can also be directly obtained from the surface of the brown alga/algae. A sample of seawater can also be obtained or collected from a distance of at least 1 m, 10 m, 50 m, 100 m, 150 m, 200 m, 250 m, 300 m, 350 m, 400 m, 450 m, 500 m, 550 m, 600 m, 650 m, 700 m, 750 m, 800 m, 850 m, 900 m, 950 m, 1 km, 2 km, 3 km, 4 km, 5 km, 6 km, 7 km, 8 km, 9 km, or 10 km from brown algae, e.g. kelp forests. However, the method for purifying fucoidan from a sample also allows quantification of minimal amounts of fucoidan from a sample, e.g. a sample obtained from open waters or from deep seas. As used herein, the terms "grow" and "is/are cultivated" can be used interchangeably. Most preferably, the sample of seawater is an environmental sample. An environmental sample as used herein usually is sea water collected/obtained from directly where the brown algae grow (e.g. they grow in an ocean or in aquaculture). In this context "collected" and "obtained" can be used interchangeably. For example, if the brown algae grow in an ocean, the environmental sample is the ocean seawater in which the brown algae grow, i.e. the seawater occurring in nature/at the natural site where the algae grow. The same explanation applies mutatis mutandis to algae grown, e.g. in aquaculture. For example, if the brown algae grow in an aquaculture, the environmental sample is the aquaculture seawater in which the brown algae grow, i.e. the seawater occurring in nature/at the (natural) site where the algae grow. In line with the above, the amount and/or concentration of fucoidan in the sample to be used herein is the amount and/or concentration fucoidan has in the corresponding sample, e.g. seawater occurring in nature/at the (natural) site where the algae grow.

In some embodiments the sample comprises a sample of seawater obtained from an ocean. In some embodiments the sample comprises a sample of seawater obtained from the Atlantic Ocean, the Pacific Ocean, the Indian Ocean, the Arctic Ocean, and/or the Southern Ocean. In some embodiments the sample comprises a sample of seawater obtained from, the Mediterranean Sea, the Baltic Sea, Red Sea, and/or Black Sea. In some embodiments, the sample comprises a sample obtained from an aquafarm, aquaculture or aquafarming. Aquaculture, also known as aquafarming, is the controlled cultivation ("farming") of aquatic organisms, including algae. Aquaculture involves cultivation of organisms in freshwater, brackish water and saltwater (herein preferably saltwater) under controlled or semi-natural conditions. Mariculture, commonly known as marine farming, refers specifically to aquaculture practiced in seawater habitats and lagoons, opposed to in freshwater aquaculture. The term "seawater" as used herein can also be "saltwater" provided that sample of saltwater is preferably obtained or collected from (an area) where brown algae grow (e.g. tanks with artificially prepared saltwater, where algae grow). A sample obtained from an aquafarm, aquaculture or aquafarming may also be referred herein as a sample of seawater. In some embodiments, the sample comprises a sample of seawater obtained from an aquaculture. In some embodiments, the sample comprises a sample of seawater obtained from an aquaculture that cultivates brown algae of the class Phaeophyceae or in which brown algae of the class Phaeophyceae grow. In some embodiments, the sample comprises a sample of seawater obtained from a brown algae aquaculture. In some embodiments, the sample comprises a sample of seawater obtained from a *Lessonia trabeculata, Durvillaea potatorum, Laminaria digitata, Ecklonia maxima, Ecklonia radiata, Fucus vesiculosus, Macrocystispyrifera, Undaria pinnatifida, Saccharina latissima, Saccharina japonica, Sargassum fluitans, Sargassum natans, Laminaria hyperborea, Ecklonia cava* or *Fucus serratus* aquaculture.

In some embodiments, a sample of seawater or seawater is not obtained or has not been obtained from biomass. In some embodiments, a sample of seawater or seawater does not comprise biomass obtained from e.g. algae. In preferred embodiments, a sample of seawater or seawater is obtained without directly collecting biomass of e.g. algae such as brown algae. In some embodiments a sample of seawater does not comprise or does essentially not consist of biomass or tissue of brown algae. As used herein, a sample of seawater is not a sample of brown algae (tissue). In some embodiments, a sample of seawater essentially does not comprise or consist of plant material or plant tissue. In some embodiments, a sample of seawater essentially does not comprise or essentially does not consist of algae tissue, such as brown algae tissue. In some embodiments a sample of seawater essentially does not comprise biomass or tissue of brown algae. In a preferred embodiment, the sample of seawater (or short seawater) is essentially free of plant material or plant tissue, specifically wherein the plant is/are brown algae. The term "plant material" as used herein refers to and can include plant tissue, (a) plant cell(s) and/or tissue debris /cell debris. "Essentially free" as used herein means that minute amounts of such "plant material" can be comprised in the sample of seawater to be used herein. These minute amounts do preferably not materially affect the amount/concentration of fucoidan in the seawater sample. In a more preferred embodiment, the sample of seawater (or short seawater) is free of plant material or plant tissue as defined herein, specifically wherein the plant is/are brown algae. "Free" as used herein means that amounts of such "plant material" are not present in the sample of seawater to be used herein or are present in (minute) amounts that are not detectable by routine methods. These minute amounts do not materially affect the amount/concentration of fucoidan in the seawater sample. Such a sample of seawater (essentially) free of plant material or plant tissue as defined herein, specifically wherein the plant is/are brown algae, can be prepared or provided by routine methods, e.g. by filtering the seawater to remove such plant material or plant tissue. For example, a prefiltration step as defined herein, e.g. filtration through a combusted glass fiber filter, can remove particulate matter (including plant material or plant tissue as defined above), so that a sample of seawater is essentially free of (plant) biomass, plant material and/or plant tissue as defined above or is free of biomass, plant material and/or plant tissue as defined above.

The explanations provided above in relation to "essentially free" apply mutatis mutandis to terms above like "a sample of seawater essentially does not comprise or consist of plant material/plant biomass or plant tissue". The explanations provided above in relation to "free" apply mutatis mutandis to terms above like "a sample of seawater does not comprise or consist of plant material/plant biomass or plant tissue".

Accordingly, the invention provides in one aspect a method for purifying fucoidan, comprising:
(i) obtaining a sample of seawater, and
(ii) purifying fucoidan from the sample.

In another aspect, the invention provides a method for purifying fucoidan, comprising purifying fucoidan from a sample, e.g. a sample of seawater.

"Sample of seawater" can be in short "seawater". Accordingly, the invention provides in another aspect a method for purifying fucoidan, comprising purifying fucoidan from seawater. In another aspect, the invention provides a method for purifying fucoidan, comprising:
(i) obtaining seawater, and
(ii) purifying fucoidan from the seawater.

In another aspect, the invention provides a method for purifying fucoidan, comprising:
(i) obtaining a sample of seawater, and
(ii) purifying fucoidan from the sample,
wherein step (i) is optional.

In another aspect, the invention provides a method for purifying fucoidan, comprising:
(i) obtaining a sample of seawater, and
(ii) purifying fucoidan from the sample,
wherein the salt concentration in the sample of seawater is about 0.085-0.7 M NaCl.

In another aspect, the invention provides a method for purifying fucoidan, comprising:
(i) obtaining a sample of seawater, and
(ii) purifying fucoidan from the sample,
wherein the salt concentration in the sample of seawater is about 0.085-0.7 M NaCl.

In another aspect, the invention provides a method for purifying fucoidan, comprising:
(i) obtaining a sample of seawater, and
(ii) purifying fucoidan from the sample,
wherein the salt concentration in the sample of seawater is about 0.085, 0.09, 0.095, 0.1, 0.15, 0.2, 0.25, 0.3, 0.31, 0.32, 0.33, 0.34, 0.35, 0.36, 0.37, 0.38, 0.39, 0.4, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.66, 0.67, 0.68, 0.69, or 0.7 M NaCl.

In another aspect, the invention provides a method for purifying fucoidan, comprising:
(i) obtaining a sample of seawater, and
(ii) purifying fucoidan from the sample,
wherein the sample of seawater has a salinity/salt concentration of at least about 5 grams salt per kilogram.

In another aspect, the invention provides a method for purifying fucoidan, comprising:
(i) obtaining a sample of seawater, and
(ii) purifying fucoidan from the sample,
wherein the sample of seawater has a salinity/salt concentration of about 0.085, 0.09, 0.095, 0.1, 0.15, 0.2, 0.25, 0.3, 0.31, 0.32, 0.33, 0.34, 0.35, 0.36, 0.37, 0.38, 0.39, 0.4, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.66, 0.67, 0.68, 0.69, or 0.7 M NaCl or about 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, 30, 30.5, 31, 31.5, 32, 32.5, 33, 33.5, 34, 34.5, 35, 35.5, 36, 36.5, 37, 37.5, 38, 38.5, 39, 39.5, or 40 grams salt per kilogram.

In another aspect, the invention provides a method for purifying fucoidan, comprising:
(i) obtaining a sample of seawater, and
(ii) purifying fucoidan from the sample,
wherein the sample of seawater comprises about 1 ml to about 10 1 of seawater.

In another aspect, the invention provides a method for purifying fucoidan, comprising:
(i) obtaining a sample of seawater, and
(ii) purifying fucoidan from the sample,
wherein the sample of seawater comprises about 100-1000 ml of seawater.

In another aspect, the invention provides a method for purifying fucoidan, comprising:
(i) obtaining a sample of seawater, and
(ii) purifying fucoidan from the sample,
wherein the sample of seawater is obtained from an area where brown algae grow.

In a preferred aspect, the invention provides a method for purifying fucoidan, comprising:
(i) obtaining a sample of seawater, and
(ii) purifying fucoidan from the sample,
wherein the salt concentration in the sample of seawater is about 0.5 M NaCl, and wherein the sample comprises about 100-1000 ml of seawater, and wherein the sample of seawater is obtained from an area where brown algae grow.

In yet another aspect, the invention provides a method for quantifying fucoidan, comprising:
(i) obtaining a sample of seawater, and
(ii) quantifying fucoidan in the sample.

In another aspect, the invention provides a method for quantifying fucoidan, comprising quantifying fucoidan in a sample, e.g. a sample of seawater.

"Sample of seawater" can be in short "seawater". Accordingly, the invention provides in another aspect a method for quantifying fucoidan, comprising quantifying fucoidan in seawater. In another aspect, the invention provides a method for quantifying fucoidan, comprising:
(i) obtaining seawater, and
(ii) quantifying fucoidan in the seawater.

In another aspect, the invention provides a method for quantifying fucoidan, comprising:
(i) obtaining a sample of seawater, and
(ii) quantifying fucoidan in the sample,
wherein step (i) is optional.

In another aspect, the invention provides a method for quantifying fucoidan, comprising:
(i) obtaining a sample of seawater, and
(ii) quantifying fucoidan in the sample,
wherein the salt concentration in the sample of seawater is about 0.085-0.7 M NaCl.

In another aspect, the invention provides a method for quantifying fucoidan, comprising:
(i) obtaining a sample of seawater, and
(ii) quantifying fucoidan in the sample,
wherein the salt concentration in the sample of seawater is about 0.085-0.7 M NaCl.

In another aspect, the invention provides a method for quantifying fucoidan, comprising:
(i) obtaining a sample of seawater, and
(ii) quantifying fucoidan in the sample,
wherein the salt concentration in the sample of seawater is about 0.085, 0.09, 0.095, 0.1, 0.15, 0.2, 0.25, 0.3, 0.31, 0.32, 0.33, 0.34, 0.35, 0.36, 0.37, 0.38, 0.39, 0.4, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.66, 0.67, 0.68, 0.69, or 0.7 M NaCl.

In another aspect, the invention provides a method for quantifying fucoidan, comprising:
(i) obtaining a sample of seawater, and
(ii) quantifying fucoidan in the sample,
wherein the sample of seawater has a salinity/salt concentration of at least about 5 grams salt per kilogram.

In another aspect, the invention provides a method for quantifying fucoidan, comprising:
(i) obtaining a sample of seawater, and
(ii) quantifying fucoidan in the sample,
wherein the sample of seawater has a salinity/salt concentration of about 0.085, 0.09, 0.095, 0.1, 0.15, 0.2, 0.25, 0.3, 0.31, 0.32, 0.33, 0.34, 0.35, 0.36, 0.37, 0.38, 0.39, 0.4, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.66, 0.67, 0.68, 0.69, or 0.7 M NaCl or about 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, 30, 30.5, 31, 31.5, 32, 32.5, 33, 33.5, 34, 34.5, 35, 35.5, 36, 36.5, 37, 37.5, 38, 38.5, 39, 39.5, or 40 grams salt per kilogram.

In another aspect, the invention provides a method for quantifying fucoidan, comprising:
(i) obtaining a sample of seawater, and
(ii) quantifying fucoidan in the sample,
wherein the sample of seawater comprises about 1 ml to about 10 1 of seawater.

In another aspect, the invention provides a method for quantifying fucoidan, comprising:
(i) obtaining a sample of seawater, and
(ii) quantifying fucoidan in the sample,
wherein the sample of seawater comprises about 100-1000 ml of seawater.

In another aspect, the invention provides a method for quantifying fucoidan, comprising:
(i) obtaining a sample of seawater, and
(ii) quantifying fucoidan in the sample,
wherein the sample of seawater is obtained from an area where brown algae grow.

In a preferred aspect, the invention provides a method for quantifying fucoidan, comprising:
(i) obtaining a sample of seawater, and
(ii) quantifying fucoidan in the sample,
wherein the salt concentration in the sample of seawater is about 0.5 M NaCl, and wherein the sample comprises about 100-1000 ml of seawater, and wherein the sample of seawater is obtained from an area where brown algae grow.

As used herein "purifying fucoidan" can include or can be "preparing/producing a (purified) fucoidan composition". Accordingly, the invention provides in one aspect a method for preparing/producing a (purified) fucoidan composition, comprising:
(i) obtaining a sample of seawater, and
(ii) purifying fucoidan/a fucoidan composition from the sample.

In another aspect, the invention provides a method for preparing/producing a (purified) fucoidan composition, comprising purifying fucoidan/a fucoidan composition from a sample, e.g. a sample of seawater.

In another aspect, the invention provides a method for preparing/producing a (purified) fucoidan composition, comprising:
(i) obtaining a sample of seawater, and
(ii) purifying fucoidan/a fucoidan composition from the sample,
wherein step (i) is optional.

In yet another aspect, the invention provides a method for preparing/producing a (purified) fucoidan composition, comprising purifying fucoidan/a fucoidan composition from a sample of seawater.

As used herein "sample" in general relates to any sample disclosed herein, such as a sample of seawater, a purified sample, an eluted sample, a precipitated sample, a dialyzed sample, or a dried sample, such as a freeze-dried sample. As used herein "sample of seawater" can be in short "seawater". Accordingly, the invention provides in one aspect a method for preparing/producing a (purified) fucoidan composition, comprising purifying fucoidan/a fucoidan composition from seawater. As used herein "sample of seawater" may also relate to a purified sample of seawater, and eluted sample of seawater, a precipitated sample of seawater, a dialyzed sample of seawater, or a dried sample of seawater, such as a freeze-dried sample of seawater.

As used herein, "aquaculture" or "aquafarm" or "aquafarming" relates to the controlled cultivation of aquatic organisms such as fish, crustaceans, mollusks, algae and other organisms of value such as aquatic plants. The terms "aquaculture" or "aquafarm" or "aquafarming" may be used interchangeably herein. In general, an aquaculture in the sense of the present invention can be concerned with cultivating any aquatic organism. A sample from such an aquafarm may comprise brackish water or saltwater. Preferably, a sample obtained from such an aquafarm comprises saltwater/seawater. An aquaculture can be located in coastal marine waters, open oceans or on land. An aquaculture as used herein may also relate to e.g. a culture of marine organisms in tanks. It is desired to also purify and/or quantify fucoidan in said samples obtained from an aquaculture, for example an aquaculture that cultivates brown algae. In some embodiment, an aquaculture cultivates a brown alga selected from the class of Phaeophyceae. In some embodiment, an aquaculture cultivates a brown alga selected from the class of Phaeophyceae and any other aquatic organisms such as fish, crustaceans, mollusks, other algae and other aquatic plants. In some embodiment, an aquaculture cultivates one or more of *Lessonia trabeculata, Durvillaea potatorum, Laminaria digitata, Ecklonia maxima, Ecklonia radiata, Fucus vesiculosus, Macrocystis pyrifera, Undaria pinnatifida, Saccharina latissima, Saccharina japonica, Sargassum fluitans, Sargassum natans, Laminaria hyperborea, Ecklonia cava* or *Fucus serratus.* In some embodiment, an aquaculture cultivates one or more of *Lessonia trabeculata, Durvillaea potatorum, Laminaria digitata, Ecklonia maxima, Ecklonia radiata, Fucus vesiculosus, Macrocystis pyrifera, Undaria pinnatifida, Saccharina latissima, Saccharina japonica, Sargassum fluitans, Sargassum natans, Laminaria hyperborea, Ecklonia cava* or *Fucus serratus* and any other aquatic organisms such as fish, crustaceans, mollusks, other algae and other aquatic plants. In a preferred embodiment, an aquaculture cultivates the brown algae *Fucus vesiculosus.* In another preferred embodiment an aquaculture cultivates the brown algae *Fucus vesiculosus* and any other aquatic organisms such as fish, crustaceans, mollusks, other algae and other aquatic plants. In general, an aquaculture can be in an ocean or a sea, e.g. in coastal waters. An aquaculture can also be on land.

Accordingly, the invention provides in one aspect, a method for purifying fucoidan, comprising:
(i) obtaining a sample of seawater, and
(ii) purifying fucoidan from the sample,
wherein the sample of seawater is obtained from an aquafarm, aquaculture or aquafarming.

In another aspect, the invention provides a method for purifying fucoidan, comprising:
(i) obtaining a sample of seawater, and
(ii) purifying fucoidan from the sample,
wherein the sample of seawater is obtained from a brown algae aquafarm, aquaculture or aquafarming.

In a preferred aspect, the invention provides a method for purifying fucoidan, comprising:
(i) obtaining a sample of seawater, and
(ii) purifying fucoidan from the sample,
wherein the sample of seawater is obtained from an aquaculture that cultivates brown algae of the class Phaeophyceae or in which brown algae of the class Phaeophyceae grow.

In a preferred aspect, the invention provides a method for purifying fucoidan, comprising:
(i) obtaining a sample of seawater, and
(ii) purifying fucoidan from the sample,
wherein the sample of seawater is obtained from a *Lessonia trabeculata, Durvillaea potatorum, Laminaria digitata, Ecklonia maxima, Ecklonia radiata, Fucus vesiculosus, Macrocystis pyrifera, Undaria pinnatifida, Saccharina latissima, Saccharina japonica, Sargassum fluitans, Sargassum natans, Laminaria hyperborea, Ecklonia cava* or *Fucus serratus* aquafarm, aquaculture or aquafarming.

In another preferred aspect, the invention provides a method for purifying fucoidan, comprising:
(i) obtaining a sample of seawater, and
(ii) purifying fucoidan from the sample,
wherein the sample of seawater is obtained from a *Fucus vesiculosus* aquaculture.

In another aspect, the invention provides a method for quantifying fucoidan, comprising:
(i) obtaining a sample of seawater, and
(ii) quantifying fucoidan in the sample,
wherein the sample of seawater is obtained from an aquafarm, aquaculture or aquafarming.

In another aspect, the invention provides a method for quantifying fucoidan, comprising:
(i) obtaining a sample of seawater, and
(ii) quantifying fucoidan in the sample,
wherein the sample of seawater is obtained from a brown algae aquafarm, aquaculture or aquafarming.

In a preferred aspect, the invention provides a method for quantifying fucoidan, comprising:
(i) obtaining a sample of seawater, and
(ii) quantifying fucoidan in the sample,
wherein the sample of seawater is obtained from an aquaculture that cultivates brown algae of the class Phaeophyceae or in which brown algae of the class Phaeophyceae grow.

In a preferred aspect, the invention provides a method for quantifying fucoidan, comprising:
(i) obtaining a sample of seawater, and
(ii) quantifying fucoidan in the sample,
wherein the sample of seawater is obtained from a *Lessonia trabeculata, Durvillaea potatorum, Laminaria digitata, Ecklonia maxima, Ecklonia radiata, Fucus vesiculosus, Macrocystis pyrifera, Undaria pinnatifida, Saccharina latissima, Saccharina japonica, Sargassum fluitans, Sargassum natans, Laminaria hyperborea, Ecklonia cava* or *Fucus serratus* aquafarm, aquaculture or aquafarming.

In another preferred aspect, the invention provides a method for quantifying fucoidan, comprising:
(i) obtaining a sample of seawater, and
(ii) quantifying fucoidan in the sample,
wherein the sample of seawater is obtained from a *Fucus vesiculosus* aquaculture.

The invention also provides samples that comprise a known quantity of fucoidan. These samples are referred herein as "fucoidan standard" or "reference sample". A fucoidan standard or reference sample can for example be generated by extracting fucoidan from biomass. Fucoidan can be extracted from biomass of a brown alga selected from the class of Phaeophyceae. Fucoidan can be extracted from biomass of *Lessonia trabeculata, Durvillaea potatorum, Laminaria digitata, Ecklonia maxima, Ecklonia radiata, Fucus vesiculosus, Macrocystis pyrifera, Undaria pinnatifida, Saccharina latissima, Saccharina japonica, Sargassum fluitans, Sargassum natans, Laminaria hyperborea, Ecklonia cava* or *Fucus serratus.* The amount of fucoidan e.g. in artificial seawater can then be adjusted to generate fucoidan standard or reference sample. One exemplary way to produce such a fucoidan standard or reference sample is described in detail in Example 2. A fucoidan standard or reference sample can for example comprise about 0.01-1 mg/l fucoidan. In some embodiments the fucoidan standard or reference sample comprises about 0.01, 0.02, 0.03, 0.04, 0.05, 0.06, 0.07, 0.08, 0.09, 0.1, 0.11, 0.12, 0.13, 0.14, 0.15, 0.16, 0.17, 0.18, 0.19, 0.2, 0.21, 0.22, 0.23, 0.24, 0.25, 0.26, 0.27, 0.28, 0.29, 0.3, 0.31, 0.32, 0.33, 0.34, 0.35, 0.36, 0.37, 0.38, 0.39, 0.4, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.66, 0.67, 0.68, 0.69, 0.7, 0.71, 0.72, 0.73, 0.74, 0.75, 0.76, 0.77, 0.78, 0.79, 0.8, 0.81, 0.82, 0.83, 0.84, 0.85, 0.86, 0.87, 0.88, 0.89, 0.9, 0.91, 0.92, 0.93, 0.94, 0.95, 0.96, 0.97, 0.98, 0.99, or 1 mg/l fucoidan.

In general, the methods for purifying and/or quantifying fucoidan of the present invention is not limited to any particular form of fucoidan standard or reference sample, rather any fucoidan standard or reference sample that comprises a known quantity of fucoidan can be used in the sense of the invention. It is desired that the fucoidan standard or reference sample matches the properties of the sample of seawater. This is particularly advantageous to produce reliable, comparable and reproducible results when purifying and/or quantifying fucoidan from a sample of seawater. Accordingly, in some embodiments the salinity/salt concentration of the fucoidan standard or reference sample resembles the salinity/salt concentration of the sample of seawater.

In some embodiments the fucoidan standard or reference sample comprise a same or similar salinity/salt concentration as the sample of seawater.

In general, the fucoidan standard or reference sample can be adjusted to the sample of seawater that is desired to be used for fucoidan purification and/or quantification. Accordingly, the above-described features in context sample of seawater, apply mutatis mutandis to the fucoidan standard or reference sample, except that the amount or concentration of the product that is to be purified/quantified is known in the fucoidan standard or reference sample.

In some embodiments, a fucoidan standard or reference sample comprises organic compounds. In some embodiments, a fucoidan standard or reference sample comprises polysaccharides. In some embodiments, a fucoidan standard or reference sample comprises fucoidan. In some embodiments a fucoidan standard or reference sample comprises one or more of laminarin, cellulose, alginate and fucoidan. In some embodiments a fucoidan standard or reference sample comprises monosaccharides. In some embodiments a fucoidan standard or reference sample comprises one or more of arabinose, fucose, galactosamine, galactose, glucose, mannose, rhamnose, and xylose.

In some embodiments, a fucoidan standard or reference sample comprises dissolved fucoidan. In some embodiments a fucoidan standard or reference sample comprises fucoidan dissolved in water. In some embodiments a fucoidan standard or reference sample comprises fucoidan dissolved in water with a salinity/salt concentration of at least 0.085 M NaCl or at least 5 grams salt per kilogram. In some embodiments, a fucoidan standard or reference sample comprises fucoidan dissolved in seawater. In some embodiments, a fucoidan standard or reference sample comprises fucoidan dissolved in artificial seawater. In some embodiments a fucoidan standard or reference sample comprises fucoidan dissolved in seawater or artificial seawater, wherein the seawater or artificial seawater has a salinity/salt concentration of about 0.085-0.7 M NaCl or about 5-40 grams salt per kilogram. In some embodiments a fucoidan standard or reference sample comprises fucoidan dissolved in seawater or artificial seawater, wherein the seawater or artificial seawater has a salinity/salt concentration of about 0.085, 0.09, 0.095, 0.1, 0.15, 0.2, 0.25, 0.3, 0.31, 0.32, 0.33, 0.34, 0.35, 0.36, 0.37, 0.38, 0.39, 0.4, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.66, 0.67, 0.68, 0.69, or 0.7 M NaCl or about 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, 30, 30.5, 31, 31.5, 32, 32.5, 33, 33.5, 34, 34.5, 35, 35.5, 36, 36.5, 37, 37.5, 38, 38.5, 39, 39.5, or 40 grams salt per kilogram. In some embodiments a fucoidan standard or reference sample comprises fucoidan dissolved in seawater or artificial seawater, wherein the seawater or artificial seawater has a salinity/salt concentration of about 0.085, 0.09, 0.095, 0.1, 0.15, 0.2, 0.25, 0.3, 0.31, 0.32, 0.33, 0.34, 0.35, 0.36, 0.37, 0.38, 0.39, 0.4, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.66, 0.67, 0.68, 0.69, or 0.7 M NaCl or about 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, 30, 30.5, 31, 31.5, 32, 32.5, 33, 33.5, 34, 34.5, 35, 35.5, 36, 36.5, 37, 37.5, 38, 38.5, 39, 39.5, or 40 grams salt per kilogram, and wherein the fucoidan standard or reference sample comprises about 0.01, 0.02, 0.03, 0.04, 0.05, 0.06, 0.07, 0.08, 0.09, 0.1, 0.11, 0.12, 0.13, 0.14, 0.15, 0.16, 0.17, 0.18, 0.19, 0.2, 0.21, 0.22, 0.23, 0.24, 0.25, 0.26, 0.27, 0.28, 0.29, 0.3, 0.31, 0.32, 0.33, 0.34, 0.35, 0.36, 0.37, 0.38, 0.39, 0.4, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.66, 0.67, 0.68, 0.69, 0.7, 0.71, 0.72, 0.73, 0.74, 0.75, 0.76, 0.77, 0.78, 0.79, 0.8, 0.81, 0.82, 0.83, 0.84, 0.85, 0.86, 0.87, 0.88, 0.89, 0.9, 0.91, 0.92, 0.93, 0.94, 0.95, 0.96, 0.97, 0.98, 0.99, or 1 mg/l fucoidan.

In the sense of the present invention a fucoidan standard or reference sample may be processed as a sample of seawater, i.e. under identical conditions. Accordingly, the methods provided herein can also be performed on a fucoidan standard or reference sample in addition or instead of a sample of seawater.

The method for purifying and/or quantifying fucoidan of the present invention can also encompass a prefiltration step. It is desired to filter a sample of seawater before subjecting the same to analytical methods. By doing so, undesired particles can be separated from the fraction of the sample comprising fucoidan which results in better purification and/or quantification. For example, a prefiltration step, e.g. filtration through a combusted glass fiber filter, can remove particulate matter (including plant material or plant tissue as defined above), so that a sample of seawater is essentially free of (plant) biomass, plant material and/or plant tissue as defined herein or is free of (plant) biomass, plant material and/or plant tissue as defined herein. As explained herein, in a preferred embodiment, the sample of seawater (or short seawater) is (essentially) free of (plant) biomass, plant material and/or plant tissue, specifically wherein the plant is/are brown algae. The term "plant material" as used herein refers to and can include plant tissue, (a) plant cell(s) and/or tissue debris /cell debris. The term (plant) biomass refers generally to the total quantity or weight of organisms (e.g. plants such as brown algae) in a given area or volume. In general, a prefiltration step in the sense of the present invention is not particularly limited, rather any means of filtrating undesired compounds/particles/matter, e.g. particles suspended in seawater. In the sense of the present invention a prefiltration step can be the first step of processing a sample of seawater. In some embodiments, the prefiltration step can be performed between any of the method steps described herein. A prefiltration step may be performed as often as necessary to separate undesired particles. In some embodiments, a prefiltration step is repeated. In some embodiments, a prefiltration step is repeated 1, 2, 3, 4, 5, 6, 7, 8, 9, or 10 times. In some embodiments, the methods of the present invention, such as the methods for purifying and/or quantifying fucoidan do not comprise a prefiltration step. In general, a prefiltration step comprises filtrating a seawater sample through a filter. Exemplary filters that can be useful in the sense of the present invention are glass fiber filters, combusted glass fiber filters, polyethersulphone (PES) filters, cellulose acetate filters, polytetrafluoroethylene (PTFE) filters, polyamide (Nylon) filters, cellulose nitrate filters, polycarbonate filters, mixed cellulose ester filters, regenerated cellulose filters, polyvinylidene difluoride (PVDF) filters, or non-woven polypropylene filters. In some embodiments, a prefiltration step comprises filtrating a sample of seawater through a filter. In some embodiments, a prefiltration step comprises filtrating a sample of seawater through a combusted glass fiber filter. In some embodiments, a prefiltration step comprises filtrating a sample of seawater through a polyethersulphone (PES) filter, cellulose acetate filter, polytetrafluoroethylene (PTFE) filter, polyamide (Nylon) filter, cellulose nitrate filter, polycarbonate filter, mixed cellulose ester filter, regenerated cellulose filter, polyvinylidene difluoride (PVDF) filter, or non-woven polypropylene filter. In some embodiments the prefiltration step is performed after (i) obtaining a sample of seawater and before (ii) purifying fucoidan from a sample.

Accordingly, the invention provides in one aspect a method for purifying fucoidan, comprising:
(i) obtaining a sample of seawater, and
(ii) purifying fucoidan from the sample,
further comprising a prefiltration step.

In another aspect the invention provides a method for purifying fucoidan, comprising:
(i) obtaining a sample of seawater, and
(ii) purifying fucoidan from the sample,
further comprising a prefiltration step, wherein the prefiltration step comprises filtrating the sample of seawater through a polyethersulphone (PES) filter, cellulose acetate filter, polytetrafluoroethylene (PTFE) filter, polyamide (Nylon) filter, cellulose nitrate filter, polycarbonate filter, mixed cellulose ester filter, regenerated cellulose filter, polyvinylidene difluoride (PVDF) filter, non-woven polypropylene filter or a combusted glass fiber filter.

In another aspect the invention provides a method for purifying fucoidan, comprising:
(i) obtaining a sample of seawater, and
(ii) purifying fucoidan from the sample,
further comprising a prefiltration step, wherein the prefiltration step comprises filtrating the sample of seawater through a combusted glass fiber filter.

In another aspect, the invention provides a method for quantifying fucoidan, comprising:
(i) obtaining a sample of seawater, and
(ii) quantifying fucoidan in the sample,
further comprising a prefiltration step.

In another aspect, the invention provides a method for quantifying fucoidan, comprising:
(i) obtaining a sample of seawater, and
(ii) quantifying fucoidan in the sample,
further comprising a prefiltration step, wherein the prefiltration step comprises filtrating the sample of seawater through a polyethersulphone (PES) filter, cellulose acetate filter, polytetrafluoroethylene (PTFE) filter, polyamide (Nylon) filter, cellulose nitrate filter, polycarbonate filter, mixed cellulose ester filter, regenerated cellulose filter, polyvinylidene difluoride (PVDF) filter, non-woven polypropylene filters or a combusted glass fiber filter.

In another aspect, the invention provides a method for quantifying fucoidan, comprising:
(i) obtaining a sample of seawater, and
(ii) quantifying fucoidan in the sample,
further comprising a prefiltration step, wherein the prefiltration step comprises filtrating the sample of seawater through a combusted glass fiber filter.

The invention provides a method for purifying and/or quantifying fucoidan, wherein the purification comprises the use of an anion exchange chromatography, e.g. via an anion exchange chromatography column. Fucoidan is a negatively charged polysaccharide. Anion exchange chromatography is a process that separates substances based on their charges using an ion-exchange resin containing positively charged groups. Anion exchange resins will bind to negatively charged molecules, displacing the counter-ion. Anion exchange chromatography can thus be used to purify negatively charged fucoidan. An anion exchange chromatography in the sense of the present invention can be performed in a column. In general, the invention is not limited to any specific anion exchange chromatography or anion exchange chromatography column, rather any anion exchange chromatography or anion exchange chromatography column can be used that is able to separate the negatively charged fucoidan from other compounds in a sample, e.g. sample of seawater. The volume of an anion exchange chromatography column can range from 1 ml to more than 120 l. The volume of an anion exchange chromatography column depends on the setup and application, column volumes that may be useful in the sense of the invention may be between about 1-200 ml. Exemplary anion exchange chromatography columns that can be used in the sense of the present invention are High Trap fast flow ANX anion exchange columns with 5 mL bed volume (Cytiva, USA). An anion exchange chromatography column is especially useful in the sense of the invention since it can be used to purify fucoidan from a sample of seawater. In some embodiments, an anion exchange chromatography column can have a volume of about 1-200 ml. In some embodiments, an anion exchange chromatography column can have a volume of about 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, or 200 ml. In some embodiments, an anion exchange chromatography column can have a volume of about 5 ml. In some embodiments, the flow rate of compounds applied onto the column, e.g. samples (such as a sample of seawater, eluted sample, precipitated sample or purified sample) or buffers as disclosed herein (such as a buffer, conditioning buffer, washing buffer, elution buffer, compounds for reconditioning, buffer A, or buffer B) can be about 0.5-10 ml/min, such as 0.5, 1, 1.5, 2, 2.5, 3, 3.5, 4, 4.5, 5, 5.5, 6, 6.5, 7, 7.5, 8, 8.5, 9, 9.5, or 10 ml/min. In some embodiments, a reduced flow rate is 1 ml/min.

Accordingly, the invention provides in one aspect a method for purifying fucoidan, comprising:
(i) obtaining a sample of seawater, and
(ii) purifying fucoidan from the sample via an anion exchange chromatography.

In another aspect, the invention provides a method for purifying fucoidan, comprising:
(i) obtaining a sample of seawater, and
(ii) purifying fucoidan from the sample via an anion exchange chromatography column.

As mentioned above, the use of an anion exchange chromatography column is particularly advantageous when purifying fucoidan from a sample of seawater. To efficiently separate fucoidan from other undesired compounds the anion exchange chromatography column can be conditioned with one or more buffers that resemble the salinity/salt concentration of the sample of seawater from which fucoidan is to be purified. As used herein "buffer(s) resembling the salinity/salt concentration of a sample" means that a buffer has a similar or identical salinity/salt concentration as the sample. As used herein "same or similar salinity/salt concentration" means that for example a buffer has an identical or comparable salinity/salt concentration as the sample. In general, the salinity/salt concentration of a buffer resembles that of a sample if all compounds solved in the sample can be solved in the buffer. Accordingly, a buffer may have a same, identical or similar salinity/salt concentration as a sample if all compounds solved in the sample can be solved in the buffer. As used herein, if the "salinity/salt concentration" is similar or resembles that of the sample, the "salinity/salt concentration" at least is in the range of about ± 25 %, preferably is in the range of about ± 10 %, more preferably ± 5 %, most preferably ± 1 % of the "salinity/salt concentration" of the sample. For example, if the "salinity/salt concentration" of the sample is 40 grams salt per kilogram, a similar or resembling "salinity/salt concentration" is at least between 30 to 50 grams salt per kilogram (about ± 25 %), preferably between 36 to 44 grams salt per kilogram (about ± 10 %), between 38 to 42 grams salt per kilogram (about ± 5 %), or between 39.4 to 40.4 grams salt per kilogram (about ± 1 %).

In the sense of the present invention, it is desired that at least one buffer, such as a buffer for conditioning a column or a washing buffer resembles the salinity/salt concentration of a sample. This enables binding of negatively charged fucoidan to the column and allows for efficient separation of undesired compounds other than fucoidan. The buffer with the corresponding salinity/salt concentration is able to solve said undesired compounds, so that they do not bind to the column resulting in increased purity of fucoidan.

Accordingly, the invention provides in one aspect a method for purifying fucoidan, comprising:
(i) obtaining a sample of seawater, and
(ii) purifying fucoidan from the sample via an anion exchange chromatography column,
wherein the column is conditioned with at least one buffer that resembles the salinity/salt concentration of the sample of seawater.

In another aspect, the invention provides a method for purifying fucoidan, comprising:
(i) obtaining a sample of seawater, and
(ii) purifying fucoidan from the sample via an anion exchange chromatography column,
wherein the column is conditioned with at least one buffer having an identical, same or similar salinity/salt concentration as the sample of seawater.

In another aspect, the invention provides a method for purifying fucoidan, comprising:
(i) obtaining a sample of seawater, and
(ii) purifying fucoidan from the sample via an anion exchange chromatography column,
wherein the column is conditioned with at least one buffer having a salinity/salt concentration corresponding to the salinity/salt concentration of the sample of seawater.

For optimal fucoidan purification and/or quantification, the salinity/salt concentration of a sample of seawater can be determined/measured before purification. The salinity/salt concentration of at least one buffer, such as a buffer for conditioning a column or a washing buffer, can then be adjusted to the individually measured salinity/salt concentration of a sample of seawater.

Accordingly, the invention provides in one aspect a method for purifying fucoidan, comprising:
(i) obtaining a sample of seawater, and
(ii) purifying fucoidan from the sample via an anion exchange chromatography column,
wherein the method further comprises measuring the salinity/salt concentration in the sample of seawater and adjusting the salinity/salt concentration of buffer A to correspond to that of the sample of seawater.

As used herein "adjusting salinity/salt concentration of a buffer to correspond to that of a sample" means that the salinity/salt concentration of a buffer is adjusted to reflect the salinity/salt concentration of a sample, e.g. sample of seawater as close as possibly, preferably adjusting the salinity/salt concentration of the buffer to be identical to that of the sample.

The anion exchange chromatography column can be conditioned with one or more buffers. In particular, the anion exchange chromatography column can be conditioned with a first buffer A and a second buffer B, preferably, wherein buffer A has a same or similar salinity/salt concentration as the sample of seawater. If the column anion exchange chromatography column is new or does not contain any contaminants, the conditioning step can comprise conditioning the column with buffer A only. In some embodiments, the conditioning step comprises conditioning the column with buffer A. In some embodiments, the method for purifying fucoidan comprising obtaining a sample of seawater, and purifying fucoidan from the sample via an anion exchange chromatography column further comprising conditioning the column with buffer A. In some embodiments, the anion exchange chromatography column is not conditioned with buffer B.

In some embodiments, the anion exchange chromatography column can be conditioned first with buffer A, followed by buffer B, followed by buffer A. In general, conditioning the column relates to incubating the column with a buffer, e.g. letting a buffer run through the column. As mentioned above, the invention is not particularly limited to any volume of buffer or particular buffer compositions, rather any volume of buffer of any composition that is able to condition the column for binding negatively charged fucoidan can be used in the sense of the invention. It is however preferred herein, that at least one conditioning buffer resembles the salinity/salt concentration of the sample of seawater, which preferably is buffer A. In general, the volume of buffers used is not particularly limited, the volumes can be defined for example via the column volume. In general, it is preferred that at least about 1-5 column volumes are used, in the context of applying any buffers to a column of the present invention. In the sense of the present invention an anion exchange chromatography column can be conditioned with about 1-5, such as about 1, 2, 3, 4, or 5 column volumes of buffer. In the sense of the present invention an anion exchange chromatography column, e.g. of 1-5 mL column volume, can be conditioned with about 5-50 ml of buffer. In some embodiments an anion exchange chromatography column can be conditioned with about 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 ml of buffer.

In some embodiments, an anion exchange chromatography column can be conditioned with about 1-5, such as 1, 2, 3, 4, or 5 column volumes of buffer A. In some embodiments, an anion exchange chromatography column can be conditioned with about 5-50 ml of buffer A. In some embodiments, an anion exchange chromatography column can be conditioned with about 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 ml of buffer A. In some embodiments, an anion exchange chromatography column can be conditioned with about 1-5, such as about 1, 2, 3, 4, or 5 column volumes of buffer B. In some embodiments, an anion exchange chromatography column can be conditioned with about 5-50 ml of buffer B. In some embodiments, an anion exchange chromatography column can be conditioned with about 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 ml of buffer B. In some embodiments, an anion exchange chromatography column can be conditioned with about 1-5, such as about 1, 2, 3, 4, or 5 column volumes of buffer A, followed by about 1-5, such as about 1, 2, 3, 4, or 5 column volumes of buffer B, followed by about 1-5, such as about 1, 2, 3, 4, or 5 column volumes of buffer A. In some embodiments, an anion exchange chromatography column can be conditioned with about 5-50 ml of buffer A, followed by about 5-50 ml of buffer B, followed by about 5-50 ml buffer A. In some embodiments, an anion exchange chromatography column can be conditioned with about 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 ml of buffer A, followed by about 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 ml of buffer B, followed by about 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 ml buffer A. In some embodiments, an anion exchange chromatography column can be conditioned with about 5 column volumes of buffer A. In some embodiments, an anion exchange chromatography column can be conditioned with about 25 ml of buffer A. In some embodiments, an anion exchange chromatography column can be conditioned with about 5 column volumes of buffer B. In some embodiments, an anion exchange chromatography column can be conditioned with about 25 ml of buffer B. In some embodiments, an anion exchange chromatography column can be conditioned with about 5 column volumes of buffer A, followed by about 5 column volumes of buffer B, followed by about 5 column volumes of buffer A. In some embodiments, an anion exchange chromatography column can be conditioned with about 25 ml of buffer A, followed by about 25 ml of buffer B, followed by about 25 ml buffer A. Conditioning of the column, e.g. with buffers, as disclosed herein may also be referred to as a "conditioning step". In some embodiments, a conditioning step is repeated. In some embodiments, a conditioning step is repeated 1, 2, 3, 4, 5, 6, 7, 8, 9, or 10 times. In some embodiments, the methods of the present invention, such as the methods for purifying and/or quantifying fucoidan do not comprise a conditioning step or conditioning of the column. As used herein "conditioning buffer" relates to a buffer that can be used to condition an anion exchange chromatography column. A conditioning buffer in the sense of the present invention preferably is buffer A and/or buffer B. In general, the composition of the buffers that can be used in the conditioning of the column, e.g. conditioning step, is not particularly limited, rather any buffer composition that is able to condition the column for binding negatively charged fucoidan can be used in the sense of the invention. It is however preferred herein, that at least one conditioning buffer resembles the salinity/salt concentration of the sample of seawater, which preferably is buffer A.

In general, conditioning buffers used in the sense of the present invention can preferably comprise salt. The pH of a buffer is preferably pH 7.5 to pH 8.5. A buffer in the sense of the present invention can comprise a salt concentration of about 0-10 M NaCl. As used herein "salinity/salt concentration" in context of a buffer preferably relates to NaCl concentration. In particular, a buffer for conditioning an anion exchange column in the sense of the present invention can comprise Tris-HCl and NaCl at appropriate concentrations in an aqueous solution. As mentioned, it is desired that at least one conditioning buffer resembles the salinity/salt concentration of the sample of seawater. Accordingly, buffer A may resemble the salinity/salt concentration of the sample of seawater, i.e. an appropriate concentration of a salt, e.g. NaCl. In some embodiments a conditioning buffer comprises about 10-30 mM Tris-HCl and about 0.05-1 M NaCl. In some embodiments a conditioning buffer comprises about 10, 11, 12, 13, 14, 15, 16, 17, 18, 19, 20, 21, 22, 23, 24, 25, 26, 27, 28, 29, or 30 mM Tris-HCl and about 0.05, 0.06, 0.07, 0.08, 0.09, 0.1, 0.11, 0.12, 0.13, 0.14, 0.15, 0.16, 0.17, 0.18, 0.19, 0.2, 0.21, 0.22, 0.23, 0.24, 0.25, 0.26, 0.27, 0.28, 0.29, 0.3, 0.31, 0.32, 0.33, 0.34, 0.35, 0.36, 0.37, 0.38, 0.39, 0.4, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.66, 0.67, 0.68, 0.69, 0.7, 0.71, 0.72, 0.73, 0.74, 0.75, 0.76, 0.77, 0.78, 0.79, 0.8, 0.81, 0.82, 0.83, 0.84, 0.85, 0.86, 0.87, 0.88, 0.89, 0.9, 0.91, 0.92, 0.93, 0.94, 0.95, 0.96, 0.97, 0.98, 0.99, or 1 MNaCl. In some embodiments a conditioning buffer is buffer A. In a preferred embodiment, buffer A comprises about 10-30 mM Tris-HCl and about 0.4-0.7 M NaCl. In another preferred embodiment, buffer A comprises about 10, 11, 12, 13, 14, 15, 16, 17, 18, 19, 20, 21, 22, 23, 24, 25, 26, 27, 28, 29, or 30 mM Tris-HCl and about 0.4, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.66, 0.67, 0.68, 0.69, or 0.7 M NaCl. In some embodiments a buffer A comprises about 20 mM Tris-HCl and about 0.5 M NaCl. In some embodiments a conditioning buffer is buffer B. In some embodiments a buffer B comprises about 10-30 mM Tris-HCl and about 1-10 MNaCl. In some embodiments a buffer B comprises about 10, 11, 12, 13, 14, 15, 16, 17, 18, 19, 20, 21, 22, 23, 24, 25, 26, 27, 28, 29, or 30 mM Tris-HCl and about 1, 2, 3, 4, 5, 6, 7, 8, 9, or 10 M NaCl. In some embodiments a buffer B comprises about 10-30 mM Tris-HCl and about 4-5, such as 4 or 5 M NaCl. In some embodiments a buffer B comprises about 20 mM Tris-HCl and about 4 M NaCl. In some embodiments a conditioning buffer can be buffer A and buffer B. In some embodiments buffer A comprises about 20 mM Tris-HCl and about 0.5 M NaCl and buffer B comprises about 20 mM Tris-HCl and about 4 M NaCl.

The invention is not particularly limited to buffers having a certain pH, the pH of a buffer can rather be any pH that is feasible for purification of fucoidan. In general, it is desired that buffers that come in contact with fucoidan have a pH in which fucoidan is stable. In some embodiments a buffer of the present invention can have a pH of about 5-10. In some embodiments a buffer of the present invention can have a pH of about 5, 6, 7, 8, 9, or 10. In a preferred embodiment, a buffer of the present invention has a pH of about 7.5-8.5. In another preferred embodiment, a buffer of the present invention has a pH of about 8. In some embodiments a conditioning buffer of the present invention can have a pH of about 5-10. In some embodiments a conditioning buffer of the present invention can have a pH of about 5, 6, 7, 8, 9, or 10. In a preferred embodiment a conditioning buffer of the present invention can have a pH of about 7.5-8.5. In a preferred embodiment a conditioning buffer of the present invention can have a pH of about 8. In some embodiments, buffer A can have a pH of about 5-10. In some embodiments, buffer A can have a pH of about 5, 6, 7, 8, 9, or 10. In a preferred embodiment, buffer A can have a pH of about 7.5-8.5. In a preferred embodiment, buffer A can have a pH of about 8. In some embodiments, buffer B can have a pH of about 5-10. In some embodiments, buffer B can have a pH of about 5, 6, 7, 8, 9, or 10. In a preferred embodiment, buffer B can have a pH of about 7.5-8.5. In a preferred embodiment, buffer B can have a pH of about 8.

Accordingly, in some embodiments buffer A comprises about 10-30 mM Tris-HCl and about 0.085-0.7 M NaCl at a pH of about 5-10. In some embodiments a buffer A comprises about 10, 11, 12, 13, 14, 15, 16, 17, 18, 19, 20, 21, 22, 23, 24, 25, 26, 27, 28, 29, or 30 mM Tris-HCl and about 0.05, 0.06, 0.07, 0.08, 0.09, 0.1, 0.11, 0.12, 0.13, 0.14, 0.15, 0.16, 0.17, 0.18, 0.19, 0.2, 0.21, 0.22, 0.23, 0.24, 0.25, 0.26, 0.27, 0.28, 0.29, 0.3, 0.31, 0.32, 0.33, 0.34, 0.35, 0.36, 0.37, 0.38, 0.39, 0.4, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.66, 0.67, 0.68, 0.69, 0.7, 0.71, 0.72, 0.73, 0.74, 0.75, 0.76, 0.77, 0.78, 0.79, 0.8, 0.81, 0.82, 0.83, 0.84, 0.85, 0.86, 0.87, 0.88, 0.89, 0.9, 0.91, 0.92, 0.93, 0.94, 0.95, 0.96, 0.97, 0.98, 0.99, or 1 M NaCl at a pH of about 5, 6, 7, 8, 9, or 10. In some embodiments buffer A comprises about 10-30 mM Tris-HCl and about 0.4-0.7 M NaCl at a pH of about 7.5-8.5. In some embodiments a buffer A comprises about 20 mM Tris-HCl and about 0.5 M NaCl at a pH of about 8. In some embodiments a buffer B comprises about 10-30 mM Tris-HCl and about 1-10 M NaCl at a pH of about 5-10. In some embodiments a buffer B comprises about 10, 11, 12, 13, 14, 15, 16, 17, 18, 19, 20, 21, 22, 23, 24, 25, 26, 27, 28, 29, or 30 mM Tris-HCl and about 1, 2, 3, 4, 5, 6, 7, 8, 9, or 10 M NaCl at a pH of about 5, 6, 7, 8, 9, or 10. In some embodiments a buffer B comprises about 10-30 mM Tris-HCl and about 4-5 M NaCl at a pH of about 7.8-8.5. In some embodiments a buffer B comprises about 20 mM Tris-HCl and about 4 M NaCl at a pH of about 8. In some embodiments buffer A comprises about 20 mM Tris-HCl and about 0.5 M NaCl at a pH of about 8 and buffer B comprises about 20 mM Tris-HCl and about 4 M NaCl at a pH of about 8.

In some embodiments, buffer A consists of about 20 mM Tris-HCl, about 0.5 M NaCl at about pH 8 in an aqueous solution. In some embodiments, buffer B consists of about 20 mM Tris-HCl, about 4 M NaCl at about pH 8 in an aqueous solution. In some embodiments buffer A consists of about 20 mM Tris-HCl, about 0.5 M NaCl at about pH 8 in an aqueous solution and buffer B consists of about 20 mM Tris-HCl, about 4 M NaCl at about pH 8 in an aqueous solution.

In some embodiments a buffer having a same or similar salinity/salt concentration as a sample, e.g. a sample of seawater, can have a salinity/salt concentration of about 0.05, 0.06, 0.07, 0.08, 0.09, 0.1, 0.11, 0.12, 0.13, 0.14, 0.15, 0.16, 0.17, 0.18, 0.19, 0.2, 0.21, 0.22, 0.23, 0.24, 0.25, 0.26, 0.27, 0.28, 0.29, 0.3, 0.31, 0.32, 0.33, 0.34, 0.35, 0.36, 0.37, 0.38, 0.39, 0.4, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.66, 0.67, 0.68, 0.69, 0.7, 0.71, 0.72, 0.73, 0.74, 0.75, 0.76, 0.77, 0.78, 0.79, 0.8, 0.81, 0.82, 0.83, 0.84, 0.85, 0.86, 0.87, 0.88, 0.89, 0.9, 0.91, 0.92, 0.93, 0.94, 0.95, 0.96, 0.97, 0.98, 0.99, or 1 M NaCl or about 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, 30, 30.5, 31, 31.5, 32, 32.5, 33, 33.5, 34, 34.5, 35, 35.5, 36, 36.5, 37, 37.5, 38, 38.5, 39, 39.5, or 40 grams salt per kilogram.

Accordingly, the invention provides in one aspect a method for purifying fucoidan, comprising:
(i) obtaining a sample of seawater, and
(ii) purifying fucoidan from the sample via an anion exchange chromatography column, wherein the column is conditioned with a buffer A and a buffer B.

In another aspect, the invention provides a method for purifying fucoidan, comprising:
(i) obtaining a sample of seawater, and
(ii) purifying fucoidan from the sample via an anion exchange chromatography column, wherein buffer A comprises a same or similar salinity/salt concentration as the sample of seawater.

In another aspect, the invention provides a method for purifying fucoidan, comprising:
(i) obtaining a sample of seawater, and
(ii) purifying fucoidan from the sample via an anion exchange chromatography column, further comprising conditioning the column with buffer A, followed by buffer B, followed by buffer A, optionally wherein buffer A comprises about 20 mM Tris-HCl, about 0.5-0.7 M NaCl at about pH 8, and wherein buffer B comprises about 20 mM Tris-HCl, about 4 M NaCl at about pH 8.

In another aspect, the invention provides a method for purifying fucoidan, comprising:
(i) obtaining a sample of seawater, and
(ii) purifying fucoidan from the sample via an anion exchange chromatography column, further comprising conditioning the column with about 25 ml of buffer A, followed by about 25 ml of buffer B, followed by about 25 ml of buffer A, optionally wherein buffer A comprises about 20 mM Tris-HCl, about 0.5-0.7 M NaCl at about pH 8, and wherein buffer B comprises about 20 mM Tris-HCl, about 4 M NaCl at about pH 8.

In another aspect, the invention provides a method for purifying fucoidan, comprising:
(i) obtaining a sample of seawater, and
(ii) purifying fucoidan from the sample via an anion exchange chromatography column, wherein buffer A consists of 20 mM Tris-HCl, 0.5 M NaCl at pH 8, and wherein buffer B consists of 20 mM Tris-HCl, 4 M NaCl at pH 8, optionally wherein buffer A and B are an aqueous solution.

The method for purifying and/or quantifying fucoidan of the present invention can further comprise a step of applying a sample, e.g. a sample of seawater, onto an anion exchange chromatography column, e.g. an anion exchange chromatography column conditioned according to the present invention. As used herein "applying a sample" or "applying a sample of seawater" can be used interchangeably with "an application step", "a step of applying a sample", "a step of applying seawater", "a step of applying a sample of seawater" or "an application step comprising applying a sample of seawater". A particular advantage of the present invention is that a sample of seawater does not have to be diluted before applying the same onto the column. This can be realized by the buffer compositions described herein above, which enable binding of fucoidan onto the column from the undiluted sample. This is particularly advantages since it simplifies the purification from e.g. a sample of seawater. In some embodiments of the methods discloses herein, a sample of seawater is undiluted. In some embodiments a sample of seawater is not diluted e.g. with ultrapure/purified water. In some embodiments, the application step comprises applying 1-1000 ml of the sample of seawater onto the conditioned column.

Accordingly, the invention provides in one aspect a method for purifying fucoidan, comprising:
(i) obtaining a sample of seawater, and
(ii) purifying fucoidan from the sample via an anion exchange chromatography column, further comprising applying the sample of seawater onto the conditioned column.

In another aspect, the invention provides a method for purifying fucoidan, comprising:
(i) obtaining a sample of seawater, and
(ii) purifying fucoidan from the sample, wherein the sample of seawater is undiluted or, wherein the sample of seawater is not diluted.

In another aspect, the invention provides a method for purifying fucoidan, comprising:
(i) obtaining a sample of seawater, and
(ii) purifying fucoidan from the sample via an anion exchange chromatography column, further comprising applying the sample of seawater onto the conditioned column, wherein the sample of seawater is undiluted or, wherein the sample of seawater is not diluted.

In another aspect, the invention provides a method for purifying fucoidan, comprising:
(i) obtaining a sample of seawater, and
(ii) purifying fucoidan from the sample via an anion exchange chromatography column, further comprising applying 1-1000 ml of the sample of seawater onto the conditioned column,

The method for purifying and/or quantifying fucoidan of the present invention can further comprise a washing step. A washing step can be applied to the anion exchange chromatography column to dissolve and elute any undesired substances/compounds/particles, e.g. undesired organic compounds. A washing step can be performed by applying e.g. a washing buffer onto the anion exchange chromatography column. It is desired that a washing buffer resembles the salinity/salt concentration of the sample from which fucoidan is to be purified, e.g. a sample of seawater as described herein. Accordingly, the features described herein above in context of adjusting the salinity/salt concentration of a buffer to that of a sample apply mutatis mutandis to a washing buffer of the present invention. The terms "wash buffer" or "washing buffer" can be used interchangeably herein. In some embodiments a washing buffer resembles the salinity/salt concentration of the sample of seawater. In some embodiments, a washing buffer has a same or similar salinity/salt concentration as the sample of seawater. In some embodiments, the salinity/salt concentration in the sample of seawater and the washing buffer is the same. In some embodiments, the salinity/salt concentration in the sample of seawater and the washing buffer is identical. In some embodiments, a washing step further comprises measuring the salinity/salt concentration in the sample of seawater and adjusting the salinity/salt concentration of the wash buffer to correspond to that of the sample of seawater. In some embodiments the washing step removes/dissolves/solves undesired substances/compounds/particles, e.g. undesired organic compounds from the sample of seawater. In some embodiments the washing step removes/dissolves/solves undesired substances/compounds/particles, e.g. undesired organic compounds from the sample of seawater by eluting them from the anion exchange chromatography column.

In some embodiments the washing step comprises applying a volume of wash buffer onto the anion exchange chromatography column. In some embodiments the washing step comprises applying about 1-5 column volumes, such as 1, 2, 3, 4, or 5 column volumes of wash buffer onto the anion exchange chromatography column. As used herein "column volumes of a buffer" relates to a volume of a buffer defined via the total volume capacity of a column. For example, 10 column volumes of wash buffer can be 100 ml if the column as ah total volume of 10 ml. In some embodiments, the washing step comprises applying about 5-50 ml of washing buffer onto an anion exchange chromatography column. In some embodiments, the washing step comprises applying about 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 ml of washing buffer onto an anion exchange chromatography column. In some embodiments, the washing step can be repeated. In some embodiments, the washing step can be repeated 1-10, such as 1, 2, 3, 4, 5, 6, 7, 8, 9, or 10 times.

In some embodiments a washing buffer comprises about 10-30 mM Tris-HCl and about 0.085-0.7 NaCl. In some embodiments a washing buffer comprises about 10, 11, 12, 13, 14, 15, 16, 17, 18, 19, 20, 21, 22, 23, 24, 25, 26, 27, 28, 29, or 30 mM Tris-HCl and about 0.085, 0.09, 0.095, 0.1, 0.15, 0.2, 0.25, 0.3, 0.31, 0.32, 0.33, 0.34, 0.35, 0.36, 0.37, 0.38, 0.39, 0.4, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.66, 0.67, 0.68, 0.69, or 0.7 M NaCl. In some embodiments a washing buffer comprises about 20 mM Tris-HCl and about 0.5 M NaCl.

The invention is not particularly limited to washing buffers having a certain pH, the pH of a washing buffer can rather be any pH that is feasible for eluting undesired compounds. In general, it is desired that a washing buffer that comes in contact with fucoidan has a pH in which fucoidan is stable. It is further desired that the washing buffer is not able to elute fucoidan from the anion exchange chromatography column. In some embodiments a washing buffer of the present invention can have a pH of about 5-10. In some embodiments a washing buffer of the present invention can have a pH of about 5, 6, 7, 8, 9, or 10. In a preferred embodiment, a washing buffer of the present invention has a pH of about 8.

In some embodiments, a washing buffer consists of about 20 mM Tris-HCl, about 0.5 M NaCl at about pH 8 in an aqueous solution.

In some embodiments a washing buffer having a same or similar salinity/salt concentration as a sample, e.g. sample of seawater, can have a salinity/salt concentration of about 0.085, 0.09, 0.095, 0.1, 0.15, 0.2, 0.25, 0.3, 0.31, 0.32, 0.33, 0.34, 0.35, 0.36, 0.37, 0.38, 0.39, 0.4, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.66, 0.67, 0.68, 0.69, or 0.7 M NaCl or about 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, 30, 30.5, 31, 31.5, 32, 32.5, 33, 33.5, 34, 34.5, 35, 35.5, 36, 36.5, 37, 37.5, 38, 38.5, 39, 39.5, or 40 grams salt per kilogram. In some embodiments, the salinity/salt concentration of a washing buffer is identical to that of a sample, e.g. sample of seawater.

In some embodiments a washing buffer is buffer A.

Accordingly, the invention provides in one aspect a method for purifying fucoidan, comprising:
(i) obtaining a sample of seawater, and
(ii) purifying fucoidan from the sample via an anion exchange chromatography column, further comprising a washing step.

In another aspect, the invention provides a method for purifying fucoidan, comprising:
(i) obtaining a sample of seawater, and
(ii) purifying fucoidan from the sample via an anion exchange chromatography column, wherein the washing step comprises applying a volume of wash buffer onto the column.

In another aspect, the invention provides a method for purifying fucoidan, comprising:
(i) obtaining a sample of seawater, and
(ii) purifying fucoidan from the sample via an anion exchange chromatography column, comprising applying a volume of wash buffer onto the column, wherein the salinity/salt concentration of the wash buffer resembles the salinity/salt concentration of the sample of seawater.

In another aspect, the invention provides a method for purifying fucoidan, comprising:
(i) obtaining a sample of seawater, and
(ii) purifying fucoidan from the sample via an anion exchange chromatography column, comprising applying a volume of wash buffer onto the column, wherein the wash buffer comprises a same or similar salinity/salt concentration as the sample of seawater.

In another aspect, the invention provides a method for purifying fucoidan, comprising:
(i) obtaining a sample of seawater, and
(ii) purifying fucoidan from the sample via an anion exchange chromatography column, further comprising a washing step, wherein the washing step removes undesired contaminants from the sample of seawater, optionally wherein contaminants are eluted by the wash buffer. In another aspect, the invention provides a method for purifying fucoidan, comprising:
   (i) obtaining a sample of seawater, and
   (ii) purifying fucoidan from the sample via an anion exchange chromatography column, further comprising a washing step, wherein the washing step comprises washing the column with about 1-5 column volumes, such as 1, 2, 3, 4, or 5 column volumes of washing buffer, optionally, wherein the washing step is repeated 1-10 times such as 1, 2, 3, 4, 5, 6, 7, 8, 9, or 10 times.

In another aspect, the invention provides a method for purifying fucoidan, comprising:
(i) obtaining a sample of seawater, and
(ii) purifying fucoidan from the sample via an anion exchange chromatography column, further comprising a washing step, wherein the washing step comprises washing the column with about 5-50 ml, such as 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 ml of washing buffer.

In another aspect, the invention provides a method for purifying fucoidan, comprising:
(i) obtaining a sample of seawater, and
(ii) purifying fucoidan from the sample via an anion exchange chromatography column, further comprising a washing step, wherein the washing step comprises washing the column with about 5-50 ml, such as 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 ml of washing buffer, and wherein the washing step is repeated 1-10 times, such as 1, 2, 3, 4, 5, 6, 7, 8, 9, or 10 times.

In another aspect, the invention provides a method for purifying fucoidan, comprising:
(i) obtaining a sample of seawater, and
(ii) purifying fucoidan from the sample via an anion exchange chromatography column, further comprising a washing step, wherein the washing step comprises applying a volume of wash buffer onto the column, and wherein the wash buffer comprises about 10-30 mM Tris-HCl and about 0.085-0.7 NaCl at a pH of about 5-10.

In another aspect, the invention provides a method for purifying fucoidan, comprising:
(i) obtaining a sample of seawater, and
(ii) purifying fucoidan from the sample via an anion exchange chromatography column, further comprising a washing step, wherein the washing step comprises applying a volume of wash buffer onto the column, and wherein the wash buffer comprises about 10, 11, 12, 13, 14, 15, 16, 17, 18, 19, 20, 21, 22, 23, 24, 25, 26, 27, 28, 29, or 30 mM Tris-HCl and about 0.085, 0.09, 0.095, 0.1, 0.15, 0.2, 0.25, 0.3, 0.31, 0.32, 0.33, 0.34, 0.35, 0.36, 0.37, 0.38, 0.39, 0.4, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.66, 0.67, 0.68, 0.69, or 0.7 M NaCl at a pH of about 5, 6, 7, 8, 9, or 10.

In a preferred aspect, the invention provides a method for purifying fucoidan, comprising:
(i) obtaining a sample of seawater, and
(ii) purifying fucoidan from the sample via an anion exchange chromatography column, further comprising a washing step, wherein the washing step comprises applying a volume of wash buffer onto the column, and wherein the wash buffer comprises about 20 mM Tris-HCl, about 0.5 M NaCl at about pH 8.

In another preferred aspect, the invention provides a method for purifying fucoidan, comprising:
(i) obtaining a sample of seawater, and
(ii) purifying fucoidan from the sample via an anion exchange chromatography column, further comprising a washing step, wherein the washing step comprises applying a volume of wash buffer onto the column, and wherein the wash buffer consists of 20 mM Tris-HCl, 0.5 M NaCl at pH 8 in an aqueous solution.

In another aspect, the invention provides a method for purifying fucoidan, comprising:
(i) obtaining a sample of seawater, and
(ii) purifying fucoidan from the sample via an anion exchange chromatography column, wherein the method further comprises measuring the salinity/salt concentration in the sample of seawater and adjusting the salinity/salt concentration of the wash buffer to correspond to that of the sample of seawater.

In another aspect, the invention provides a method for purifying fucoidan, comprising:
(i) obtaining a sample of seawater, and
(ii) purifying fucoidan from the sample via an anion exchange chromatography column, wherein the method further comprises measuring the salinity/salt concentration in the sample of seawater and adjusting the salinity/salt concentration of the wash buffer to correspond to that of the sample of seawater before commencing the washing step.

The method for purifying and/or quantifying fucoidan of the present invention can further comprise an elution step. In general, an elution step can be performed to detach fucoidan bound to the anion exchange chromatography column thereby eluting the fucoidan from the column. An elution step can comprise applying a volume of elution buffer to an anion exchange chromatography column. The invention is not particularly limited to any specific elution buffer volumes or elution buffer composition, rather any elution buffer volume or composition may be used that is able to unbind/detach and elute fucoidan from the anion exchange chromatography column. In some embodiments the elution step can comprise applying a volume of elution buffer to an anion exchange chromatography column. In some embodiments the elution step comprises applying about 1-5 column volumes, such as 1, 2, 3, 4, or 5 column volumes of elution buffer onto the anion exchange chromatography column. In some embodiments, the elution step comprises applying about 5-50 ml of elution buffer onto an anion exchange chromatography column. In some embodiments, the elution step comprises applying about 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 ml of elution buffer onto an anion exchange chromatography column. In some embodiments, the elution step can be repeated. In some embodiments, the elution step can be repeated 1-10, such as 1, 2, 3, 4, 5, 6, 7, 8, 9, or 10, times. It is also envisioned that the elution step can be repeated with only one volume of elution buffer, e.g. one volume is used to be eluted and subsequently collected, the same volume is then applied to the column again to elute a second or further time. In some embodiments the elution step can be repeated with a single, e.g. the same, volume of elution buffer. In some embodiments the elution step can be repeated with a single, e.g. the same, volume of elution buffer 1-10, such as 1, 2, 3, 4, 5, 6, 7, 8, 9, or 10, times. In some embodiments, the elution step comprises incubating the elution buffer for a certain time in the anion exchange chromatography column. In some embodiments, the elution step comprises incubating the elution buffer for about 1-30 min in the anion exchange chromatography column.

In some embodiments an elution buffer comprises about 10-30 mM Tris-HCl and about 0.085-0.7 NaCl. In some embodiments an elution buffer comprises about 10, 11, 12, 13, 14, 15, 16, 17, 18, 19, 20, 21, 22, 23, 24, 25, 26, 27, 28, 29, or 30 mM Tris-HCl and about 1, 2, 3, 4, 5, 6, 7, 8, 9, or 10 M NaCl. In some embodiments an elution buffer comprises about 20 mM Tris-HCl and about 4 M NaCl.

The invention is not particularly limited to elution buffers having a certain pH, the pH of a elution buffer can rather be any pH that is feasible for eluting fucoidan. In general, it is desired that an elution buffer that comes in contact with fucoidan has a pH in which fucoidan is stable. It is further desired that the elution buffer is able to elute fucoidan from an anion exchange chromatography column. In some embodiments an elution buffer of the present invention can have a pH of about 5-10. In some embodiments an elution buffer of the present invention can have a pH of about 5, 6, 7, 8, 9, or 10. In a preferred embodiment, an elution buffer of the present invention has a pH of about 8. In some embodiments, an elution buffer consists of about 20 mM Tris-HCl, about 4 M NaCl at about pH 8. In some embodiments, an elution buffer consists of about 20 mM Tris-HCl, about 4 M NaCl at about pH 8 in an aqueous solution. In some embodiments an elution buffer is buffer B.

Accordingly, the invention provides in one aspect a method for purifying fucoidan, comprising:
(i) obtaining a sample of seawater, and
(ii) purifying fucoidan from the sample via an anion exchange chromatography column, further comprising an elution step.

In another aspect, the invention provides a method for purifying fucoidan, comprising:
(i) obtaining a sample of seawater, and
(ii) purifying fucoidan from the sample via an anion exchange chromatography column, further comprising an elution step comprising applying a volume of elution buffer to the column.

In another aspect, the invention provides a method for purifying fucoidan, comprising:
(i) obtaining a sample of seawater, and
(ii) purifying fucoidan from the sample via an anion exchange chromatography column, further comprising an elution step comprising applying a volume of elution buffer to the column, wherein the elution buffer comprises about 10-30 mM Tris-HCl and about 1-10 NaCl at a pH of about 5-10.

In another aspect, the invention provides a method for purifying fucoidan, comprising:
(i) obtaining a sample of seawater, and
(ii) purifying fucoidan from the sample via an anion exchange chromatography column, further comprising an elution step comprising applying a volume of elution buffer to the column, wherein the elution buffer comprises about 10, 11, 12, 13, 14, 15, 16, 17, 18, 19, 20, 21, 22, 23, 24, 25, 26, 27, 28, 29, or 30 mM Tris-HCl and about 1, 2, 3, 4, 5, 6, 7, 8, 9, or 10 M at a pH of about 5-10.

In a preferred aspect, the invention provides a method for purifying fucoidan, comprising:
(i) obtaining a sample of seawater, and
(ii) purifying fucoidan from the sample via an anion exchange chromatography column, further comprising an elution step comprising applying a volume of elution buffer to the column, wherein the elution buffer comprises about 20 mM Tris-HCl, about 4 M NaCl at about pH 8.

In another preferred aspect, the invention provides a method for purifying fucoidan, comprising:
(i) obtaining a sample of seawater, and
(ii) purifying fucoidan from the sample via an anion exchange chromatography column, further comprising an elution step comprising applying a volume of elution buffer to the column, wherein the elution buffer consists of about 20 mM Tris-HCl, about 4 M NaCl at about pH 8 in an aqueous solution.

In another aspect, the invention provides a method for purifying fucoidan, comprising:
(i) obtaining a sample of seawater, and
(ii) purifying fucoidan from the sample via an anion exchange chromatography column, further comprising an elution step, wherein the elution step comprises applying about 5-50 ml, such as about 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 ml, of elution buffer onto the column.

In another aspect, the invention provides a method for purifying fucoidan, comprising:
(i) obtaining a sample of seawater, and
(ii) purifying fucoidan from the sample via an anion exchange chromatography column, further comprising an elution step, wherein the elution step comprises applying about 1-5 column volumes, such as 1, 2, 3, 4, or 5 column volumes of elution buffer onto the column, optionally wherein the elution step is repeated 1-10 times, such as 1, 2, 3, 4, 5, 6, 7, 8, 9, or 10 times

In another aspect, the invention provides a method for purifying fucoidan, comprising:
(i) obtaining a sample of seawater, and
(ii) purifying fucoidan from the sample via an anion exchange chromatography column, further comprising an elution step, wherein the elution step comprises applying about 5-50 ml, such as about 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 ml, of elution buffer onto the column and wherein the elution step is repeated 1-10 times, such as 1, 2, 3, 4, 5, 6, 7, 8, 9, or 10 times

In another aspect, the invention provides a method for purifying fucoidan, comprising:
(i) obtaining a sample of seawater, and
(ii) purifying fucoidan from the sample via an anion exchange chromatography column, further comprising an elution step, wherein the elution step comprises applying about 5-50 ml, such as about 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 ml, of elution buffer onto the column and wherein the elution step is repeated with a single, e.g. the same, volume of elution buffer 1-10, such as 1, 2, 3, 4, 5, 6, 7, 8, 9, or 10, times

The method for purifying and/or quantifying fucoidan of the present invention can further comprise an optional reconditioning step. Such an optional reconditioning step can be performed onto the eluted anion exchange chromatography column to store or reuse the column. For this, the column can be rinsed with buffer B followed by ultrapure water, followed by 1 M NaOH at a reduced flow rate, followed by ultrapure water. For example, the column can be rinsed with about 1-5 column volumes of buffer B, followed by 1-5 column volumes of ultrapure water, followed by 1-5 column volumes of 1 M NaOH at reduced flow rate, followed by 1-5 column volumes of ultrapure water. As used herein, "ultrapure water" in general relates to water with minimal amounts of salts or no salts, such as deionized water (diH₂O), distilled water (dH₂O), double-distilled water (ddH₂O) or water purified using a Millipore Milli-Q lab water system (Milli-Q water). In some embodiments, the reduced flow rate is 1 ml/min.

Accordingly, the invention provides in one aspect a method a method for purifying fucoidan, comprising:
(i) obtaining a sample of seawater, and
(ii) purifying fucoidan from the sample via an anion exchange chromatography column, further comprising a reconditioning step, wherein the column is rinsed with about 1-5 column volumes of buffer B, followed by 1-5 column volumes of ultrapure water, followed by 1-5 column volumes of 1 M NaOH at reduced flow rate, followed by 1-5 column volumes of ultrapure water.

The invention also provides means for precipitating fucoidan from a sample, e.g. a sample of seawater, thereby purifying the fucoidan. Accordingly, the invention provides a method for purifying fucoidan, comprising (i) obtaining a sample of seawater, and (ii) purifying fucoidan from the sample, wherein step (ii) comprises a precipitation step. In general, the precipitation step can be performed independently from, or in combination with the purification via an anion exchange chromatography column. In particular, it is envisioned, that an elution of fucoidan obtained by the purification via an anion exchange chromatography column as described herein above can be further subjected to a precipitation. Alternatively, the purification of fucoidan can be performed via precipitation followed by the purification via an anion exchange chromatography column as described herein above. The purification of fucoidan can also be performed via precipitation e.g. without performing a purification via an anion exchange chromatography column. Accordingly, a precipitation step in the sense of the present invention can comprise reducing the salinity/salt concentration in the sample of seawater. The sample of seawater can be for example be diluted with ultrapure water, such as deionized water (diHzO), distilled water (dHzO), double-distilled water (ddHzO) or water purified using a Millipore Milli-Q lab water system (Milli-Q water). In some embodiments a sample is diluted with one sample volume of ultrapure water. In some embodiments a sample is diluted 1:1 with ultrapure water. In some embodiments a sample of seawater is diluted 1:1 with ultrapure water. In some embodiments, seawater is diluted 1:1 with ultrapure water. Precipitation of fucoidan can be induced by addition of metal ions, addition of alcohols, addition of amphiphilic compounds, or by lowering the pH. Exemplary metal ions can be magnesium, iron, or aluminum. Exemplary alcohols can be ethanol or 2-propanol. The precipitation step can comprise adding an amphiphilic compound to the sample of seawater. The invention is not particularly limited to any amphiphilic compound, rather any amphiphilic compound can be used that is able to form fucoidan precipitate in the sample, e.g. sample of seawater. Exemplary amphiphilic compounds are soaps, detergents, surfactants, phospholipids, cholesterol, glycolipids, fatty acids, bile acids, saponins or quaternary ammonium surfactants. In some embodiments an amphiphilic compound is a quaternary ammonium surfactant. In some embodiments an amphiphilic compound is cetyltrimethylammonium bromide. In general, an amphiphilic compound may be added in any amount that is able to cause the formation of fucoidan precipitates in the sample, e.g. sample of seawater. Accordingly, in some embodiments, the concentration of the amphiphilic compound in the sample, e.g. sample of seawater can be about 0.01-1 g/l. In some embodiments, the concentration of the amphiphilic compound in the sample, e.g. sample of seawater can be about 0.01, 0.02, 0.03, 0.04, 0.05, 0.06, 0.07, 0.08, 0.09, 0.1, 0.11, 0.12, 0.13, 0.14, 0.15, 0.16, 0.17, 0.18, 0.19, 0.2, 0.21, 0.22, 0.23, 0.24, 0.25, 0.26, 0.27, 0.28, 0.29, 0.3, 0.31, 0.32, 0.33, 0.34, 0.35, 0.36, 0.37, 0.38, 0.39, 0.4, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.66, 0.67, 0.68, 0.69, 0.7, 0.71, 0.72, 0.73, 0.74, 0.75, 0.76, 0.77, 0.78, 0.79, 0.8, 0.81, 0.82, 0.83, 0.84, 0.85, 0.86, 0.87, 0.88, 0.89, 0.9, 0.91, 0.92, 0.93, 0.94, 0.95, 0.96, 0.97, 0.98, 0.99, or 1 g/l. In a preferred embodiment, the concentration of the amphiphilic compound in the sample, e.g. sample of seawater can be about 0.1 g/l.

In some embodiments, the precipitation step comprises adding a quaternary ammonium surfactant to the sample, e.g. sample of seawater. In some embodiments, the precipitation step comprises adding a quaternary ammonium surfactant to the sample of seawater, wherein the concentration of the quaternary ammonium surfactant in the sample, e.g. sample of seawater, can be about 0.01, 0.02, 0.03, 0.04, 0.05, 0.06, 0.07, 0.08, 0.09, 0.1, 0.11, 0.12, 0.13, 0.14, 0.15, 0.16, 0.17, 0.18, 0.19, 0.2, 0.21, 0.22, 0.23, 0.24, 0.25, 0.26, 0.27, 0.28, 0.29, 0.3, 0.31, 0.32, 0.33, 0.34, 0.35, 0.36, 0.37, 0.38, 0.39, 0.4, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.66, 0.67, 0.68, 0.69, 0.7, 0.71, 0.72, 0.73, 0.74, 0.75, 0.76, 0.77, 0.78, 0.79, 0.8, 0.81, 0.82, 0.83, 0.84, 0.85, 0.86, 0.87, 0.88, 0.89, 0.9, 0.91, 0.92, 0.93, 0.94, 0.95, 0.96, 0.97, 0.98, 0.99, or 1 g/l. In a preferred embodiment, the precipitation step comprises adding a quaternary ammonium surfactant to the sample of seawater, wherein the concentration of the quaternary ammonium surfactant in the sample, e.g. sample of seawater can be about 0.1 g/l.

The precipitation step of the present invention can further comprise a filtration step, wherein the precipitated sample is filtrated. The filtration step is preferably performed after fucoidan precipitates have formed as described above. When filtrating the precipitated sample, the fucoidan precipitates remain on or in the filter. Exemplary filters that can be used in the filtration step for filtrating the precipitate are a polyethersulphone (PES) filter, cellulose acetate filter, polytetrafluoroethylene (PTFE) filter, polyamide (Nylon) filter, cellulose nitrate filter, polycarbonate filter, mixed cellulose ester filter, regenerated cellulose filter, polyvinylidene difluoride (PVDF) filter, non-woven polypropylene filter or a combusted glass fiber filter. The precipitates can subsequently be resuspended e.g. in ultrapure water, such as deionized water (diH₂O), distilled water (dH₂O), double-distilled water (ddH₂O) or water purified using a Millipore Milli-Q lab water system (Milli-Q water), e.g. for later quantification. In general, a filtration step may be performed as often as necessary to separate the fucoidan precipitates from the sample, e.g. sample of seawater. In some embodiments, a filtration step is repeated. In some embodiments, a filtration step is repeated 1, 2, 3, 4, 5, 6, 7, 8, 9, or 10 times. It is preferred herein, that the filtration step in the context of the precipitation step is repeated with the same filter, i.e. without exchanging filters between repeated filtration steps. In some embodiments, the precipitation step can comprise alternatively to the filtration step or in addition to the filtration step a centrifugation step, wherein the fucoidan precipitate is separated. The separated fucoidan precipitate can be resuspended. The precipitation step can further comprise resuspending the fucoidan precipitate in ultrapure water, such as deionized water (diH₂O), distilled water (dH₂O), double-distilled water (ddH₂O) or water purified using a Millipore Milli-Q lab water system (Milli-Q water).

Accordingly, the invention provides in one aspect a method for purifying fucoidan, comprising:
(i) obtaining a sample of seawater, and
(ii) purifying fucoidan from the sample, comprising a precipitation step.

In another aspect, the invention provides a method for purifying fucoidan, comprising:
(i) obtaining a sample of seawater, and
(ii) purifying fucoidan from the sample, comprising a precipitation step, wherein fucoidan in the sample of seawater is precipitated.

In another aspect, the invention provides a method for purifying fucoidan, comprising:
(i) obtaining a sample of seawater, and
(ii) purifying fucoidan from the sample, comprising a precipitation step, wherein the sample of seawater is diluted with ultrapure water.

In another aspect, the invention provides a method for purifying fucoidan, comprising:
(i) obtaining a sample of seawater, and
(ii) purifying fucoidan from the sample, comprising a precipitation step, wherein the sample of seawater is diluted 1:1 with ultrapure water.

In another aspect, the invention provides a method for purifying fucoidan, comprising:
(i) obtaining a sample of seawater, and
(ii) purifying fucoidan from the sample, comprising a precipitation step, wherein the sample of seawater is diluted with ultrapure water, and wherein an amphiphilic compound is added to the sample of seawater.

In another aspect, the invention provides a method for purifying fucoidan, comprising:
(i) obtaining a sample of seawater, and
(ii) purifying fucoidan from the sample, comprising a precipitation step, wherein the sample of seawater is diluted with ultrapure water, and wherein an amphiphilic compound is added to the sample of seawater, wherein the concentration of the amphiphilic compound in the sample of seawater is about 0.01-1 g/l, such as about 0.01, 0.02, 0.03, 0.04, 0.05, 0.06, 0.07, 0.08, 0.09, 0.1, 0.11, 0.12, 0.13, 0.14, 0.15, 0.16, 0.17, 0.18, 0.19, 0.2, 0.21, 0.22, 0.23, 0.24, 0.25, 0.26, 0.27, 0.28, 0.29, 0.3, 0.31, 0.32, 0.33, 0.34, 0.35, 0.36, 0.37, 0.38, 0.39, 0.4, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.66, 0.67, 0.68, 0.69, 0.7, 0.71, 0.72, 0.73, 0.74, 0.75, 0.76, 0.77, 0.78, 0.79, 0.8, 0.81, 0.82, 0.83, 0.84, 0.85, 0.86, 0.87, 0.88, 0.89, 0.9, 0.91, 0.92, 0.93, 0.94, 0.95, 0.96, 0.97, 0.98, 0.99, or 1 g/l.

In a preferred aspect, the invention provides a method for purifying fucoidan, comprising:
(i) obtaining a sample of seawater, and
(ii) purifying fucoidan from the sample, comprising a precipitation step, wherein the sample of seawater is diluted with ultrapure water, and wherein an amphiphilic compound is added to the sample of seawater, wherein the concentration of the amphiphilic compound in the sample of seawater is about 0.1 g/l.

In another aspect, the invention provides a method for purifying fucoidan, comprising:
(i) obtaining a sample of seawater, and
(ii) purifying fucoidan from the sample, comprising a precipitation step, wherein the sample of seawater is diluted with ultrapure water, and wherein metal ions, alcohols, low pH acids, or quaternary ammonium surfactants are added to the sample of seawater.

In another aspect, the invention provides a method for purifying fucoidan, comprising:
(i) obtaining a sample of seawater, and
(ii) purifying fucoidan from the sample, comprising a precipitation step, wherein the sample of seawater is diluted with ultrapure water, and wherein quaternary ammonium surfactants are added to the sample of seawater, wherein the concentration of the quaternary ammonium surfactants in the sample of seawater is about 0.01-1 g/l, such as about 0.01, 0.02, 0.03, 0.04, 0.05, 0.06, 0.07, 0.08, 0.09, 0.1, 0.11, 0.12, 0.13, 0.14, 0.15, 0.16, 0.17, 0.18, 0.19, 0.2, 0.21, 0.22, 0.23, 0.24, 0.25, 0.26, 0.27, 0.28, 0.29, 0.3, 0.31, 0.32, 0.33, 0.34, 0.35, 0.36, 0.37, 0.38, 0.39, 0.4, 0.41, 0.42, 0.43, 0.44, 0.45, 0.46, 0.47, 0.48, 0.49, 0.5, 0.51, 0.52, 0.53, 0.54, 0.55, 0.56, 0.57, 0.58, 0.59, 0.6, 0.61, 0.62, 0.63, 0.64, 0.65, 0.66, 0.67, 0.68, 0.69, 0.7, 0.71, 0.72, 0.73, 0.74, 0.75, 0.76, 0.77, 0.78, 0.79, 0.8, 0.81, 0.82, 0.83, 0.84, 0.85, 0.86, 0.87, 0.88, 0.89, 0.9, 0.91, 0.92, 0.93, 0.94, 0.95, 0.96, 0.97, 0.98, 0.99, or 1 g/l.

In another preferred aspect, the invention provides a method for purifying fucoidan, comprising:
(i) obtaining a sample of seawater, and
(ii) purifying fucoidan from the sample, comprising a precipitation step, wherein the sample of seawater is diluted with ultrapure water, and wherein quaternary ammonium surfactants are added to the sample of seawater, wherein the concentration of the quaternary ammonium surfactants in the sample of seawater is 0.1 g/l.

In another aspect, the invention provides a method for purifying fucoidan, comprising:
(i) obtaining a sample of seawater, and
(ii) purifying fucoidan from the sample, comprising a precipitation step, wherein the sample of seawater is diluted with ultrapure water, and wherein an amphiphilic compound is added to the sample of seawater, wherein the concentration of the amphiphilic compound in the sample of seawater is about 0.01-1 g/l, further comprising a filtration step comprising filtrating the sample of seawater through a filter, such as a polyethersulphone (PES) filter, cellulose acetate filter, polytetrafluoroethylene (PTFE) filter, polyamide (Nylon) filter, cellulose nitrate filter, polycarbonate filter, mixed cellulose ester filter, regenerated cellulose filter, polyvinylidene difluoride (PVDF) filter, non-woven polypropylene filter or a combusted glass fiber filter.

In another aspect, the invention provides a method for purifying fucoidan, comprising:
(i) obtaining a sample of seawater, and
(ii) purifying fucoidan from the sample, comprising a precipitation step, wherein the sample of seawater is diluted with ultrapure water, and wherein an amphiphilic compound is added to the sample of seawater, wherein the concentration of the amphiphilic compound in the sample of seawater is about 0.01-1 g/l, further comprising a filtration step comprising filtrating the sample of seawater through a filter, such as a polyethersulphone (PES) filter, cellulose acetate filter, polytetrafluoroethylene (PTFE) filter, polyamide (Nylon) filter, cellulose nitrate filter, polycarbonate filter, mixed cellulose ester filter, regenerated cellulose filter, polyvinylidene difluoride (PVDF) filter, non-woven polypropylene filter or a combusted glass fiber filter, further comprising an elution or resuspension step, wherein the precipitated fucoidan is eluted or resuspended from the filter.

The present invention also provides a method for quantifying fucoidan. Any of the methods for purifying fucoidan can further comprise a step (iii) quantifying fucoidan in a sample, e.g. sample of seawater, eluted sample, or precipitated sample. In some embodiments step (iii) quantifying fucoidan comprised in a method for purifying fucoidan can comprise a method for quantifying fucoidan as disclosed herein. As used herein "purified sample" in general relates to a sample that predominantly comprises the polysaccharide fucoidan. A "purified sample" may preferably be a sample comprising fucoidan obtained from the elution and/or precipitation step as described above. Quantification of fucoidan in e.g. a purified sample can be achieved by different means. The methods for purifying fucoidan of the present invention are particularly advantageous for quantification as they allow for more robust and reliable quantification of fucoidan. In particular, the purification of fucoidan before quantification avoids quantification of contaminants, e.g. other organic compounds such as other polysaccharides or monosaccharides.

Fucoidan can be quantified by different means, for example, mass spectrometry, gas chromatography, liquid chromatography, capillary electrophoresis, high-performance liquid chromatography, anion exchange chromatography with pulsed amperometric detection (HPAEC-PAD), size exclusion chromatography, acid hydrolysis followed by monosaccharide quantification, acid hydrolysis of sulfated fucans, acid hydrolysis followed by nuclear magnetic resonance (NMR) spectroscopy, or carbohydrate-polyacrylamide gel electrophoresis (C-PAGE) analyses (see e.g. 104).

The inventors have found a novel method to specifically quantify fucoidan in a sample of seawater, or a purified sample. Fucoidan can be degraded by specialized bacteria that require dozens of different enzymes. For example, *Lentimonas sp.* CC4 produces enzymes that can degrade fucoidan. These enzymes can be e.g. obtained from culturing *Lentimonas sp.* CC4. The bacteria can further be lysed followed by separation of the cellular fraction and supernatant comprising the desired fucoidan-degrading enzymes. The fucoidan-degrading enzymes degrade fucoidan into monosaccharides. Degradation of fucoidan with these fucoidan-degrading enzymes is particularly advantageous since due to the specificity to fucoidan, other compounds in a sample, e.g. organic compounds or polysaccharides other than fucoidan are not degraded. This allows for robust and reliable quantification of fucoidan e.g. by quantifying the obtained monosaccharides. Quantification of the fucoidan can be realized by comparison of the yield obtained by digestion of a sample with fucoidan-degrading enzymes. In particular, a fucoidan standard or reference sample as disclosed herein can be used to digest known amounts/quantities of fucoidan with the fucoidan-degrading enzymes, the resulting monosaccharides can subsequently be quantified. This can be repeated for different amounts/quantities of fucoidan in a fucoidan standard or reference sample. The resulting data quantification data for different amounts/quantities of fucoidan in the standard or reference sample can be used to generate a fucoidan standard curve. This standard curve can be used to quantify fucoidan in any desired sample, e.g. sample of seawater, according to the enzymatic assay disclosed herein. In some embodiments the enzyme assay as disclosed herein can be comprised in a method for quantifying fucoidan of the present invention. In some embodiments, the enzyme assay as disclosed herein can be comprised in a (iii) quantifying fucoidan in a sample. The terms "fucoidan standard curve", "standard curve", or "calibration curve" can be used interchangeable herein.

When applying the enzymatic assay to a sample, a sample of seawater may be obtained and contacted with enzymes capable of digesting fucoidan, e.g. fucoidan-degrading enzymes obtained from culturing *Lentimonas sp.* CC4. The fucoidan-degrading enzymes then digest the fucoidan in the sample into monosaccharides, e.g. fucose. The fucose then can be quantified according to the standard means for quantifying monosaccharides as disclosed herein. The yield of fucoidan specific monosaccharides is then compared to a fucoidan standard, e.g. the fucoidan standard curve as described herein above. By comparing the yield of monosaccharides obtained from the digestion by fucoidan-degrading enzymes with the standard curve, the initial amount/quantity of fucoidan in the sample is obtained. Digestion of fucoidan in a sample can be performed in a reaction mix. In some embodiments, a sample of seawater is contacted with enzymes capable of digesting fucoidan in a reaction mix. In some embodiments, a sample of seawater is contacted with enzymes capable of digesting fucoidan in a reaction mix, wherein the reaction mix comprises a sample of seawater, sea salt, CaCl₂ and enzymes capable of digesting fucoidan. In some embodiments, a rection mix comprises about 10-990 µl of a (dialyzed) sample of seawater, about 0.1-5% sea salts, about 1-100 mM CaCl₂ and about 1-1000 µg enzymes capable of digesting fucoidan. In some embodiments, a sample of seawater is contacted with enzymes capable of digesting fucoidan in a reaction mix, wherein the reaction mix comprises a dialyzed sample of seawater, sea salt, CaCh and enzymes capable of digesting fucoidan. In a preferred embodiment, a sample of seawater is contacted with enzymes capable of digesting fucoidan in a reaction mix, wherein the reaction mix comprises about 80 µl dialyzed sample of seawater, about 0.7% sea salt, about 10 mM CaCh and about 10 µg of enzymes capable of digesting fucoidan. In some embodiments, the contacting step further comprises incubating the reaction mix. In some embodiments, the contacting step further comprises incubating the reaction mix for about 0.1-24 h at about 20-50°C. In a preferred embodiment, the contacting step further comprises incubating the reaction mix for about 3 h at about 37°C. After incubating the reaction mix comprises monosaccharides originating from the digested fucoidan. The digestion in the reaction mix can be inactivated by heating the sample to about 99°C for about 1-15 min. The reaction mix can then be spun down and the monosaccharides can be collected from the supernatant.

Accordingly, the invention provides in one aspect a method for quantifying fucoidan, comprising:
(i) obtaining a sample of seawater, and
(ii) quantifying fucoidan in the sample.

In another aspect, the invention provides a method for quantifying fucoidan, comprising:
(i) obtaining a sample of seawater, and
(ii) quantifying fucoidan in the sample, comprising a contacting step.

In another aspect, the invention provides a method for quantifying fucoidan, comprising:
(i) obtaining a sample of seawater, and
(ii) quantifying fucoidan in the sample, comprising a contacting step comprising contacting a sample with enzymes capable of digesting fucoidan.

In another aspect, the invention provides a method for quantifying fucoidan, comprising:
(i) obtaining a sample of seawater, and
(ii) quantifying fucoidan in the sample, comprising a contacting step comprising contacting a sample with enzymes capable of digesting fucoidan, wherein the sample of seawater is contacted with enzymes capable of digesting fucoidan in a reaction mix comprising a dialyzed sample of seawater, sea salt, CaCl₂ and enzymes capable of digesting fucoidan.

In another aspect, the invention provides a method for quantifying fucoidan, comprising:
(i) obtaining a sample of seawater, and
(ii) quantifying fucoidan in the sample, comprising a contacting step comprising contacting a sample with enzymes capable of digesting fucoidan, wherein the sample of seawater is contacted with enzymes capable of digesting fucoidan in a reaction mix, comprising about 10-990 µl dialyzed sample of seawater, about 0.1-5% sea salt, about 1-100 mM CaCl₂ and about 1-1000 µg of enzymes capable of digesting fucoidan.

In another aspect, the invention provides a method for quantifying fucoidan, comprising:
(i) obtaining a sample of seawater, and
(ii) quantifying fucoidan in the sample, comprising a contacting step comprising contacting a sample with enzymes capable of digesting fucoidan, wherein the sample of seawater is contacted with enzymes capable of digesting fucoidan in a reaction mix comprising a dialyzed sample of seawater, sea salt, CaCl₂ and enzymes capable of digesting fucoidan, further comprising incubating the reaction mix for about 0.1-24 h at about 20-50°C, preferably for about 3 h at about 37°C.

In another aspect, the invention provides a method for quantifying fucoidan, comprising:
(i) obtaining a sample of seawater, and
(ii) quantifying fucoidan in the sample, comprising contacting a sample with enzymes capable of digesting fucoidan, and comprising quantifying monosaccharides obtained by digestion of fucoidan.

In another aspect, the invention provides a method for quantifying fucoidan, comprising:
(i) obtaining a sample of seawater, and
(ii) quantifying fucoidan in the sample, comprising contacting a sample with enzymes capable of digesting fucoidan, comprising quantifying monosaccharides obtained by digestion of fucoidan, comprising comparing the yield of quantified monosaccharides obtained by digestion of fucoidan with a fucoidan standard curve, thereby quantifying the initial amount/quantity of fucoidan in the sample.

In another aspect, the invention provides a method for quantifying fucoidan, comprising:
(i) obtaining a sample of seawater, and
(ii) quantifying fucoidan in the sample,
wherein step (ii) comprises:
(a) contacting the sample with enzymes capable of digesting fucoidan,
(b) quantifying monosaccharides obtained in (a),
(c) comparing the yield in step (b) with a fucoidan standard curve, thereby quantifying the initial amount/quantity of fucoidan in the sample.

In another aspect, the invention provides a method for quantifying fucoidan, comprising:
(a) obtaining a sample of seawater, and
(b) contacting the sample with enzymes capable of digesting fucoidan,
(c) quantifying monosaccharides obtained in (b),
(d) comparing the yield in step (c) with a fucoidan standard curve, thereby quantifying the initial amount/quantity of fucoidan in the sample.

In general, the enzyme assay of the present invention can be performed on any sample where quantification of fucoidan is desired. The specificity of the fucoidan-degrading enzymes allows to perform the enzyme assay directly onto a sample of seawater, e.g. without the need of any processing steps such as filtration/purification. It is however envisioned that the enzyme assay can also be performed on a sample where fucoidan has been purified, e.g. an eluted sample and/or precipitated sample as described herein. When applying the enzyme assay onto an eluted sample and/or precipitated sample as described herein, the reliability of the quantification may be increased.

Accordingly, the invention provides in one aspect a method for purifying and quantifying fucoidan, comprising:
(i) obtaining a sample of seawater, and
(ii) purifying fucoidan from the sample and
(iii) quantifying fucoidan in the sample, comprising contacting a sample with enzymes capable of digesting fucoidan.

In another aspect, the invention provides a method for purifying and quantifying fucoidan, comprising:
(i) obtaining a sample of seawater, and
(ii) purifying fucoidan from the sample and
(iii) quantifying fucoidan in the sample, comprising contacting a sample with enzymes capable of digesting fucoidan, comprising quantifying monosaccharides obtained by digestion of fucoidan.

In another aspect, the invention provides a method for purifying fucoidan and quantifying, comprising:
(i) obtaining a sample of seawater, and
(ii) purifying fucoidan from the sample and
(iii) quantifying fucoidan in the sample, comprising contacting a sample with enzymes capable of digesting fucoidan, comprising quantifying monosaccharides obtained by digestion of fucoidan, comprising comparing the yield of quantified monosaccharides obtained by digestion of fucoidan with a fucoidan standard curve, thereby quantifying the initial amount/quantity of fucoidan in the sample.

In another aspect, the invention provides a method for purifying and quantifying fucoidan, comprising:
(a) obtaining a sample of seawater, and
(b) purifying fucoidan from the sample and
(c) contacting the sample with enzymes capable of digesting fucoidan,
(d) quantifying monosaccharides obtained in (c),
(e) comparing the yield in step (d) with a fucoidan standard curve, thereby quantifying the initial amount/quantity of fucoidan in the sample.

The quantification steps and/or methods for quantifying fucoidan of the present invention can optionally further comprise generating a fucoidan standard curve. It is however also envisioned that generating a fucoidan standard curve is not part of any of the quantification steps and/or methods for quantifying fucoidan as disclosed herein. A fucoidan standard curve may be already available or can be established before performing the quantification steps and/or methods for quantifying fucoidan of the present invention. The generation of fucoidan standard curve can, however, also be comprised in the quantification steps and/or methods for quantifying fucoidan of the present invention. In some embodiments, the quantification steps and/or methods for quantifying fucoidan of the present invention can further comprise generating a fucoidan standard curve. In some embodiments, the quantification steps and/or methods for quantifying fucoidan of the present invention do not comprise generating a fucoidan standard curve. In some embodiments, the quantification steps and/or methods for quantifying fucoidan of the present invention can further comprise contacting a sample comprising a known amount/quantity of fucoidan, e.g. fucoidan standard or reference sample as disclosed herein, with enzymes capable of digesting fucoidan. In some embodiments, the quantification steps and/or methods for quantifying fucoidan of the present invention can further comprise contacting a sample comprising a known amount/quantity of fucoidan, e.g. fucoidan standard or reference sample as disclosed herein, with enzymes capable of digesting fucoidan, quantifying the obtained monosaccharides and generating a fucoidan standard curve by correlating the obtained monosaccharides with the known amount/quantity in the sample. In some embodiments, the steps for generating a fucoidan standard curve are repeated with desired samples having a desired known amount/quantity of fucoidan.

Accordingly, the invention provides in one aspect, a method for quantifying fucoidan, comprising:
(i) obtaining a sample of seawater, and
(ii) quantifying fucoidan in the sample, further comprising generating a fucoidan standard curve.

In another aspect, the invention provides a method for quantifying fucoidan, comprising:
(i) obtaining a sample of seawater, and
(ii) quantifying fucoidan in the sample, further comprising:
   (a) contacting a sample comprising a known amount/quantity of fucoidan with enzymes capable of digesting fucoidan
   (b) quantifying monosaccharides obtained in (a),
   (c) generating a fucoidan standard curve by correlating the yield of monosaccharides in (b) with the known amount/quantity of fucoidan in the sample of (a).

In another aspect, the invention provides a method for quantifying fucoidan, comprising:
(a) contacting a sample comprising a known amount/quantity of fucoidan with enzymes capable of digesting fucoidan
(b) quantifying monosaccharides obtained in (a),
(c) generating a fucoidan standard curve by correlating the yield of monosaccharides in (b) with the known amount/quantity of fucoidan in the sample of (a),
(d) obtaining a sample of seawater,
(e) contacting the sample of seawater with enzymes capable of digesting fucoidan,
(f) quantifying monosaccharides obtained in (e),
(g) comparing the yield in step (f) with a fucoidan standard curve, e.g. of (c), thereby quantifying the initial amount/quantity of fucoidan in the sample of seawater.

The present invention is not particularly limited to any means for contacting a sample, e.g. sample of seawater, rather any means that are feasible to contact a sample, e.g. a sample of seawater or fucoidan as such, can be used in the sense of the invention. In general, "contacting" or "come in contact with" as used herein means that two compounds are being brought into close proximity so that a reaction can happen. In the sense of the present invention, contacting can for example relate to mixing a sample of seawater with a buffer or reaction mix. In the sense of the invention, contacting can also mean that a fucoidan-degrading enzyme is brought into close proximity with fucoidan, e.g. mixed with a sample of seawater that comprises fucoidan. Exemplary means of contacting a sample, e.g. a sample of seawater, are mixing the sample with the compound to be contacted, e.g. a buffer or reaction mix, or fucoidan-degrading enzyme. For example, any sample as disclosed herein, such as a sample of seawater, purified sample, eluted sample, precipitated sample, dialyzed sample, or dried sample, such as a freeze-dried sample, may be contacted by mixing said sample with the compound to be contacted, e.g. a buffer or reaction mix.

It is also envisioned herein, that the method for quantifying fucoidan of the present invention further comprises a step of producing enzymes that are capable of degrading fucoidan, e.g. fucoidan-degrading enzymes. Such a step may comprise culturing fucoidan-degrading microorganisms, such as bacteria belonging to the phyla Verrucomicrobiota, Planktomycetota or Bacteroidota, and isolating fucoidan-degrading enzymes from said microorganisms. In a preferred embodiment, such a step may comprise culturing *Lentimonas sp.* CC4, and isolating fucoidan-degrading enzymes from said microorganism. The invention is not particularly limited to any microorganisms, rather any microorganisms that is able to degrade fucoidan can be used in the sense of the present invention. The invention is also not particularly limited to any fucoidan-degrading enzymes, rather any enzymes, enzyme composition or enzyme mix that is able to degrade fucoidan, e.g. into monosaccharides, can be used in the sense of the present invention. In a preferred embodiment a fucoidan-degrading microorganism is *Lentimonas sp.* CC4. In some embodiments a fucoidan-degrading enzyme is one or more of a fucoidanase, sulfatase and/or fucosidase. In a preferred embodiment, a fucoidan-degrading enzyme composition comprises one or more of a fucoidanase, sulfatase and/or fucosidase. The fucoidan degrading enzymes may be isolated from a culture of fucoidan-degrading microorganisms by lysis of the fucoidan-degrading microorganisms, followed a separation by centrifugation. The lysis and centrifugation separate the cellular fraction of fucoidan-degrading microorganisms and the supernatant containing the soluble cytosolic proteins including the fucoidan-degrading enzymes. The fucoidan-degrading enzymes can subsequently be obtained by collecting the supernatant. In some embodiments, the method for quantifying fucoidan further comprises culturing a fucoidan-degrading microorganisms. In some embodiments, the method for quantifying fucoidan further comprises culturing *Lentimonas sp.* CC4. In some embodiments, the method for quantifying fucoidan further comprises culturing a fucoidan-degrading microorganism and isolating fucoidan-degrading enzymes. In some embodiments, the method for quantifying fucoidan further comprises culturing *Lentimonas sp.* CC4 and isolating fucoidanases, sulfatases and/or fucosidases. In some embodiments, the method for quantifying fucoidan further comprises culturing a fucoidan-degrading microorganisms and isolating fucoidan-degrading enzymes, wherein the isolating step comprises lysis of fucoidan-degrading microorganisms and separation of fucoidan-degrading enzymes, e.g. by centrifugation. In some embodiments, the method for quantifying fucoidan further comprises culturing *Lentimonas sp.* CC4 and isolating fucoidanases, sulfatases and/or fucosidases, wherein the isolating step comprises lysis of *Lentimonas sp.* CC4 and separation of fucoidanases, sulfatases and/or fucosidases, e.g. by centrifugaiton. In some embodiments the separation step comprises separating a cellular fraction from an enzyme fraction, e.g. by centrifugation. In some embodiments, the separation step comprises centrifuging the lysed fucoidan-degrading microorganisms. In some embodiments, the enzyme fraction is comprised in the supernatant. An exemplary method for producing enzymes that are capable of degrading fucoidan, e.g. fucoidan-degrading enzymes is provided in the appended Example 2.

Accordingly, the invention provides in one aspect a method for quantifying fucoidan, comprising:
(i) obtaining a sample of seawater, and
(ii) quantifying fucoidan in the sample,
wherein the method further comprises culturing a fucoidan-degrading microorganism, e.g. bacteria belonging to the phyla Verrucomicrobiota, Planktomycetota or Bacteroidota.

In another aspect, the invention provides a method for quantifying fucoidan, comprising:
(i) obtaining a sample of seawater, and
(ii) quantifying fucoidan in the sample,
wherein the method further comprises culturing *Lentimonas sp.* CC4.

In another aspect, the invention provides a method for quantifying fucoidan, comprising:
(i) obtaining a sample of seawater, and
(ii) quantifying fucoidan in the sample,
wherein the method further comprises culturing a fucoidan-degrading microorganism, and isolating fucoidan-degrading enzymes from the culture of the fucoidan-degrading microorganism.

In another aspect, the invention provides a method for quantifying fucoidan, comprising:
(i) obtaining a sample of seawater, and
(ii) quantifying fucoidan in the sample,
wherein the method further comprises culturing a fucoidan-degrading microorganism, and isolating fucoidanases, sulfatases and/or fucosidases from the culture of the fucoidan-degrading microorganism.

In another aspect, the invention provides a method for quantifying fucoidan, comprising:
(i) obtaining a sample of seawater, and
(ii) quantifying fucoidan in the sample,
wherein the method further comprises culturing *Lentimonas sp.* CC4 and isolating fucoidanases, sulfatases and/or fucosidases from the culture of *Lentimonas sp.* CC4.

In another aspect, the invention provides a method for quantifying fucoidan, comprising:
(i) obtaining a sample of seawater, and
(ii) quantifying fucoidan in the sample,
wherein the method further comprises culturing a fucoidan-degrading microorganism, and isolating fucoidan-degrading enzymes from the culture of the fucoidan-degrading microorganism, wherein the isolating step comprises lysis of fucoidan-degrading microorganisms and separation of fucoidan-degrading enzymes.

In another aspect, the invention provides a method for quantifying fucoidan, comprising:
(i) obtaining a sample of seawater, and
(ii) quantifying fucoidan in the sample,
wherein the method further comprises culturing *Lentimonas sp.* CC4, and isolating fucoidanases, sulfatases and/or fucosidases from the culture of *Lentimonas sp.* CC4, wherein the isolating step comprises lysis of *Lentimonas sp.* CC4 and separation of fucoidanases, sulfatases and/or fucosidases.

In another aspect, the invention provides a method for quantifying fucoidan, comprising:
(i) obtaining a sample of seawater, and
(ii) quantifying fucoidan in the sample,
wherein the method further comprises culturing a fucoidan-degrading microorganism, and isolating fucoidan-degrading enzymes from the culture of the fucoidan-degrading microorganism, wherein the isolating step comprises lysis of fucoidan-degrading microorganisms and separation of fucoidan-degrading enzymes, wherein the separation step comprises separating a cellular fraction from an enzyme fraction, e.g. by centrifugation.

In another aspect, the invention provides a method for quantifying fucoidan, comprising:
(i) obtaining a sample of seawater, and
(ii) quantifying fucoidan in the sample,
wherein the method further comprises culturing *Lentimonas sp.* CC4, and isolating fucoidanases, sulfatases and/or fucosidases from the culture of *Lentimonas sp.* CC4, wherein the isolating step comprises lysis of *Lentimonas sp.* CC4 and separation of fucoidanases, sulfatases and/or fucosidases, wherein the separation step comprises separating a cellular fraction from an enzyme fraction, e.g. by centrifugation.

In another aspect, the invention provides a method for quantifying fucoidan, comprising:
(i) obtaining a sample of seawater, and
(ii) quantifying fucoidan in the sample,
wherein the method further comprises culturing fucoidan-degrading microorganisms, and isolating fucoidan-degrading enzymes from the culture of fucoidan-degrading microorganisms, optionally wherein the fucoidan degrading enzymes are obtained by a lysis step of the fucoidan-degrading microorganisms followed by a separation step that separates the culture into a cellular fraction and a fraction comprising enzymes capable of digesting fucoidan, e.g. in the supernatant.

In general, the invention is not particularly limited to performing certain steps in a certain order. The skilled person is aware that steps are to be performed in an order that makes technical sense. In any of the methods disclosed herein step (a) of obtaining a sample of seawater can be optional. Accordingly, the method for purifying and/or quantifying fucoidan of the present invention preferably comprises the following steps to be performed in chronological/alphabetical order:
(a) an initial step of obtaining a sample of seawater,
(b) a prefiltration step,
(c) a conditioning step,
(d) an application step,
(e) a washing step,
(f) an elution step,
(g) an optional reconditioning step,
(h) a quantification step.

In some embodiments steps (b)-(f) as defined above may further define step (ii) purifying fucoidan from a sample of seawater as disclosed herein. In some embodiments steps (b)-(f) as defined above may be comprised in step (ii) purifying fucoidan from a sample of seawater as disclosed herein.

In another aspect, the method for purifying and/or quantifying fucoidan of the present invention preferably comprises the following steps to be performed in chronological/alphabetical order:
(a) an initial step of obtaining a sample of seawater,
(b) a prefiltration step,
(c) a precipitation step,
(d) a filtration step,
(e) an elution or resuspension step,
(f) a quantification step.

In another aspect, the method for purifying and/or quantifying fucoidan of the present invention preferably comprises the following steps to be performed in chronological/alphabetical order:
(a) an initial step of obtaining a sample of seawater,
(b) a prefiltration step,
(c) a conditioning step,
(d) an application step,
(e) a washing step,
(f) an elution step,
(g) an optional reconditioning step,
(h) a precipitation step,
(i) a filtration step,
(j) an elution or resuspension step,
(k) a quantification step.

In another aspect, the method for purifying and/or quantifying fucoidan of the present invention preferably comprises the following steps to be performed in chronological/alphabetical order:
(a) an initial step of obtaining a sample of seawater,
(b) a prefiltration step,
(c) a precipitation step,
(d) a filtration step,
(e) an elution or resuspension step,
(f) a conditioning step,
(g) an application step,
(h) a washing step,
(i) an elution step,
(j) an optional reconditioning step,
(k) a quantification step.

In another aspect, the method for quantifying fucoidan of the present invention preferably comprises the following steps to be performed in chronological/alphabetical order:
(a) an initial step of obtaining a sample of seawater,
(b) a contacting step,
(c) a quantification step of monosaccharides,
(d) an optional step generating a fucoidan standard curve,
(e) an evaluation step.

In some embodiments step (iii) quantifying fucoidan in the sample of the method for purifying fucoidan of the present invention can comprise the method for quantifying fucoidan of the present invention.

Accordingly, the method for purifying and/or quantifying fucoidan of the present invention preferably comprises the following steps to be performed in chronological/alphabetical order:
(a) an initial step of obtaining a sample of seawater,
(b) a prefiltration step,
(c) a conditioning step,
(d) an application step,
(e) a washing step,
(f) an elution step,
(g) an optional reconditioning step,
(h) a contacting step,
(i) a quantification step of monosaccharides,
(j) an optional step generating a fucoidan standard curve,
(k) an evaluation step.

In another aspect, the method for purifying and/or quantifying fucoidan of the present invention preferably comprises the following steps to be performed in chronological/alphabetical order:
(a) an initial step of obtaining a sample of seawater,
(b) a prefiltration step,
(c) a precipitation step,
(d) a filtration step,
(e) an elution or resuspension step,
(f) a contacting step,
(g) a quantification step of monosaccharides,
(h) an optional step generating a fucoidan standard curve,
(i) an evaluation step.

In another aspect, the method for purifying and/or quantifying fucoidan of the present invention preferably comprises the following steps to be performed in chronological/alphabetical order:
(a) an initial step of obtaining a sample of seawater,
(b) a prefiltration step,
(c) a conditioning step,
(d) an application step,
(e) a washing step,
(f) an elution step,
(g) an optional reconditioning step,
(h) a precipitation step,
(i) a filtration step,
(j) an elution or resuspension step,
(k) a contacting step,
(l) a quantification step of monosaccharides,
(m) an optional step generating a fucoidan standard curve,
(n) an evaluation step.

In another aspect, the method for purifying and/or quantifying fucoidan of the present invention preferably comprises the following steps to be performed in chronological/alphabetical order:
(a) an initial step of obtaining a sample of seawater,
(b) a prefiltration step,
(c) a precipitation step,
(d) a filtration step,
(e) an elution or resuspension step,
(f) a conditioning step,
(g) an application step,
(h) a washing step,
(i) an elution step,
(j) an optional reconditioning step,
(k) a contacting step,
(l) a quantification step of monosaccharides,
(m) an optional step generating a fucoidan standard curve,
(n) an evaluation step.

In a preferred aspect, the invention provides a method for purifying fucoidan, comprising:
(a) an initial step of obtaining a sample of seawater,
(b) a prefiltration step, comprising filtrating the sample of seawater through a combusted glass fiber filter,
(c) a conditioning step, comprising conditioning an anion exchange chromatography column with about 1-5 column volumes of buffer A comprising about 20 mM Tris-HCl, about 0.5 M NaCl at about pH 8, followed by about 1-5 column volumes of buffer B comprising about 20 mM Tris-HCl, about 4 M NaCl at about pH 8, followed by about 1-5 column volumes of buffer A comprising about 20 mM Tris-HCl, about 0.5 M NaCl at about pH 8,
(d) an application step, comprising applying the sample of seawater onto the column
(e) a washing step, washing the column with about 1-5 column volumes of wash buffer comprising about 20 mM Tris-HCl, about 0.5 MNaCl at about pH 8, optionally repeated the washing step 1-10 times,
(f) an elution step, comprising eluting fucoidan with about 1-5 column volumes of elution buffer comprising about 20 mM Tris-HCl, about 4 M NaCl at about pH 8,
(g) an optional reconditioning step, comprising rinsing the column with about 1-5 column volumes of buffer B comprising about 20 mM Tris-HCl, about 0.5 M NaCl, at about pH 8, followed by about 1-5 column volumes of ultrapure water, followed by about 1-5 column volumes of about 1 M NaOH at a reduced flow rate, followed by about 1-5 column volumes of ultrapure water,
(h) a quantification step,
preferably wherein steps (a)-(h) are performed in chronological/alphabetical order.

In a preferred aspect, the invention provides a method for purifying fucoidan, comprising:
(a) an initial step of obtaining a sample of seawater,
(b) a prefiltration step, comprising filtrating the sample of seawater through a combusted glass fiber filter,
(c) a precipitation step, comprising reducing the salinity/salt concentration in the sample of seawater, optionally diluting the sample of seawater with ultrapure water, and adding a quaternary ammonium surfactant to the sample of seawater, wherein the concentration of the amphiphilic compound in the sample of seawater is about 0.1 g/l,
(d) a filtration step, comprising filtrating the sample of seawater through a combusted glass fiber filter,
(e) an elution or resuspension step, wherein the precipitated fucoidan is eluted or resuspended from the filter,
(f) a quantification step,
preferably wherein steps (a)-(f) are performed in chronological/alphabetical order.

In a preferred aspect, the invention provides a method for quantifying fucoidan, comprising:
(a) an initial step of obtaining a sample of seawater or a fucoidan standard/reference sample,
(b) a contacting step, comprising contacting a sample with enzymes capable of digesting fucoidan,
(c) a quantification step, comprising quantifying monosaccharides obtained in (b), preferably wherein the monosaccharide is fucose,
(d) an optional step generating a fucoidan standard curve, comprising correlating the yield of monosaccharides in (c) with the known amount/quantity of the fucoidan standard/reference sample of (a),
(e) an evaluation step, comprising comparing the yield of monosaccharides, preferably fucose, in step (c) with a fucoidan standard curve, e.g. of (d), thereby quantifying the initial amount/quantity of fucoidan in the sample of seawater,
preferably wherein steps (a)-(e) are performed in chronological/alphabetical order.

In another preferred aspect, the invention provides a method for quantifying fucoidan, comprising:
(a) an initial step of obtaining a sample of seawater,
(b) a contacting step, comprising contacting a sample with enzymes capable of digesting fucoidan,
(c) a quantification step, comprising quantifying monosaccharides obtained in (b), preferably wherein the monosaccharide is fucose,
(d) an evaluation step, comprising comparing the yield of monosaccharides, preferably fucose, in step (c) with a fucoidan standard curve, thereby quantifying the initial amount/quantity of fucoidan in the sample of seawater,
preferably wherein steps (a)-(d) are performed in chronological/alphabetical order.

In another preferred aspect, the invention provides a method for purifying and quantifying fucoidan, comprising:
(a) an initial step of obtaining a sample of seawater,
(b) a prefiltration step, comprising filtrating the sample of seawater through a combusted glass fiber filter,
(c) a conditioning step, comprising conditioning an anion exchange chromatography column with about 1-5 column volumes of buffer A comprising about 20 mM Tris-HCl, about 0.5 M NaCl at about pH 8, followed by about 1-5 column volumes of buffer B comprising about 20 mM Tris-HCl, about 4 M NaCl at about pH 8, followed by about 1-5 column volumes of buffer A comprising about 20 mM Tris-HCl, about 0.5 M NaCl at about pH 8,
(d) an application step, comprising applying the sample of seawater onto the column
(e) a washing step, washing the column with about 1-5 column volumes of wash buffer comprising about 20 mM Tris-HCl, about 0.5 MNaCl at about pH 8, optionally repeated the washing step 1-10 times,
(f) an elution step, comprising eluting fucoidan with about 1-5 column volumes of elution buffer comprising about 20 mM Tris-HCl, about 4 M NaCl at about pH 8,
(g) an optional reconditioning step, comprising rinsing the column with about 1-5 column volumes buffer B comprising about 20 mM Tris-HCl, about 0.5 M NaCl, at about pH 8, followed by about 1-5 column volumes of ultrapure water, followed by about 1-5 column volumes of about 1 M NaOH at a reduced flow rate, followed by about 1-5 column volumes of ultrapure water,
(h) a contacting step, comprising contacting the eluted sample of (f) with enzymes capable of digesting fucoidan,
(i) a quantification step, comprising quantifying monosaccharides obtained in (h), preferably wherein the monosaccharide is fucose,
(j) an evaluation step, comprising comparing the yield of monosaccharides, preferably fucose, in step (i) with a fucoidan standard curve, thereby quantifying the initial amount/quantity of fucoidan in the sample of seawater,
preferably wherein steps (a)-(j) are performed in chronological/alphabetical order.

In a preferred aspect, the invention provides a method for purifying fucoidan, comprising:
(a) an initial step of obtaining a sample of seawater,
(b) a prefiltration step, comprising filtrating the sample of seawater through a combusted glass fiber filter,
(c) a precipitation step, comprising reducing the salinity/salt concentration in the sample of seawater, optionally diluting the sample of seawater with ultrapure water, and adding a quaternary ammonium surfactant to the sample of seawater, wherein the concentration of the amphiphilic compound in the sample of seawater is about 0.1 g/l,
(d) a filtration step, comprising filtrating the sample of seawater through a combusted glass fiber filter,
(e) an elution or resuspension step, wherein the precipitated fucoidan is eluted or resuspended from the filter,
(f) a contacting step, comprising contacting the eluted or resuspended sample of (e) with enzymes capable of digesting fucoidan,
(g) a quantification step, comprising quantifying monosaccharides obtained in (f), preferably wherein the monosaccharide is fucose,
(h) an evaluation step, comprising comparing the yield of monosaccharides, preferably fucose, in step (g) with a fucoidan standard curve, thereby quantifying the initial amount/quantity of fucoidan in the sample of seawater,
preferably wherein steps (a)-(h) are performed in chronological/alphabetical order.

It may be preferred herein that method steps comprised in a method for purifying fucoidan of the present invention are performed in a certain order. In some embodiments, an optional step of obtaining a sample, e.g. a sample of seawater is performed first. In some embodiments a conditioning step is performed after an optional step of obtaining a sample, e.g. a sample of seawater or is performed as a first step. In some embodiments an application step is performed after a conditioning step. In some embodiments a washing step is performed after an application step. In some embodiments an elution step is performed after a washing step. In some embodiments a reconditioning step is performed after an elution step. In some embodiments a quantification step is performed after an elution step. In some embodiments a quantification step is performed after step (ii) purifying fucoidan from the sample. In some embodiments an optional step of obtaining a sample, e.g. a sample of seawater, is performed first, followed by a conditioning step, followed by an application step, followed by a washing step, followed by an elution step, optionally followed by a reconditioning step, followed by a quantification step. The quantification step may comprise a method for quantifying fucoidan as disclosed herein. It may be preferred herein that method steps comprised in a method for quantifying fucoidan of the present invention are performed in a certain order. In some embodiments, an optional step of obtaining a sample, e.g. a sample of seawater is performed first. In some embodiments an optional culturing step is performed after an optional step of obtaining a sample, e.g. a sample of seawater. In some embodiments an optional lysis step is performed after an optional culturing step. In some embodiments an optional isolation step is performed after an optional lysis step. In some embodiments a contacting step is performed after an optional isolation step or an optional step of obtaining a sample, e.g. a sample of seawater, or is performed as a first step. In some embodiments, a quantification step is performed after a contacting step. In some embodiments an optional step generating a fucoidan standard curve is performed after a quantification step. In some embodiments an evaluation step is performed after a quantification step or after an optional step generating a fucoidan standard curve.

In some embodiments, an optional step of obtaining a sample, e.g. a sample of seawater is performed first, followed by an optional culturing step, followed by an optional lysis step, followed by an optional isolation step, followed by a contacting step, followed by a quantification step, followed by an optional step generating a fucoidan standard curve, followed by an evaluation step.

In some embodiments, a contacting step is performed first, followed by a quantification step, followed by an optional step generating a fucoidan standard curve, followed by an evaluation step.

Any of the methods for quantifying fucoidan or a step (iii) quantifying fucoidan in a sample, e.g. sample of seawater, or eluted sample can optionally further comprise a step of dialyzing the sample. A sample, such as a sample of seawater or an eluted sample may be dialyzed to remove salts. A sample, such as a sample of seawater or an eluted sample may also be dialyzed to reduce the salinity/salt concentration. A sample, such as a sample of seawater or an eluted sample may also be dialyzed to remove salts, and be subsequently included in a reaction mixture, e.g. buffer, that has a desired composition and e.g. a desired salinity/salt concentration. Removing salts or reducing salinity/salt concentration in a sample can be desired for subsequent processing. In some embodiments a precipitated sample as disclosed herein is not dialyzed. Precipitated samples can be resuspended for subsequent purification and/or quantification, accordingly a desired composition and/or salinity/salt concentration can be adjusted in the resuspension medium, e.g. a buffer. In some embodiments, samples, such as a sample of seawater or an eluted sample or a purified sample, can be dialyzed through 1kDa membranes against purified water. In some embodiments the dialyzing step can further comprise drying the sample. In some embodiments the dialyzing step can further comprise drying the dialyzed sample and resuspending the sample.

Accordingly, the invention provides in one aspect a method for purifying fucoidan, comprising:
(i) obtaining a sample of seawater, further comprising dialyzing the sample, and
(ii) purifying fucoidan from the sample.

In another aspect, the invention provides a method for purifying fucoidan, comprising:
(i) obtaining a sample of seawater, and
(ii) purifying fucoidan from the sample, further comprising dialyzing the sample, preferably comprising dialyzing the eluted sample.

In another aspect, the invention provides a method for quantifying fucoidan, comprising:
(i) obtaining a sample of seawater, further comprising dialyzing the sample, and
(ii) quantifying fucoidan in the sample.

Any of the methods for quantifying fucoidan or a step (iii) quantifying fucoidan in a sample, e.g. sample of seawater, or eluted sample can optionally, or alternatively to the enzyme assay disclosed herein above comprise a step of acid hydrolysis followed by monosaccharide quantification. In brief, samples, e.g. sample of seawater, or eluted sample, can be dialyzed against purified water to remove salts and small molecules. Dialyzed samples can be freeze dried and resuspended in purified water. A precipitated sample as disclosed herein can be resuspended in purified water. Resuspended samples, such as a sample of seawater, an eluted sample or a precipitated sample, can be combined with HCl and incubated for 24 h at 100°C to achieve complete acid hydrolysis of polysaccharides in the sample. The obtained monosaccharides can be quantified by methods known to the skilled person e.g. mass spectrometry, gas chromatography, liquid chromatography, capillary electrophoresis, high-performance liquid chromatography, anion exchange chromatography with pulsed amperometric detection (HPAEC-PAD) (see e.g. 102, 103). In general, when quantifying fucoidan, monosaccharides are quantified which are then correlated to the fucoidan content in the initial sample. Fucose is a monosaccharide product whose concentration significantly correlates with fucoidan concentration in initial/input sample. Accordingly, any of the methods for quantifying fucoidan or a step (iii) quantifying fucoidan in a sample as disclosed herein can comprise quantifying a monosaccharide, such as fucose. In some embodiments, a method for quantifying fucoidan comprises quantification of a monosaccharide. In some embodiments, a method for quantifying fucoidan comprises quantification of fucose.

Exemplary means to quantify monosaccharides are disclosed in detail in the appended Examples.

Accordingly, the invention provides in one aspect a method for purifying and quantifying fucoidan, comprising:
(i) obtaining a sample of seawater, and
(ii) purifying fucoidan from the sample, and
(iii) quantifying fucoidan in the sample.

In another aspect, the invention provides a method for purifying and quantifying fucoidan, comprising:
(i) obtaining a sample of seawater, and
(ii) purifying fucoidan from the sample, and
(iii) quantifying fucoidan in the sample,
wherein the quantifying step comprises processing of fucoidan into monosaccharides.

In another aspect, the invention provides a method for purifying and quantifying fucoidan, comprising:
(i) obtaining a sample of seawater, and
(ii) purifying fucoidan from the sample, and
(iii) quantifying fucoidan in the sample,
wherein the quantifying step comprises processing of fucoidan into monosaccharides via acid hydrolysis.

In another aspect, the invention provides a method for purifying and quantifying fucoidan, comprising:
(i) obtaining a sample of seawater, and
(ii) purifying fucoidan from the sample, and
(iii) quantifying fucoidan in the sample,
wherein the quantifying step comprises processing of fucoidan into monosaccharides via acid hydrolysis and quantifying the monosaccharides.

In another aspect, the invention provides a method for purifying and quantifying fucoidan, comprising:
(i) obtaining a sample of seawater, and
(ii) purifying fucoidan from the sample, and
(iii) quantifying fucoidan in the sample,
wherein the quantifying step comprises processing of fucoidan into fucose via acid hydrolysis and quantifying the fucose.

In another aspect, the invention provides a method for quantifying fucoidan, comprising:
(i) obtaining a sample of seawater, and
(ii) quantifying fucoidan in the sample,
wherein the quantifying step comprises processing of fucoidan into monosaccharides.

In another aspect, the invention provides a method for quantifying fucoidan, comprising:
(i) obtaining a sample of seawater, and
(ii) quantifying fucoidan in the sample,
wherein the quantifying step comprises processing of fucoidan into monosaccharides via acid hydrolysis.

In another aspect, the invention provides a method for quantifying fucoidan, comprising:
(i) obtaining a sample of seawater, and
(ii) quantifying fucoidan in the sample,
wherein the quantifying step comprises processing of fucoidan into monosaccharides via acid hydrolysis and quantifying the monosaccharides.

In another aspect, the invention provides a method for quantifying fucoidan, comprising:
(i) obtaining a sample of seawater, and
(ii) quantifying fucoidan in the sample,
wherein the quantifying step comprises processing of fucoidan into fucose via acid hydrolysis and quantifying the fucose.

In another aspect, the invention provides a method for quantifying fucoidan, comprising:
(i) obtaining a sample of seawater, and
(ii) quantifying fucoidan in the sample,
wherein the quantifying step comprises quantifying fucose.

It is also envisioned herein that a step of quantifying fucoidan in the sample can comprise contacting fucoidan in a sample with an antibody. For example, quantitative binding of a fucoidan antibody can be detected by an enzyme-linked immunosorbent assay (ELISA) on dialyzed samples, e.g. sample of seawater, purified sample, eluted sample, or precipitated and resuspended sample. Exemplary anti-fucoidan antibodies that can be used in the sense of the invention are BAM1, BAM2 or BAM7 (SeaProbes, Roscoff, France).

Accordingly, the invention provides in one aspect a method for purifying and quantifying fucoidan, comprising:
(i) obtaining a sample of seawater, and
(ii) purifying fucoidan from the sample, and
(iii) quantifying fucoidan in the sample, comprising contacting the sample with an anti-fucoidan antibody.

In another aspect, the invention provides a method for purifying and quantifying fucoidan, comprising:
(i) obtaining a sample of seawater, and
(ii) purifying fucoidan from the sample, and
(iii) quantifying fucoidan in the sample, comprising contacting the sample with an anti-fucoidan antibody, and quantifying the binding of fucoidan in the sample to the anti-fucoidan antibody, optionally wherein the anti-fucoidan antibody is selected from the group of BAM1, BAM2 or BAM7.

In another aspect, the invention provides a method for quantifying fucoidan, comprising:
(i) obtaining a sample of seawater, and
(ii) quantifying fucoidan in the sample, comprising contacting the sample with an anti-fucoidan antibody.

In another aspect, the invention provides a method for quantifying fucoidan, comprising:
(i) obtaining a sample of seawater, and
(ii) quantifying fucoidan in the sample, comprising contacting the sample with an anti-fucoidan antibody, and quantifying the binding of fucoidan in the sample to the anti-fucoidan antibody, optionally wherein the anti-fucoidan antibody is selected from the group of BAM1, BAM2 or BAM7.

In yet another aspect, the invention provides a method for quantifying fucoidan, comprising:
(i) obtaining a sample of seawater, and
(ii) quantifying fucoidan in the sample, comprising processing of fucoidan into monosaccharides.

In another aspect, the invention provides a method for quantifying fucoidan, comprising:
(i) obtaining a sample of seawater, and
(ii) quantifying fucoidan in the sample, comprising acid hydrolysis of the sample of seawater.

In another aspect, the invention provides a method for quantifying fucoidan, comprising:
(i) obtaining a sample of seawater, and
(ii) quantifying fucoidan in the sample, comprising acid hydrolysis of polysaccharides in the sample of seawater.

The present invention also provides a composition comprising fucoidan produced by a method for purifying fucoidan of the present invention. Such a composition is particularly advantageous since the purity of fucoidan allows for robust and reliable quantification. Thus, it may be desired that a purified fucoidan composition can be used for quantification of fucoidan, thereby avoiding contamination of other organic compounds, e.g. polysaccharides other than fucoidan. Accordingly, the invention provides in one aspect a composition comprising fucoidan produced by a method for purifying fucoidan of the present invention. In another aspect, the invention provides a composition comprising fucoidan produced by a method for purifying fucoidan as disclosed herein.

In some embodiments, any of the methods disclosed herein may not comprise a step of obtaining a sample of seawater. In any of the methods disclosed herein a step of obtaining a sample of seawater can be optional. Any of the methods disclosed herein can also be performed without a step of obtaining a sample of seawater. Any of the methods disclosed herein can also be performed without a step of obtaining a sample of seawater, i.e. on a sample that has already been obtained.

Unless otherwise defined, all terms of art, notations and other scientific terminology used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a difference over what is generally understood in the art. The techniques and procedures described or referenced herein are generally well understood and commonly employed using conventional methodologies by those skilled in the art. As appropriate, procedures involving the use of commercially available kits and reagents are generally carried out in accordance with manufacturer-defined protocols and conditions unless otherwise noted.

As used herein, the singular forms "a," "an," and "the" include the plural referents unless the context clearly indicates otherwise. The terms "include," "such as," and the like are intended to convey inclusion without limitation, unless otherwise specifically indicated.

As used herein, the term "or" is generally employed in its usual sense including "and/or" unless the content clearly dictates otherwise. The term "and/or" means one or all of the listed elements or a combination of any two or more of the listed elements.

As used herein, the term "comprising" also specifically includes embodiments "consisting of' and "consisting essentially of' the recited elements, unless specifically indicated otherwise.

As used herein, the term "about" indicates and encompasses an indicated value and a range above and below that value. In certain embodiments, the term "about" indicates the designated value ± 10%, ± 5%, or ± 1%. In certain embodiments, where applicable, the term "about" indicates the designated value(s) ± one standard deviation of that value(s).

The disclosures in context of the methods described herein are disclosed as corresponding use *mutatis mutandis.* The disclosures in context of the use described herein are disclosed as corresponding methods *mutatis mutandis.*

This specification cites a number of documents including non-patent literature, e.g. scientific publications and manufacturer's manuals as well as patent documents. The disclosure of these documents is herewith incorporated by reference in their entirety. More specifically, all referenced documents are incorporated by reference to the same extent as if each individual document was specifically and individually indicated to be incorporated by reference.

### Brief description of the drawings:

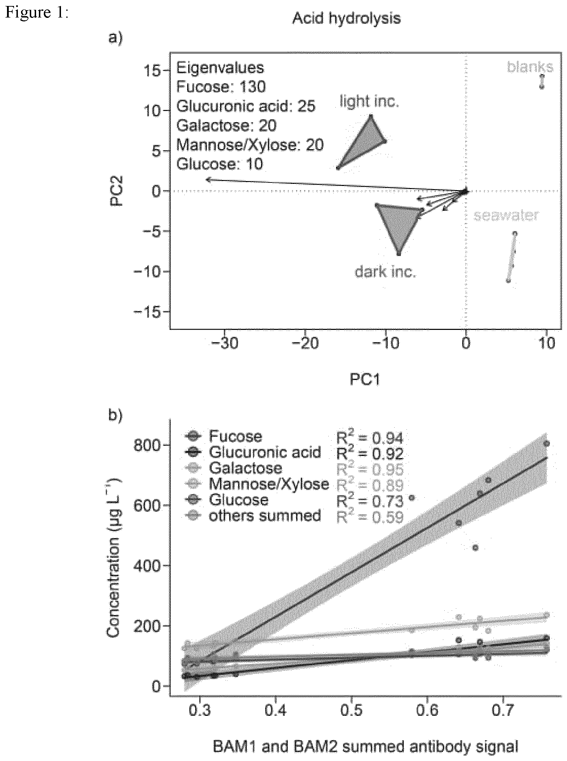

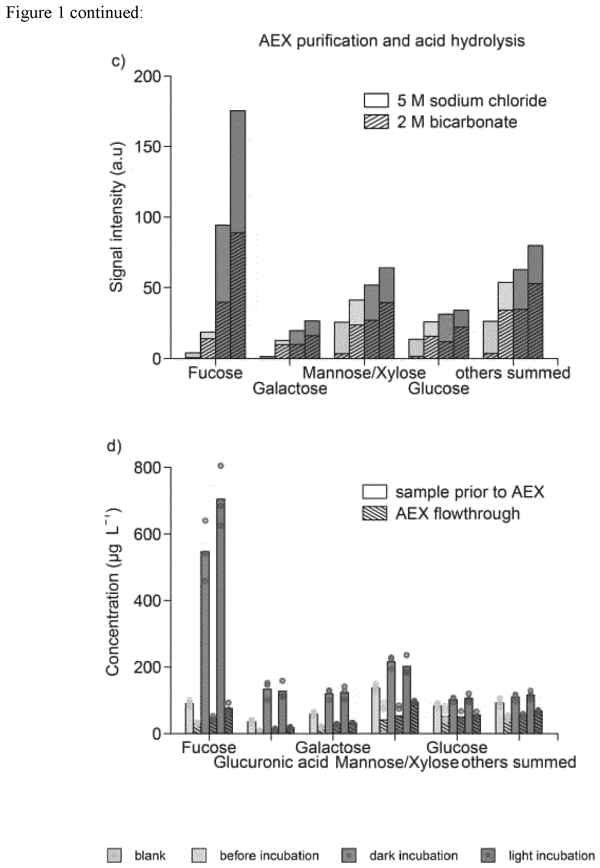

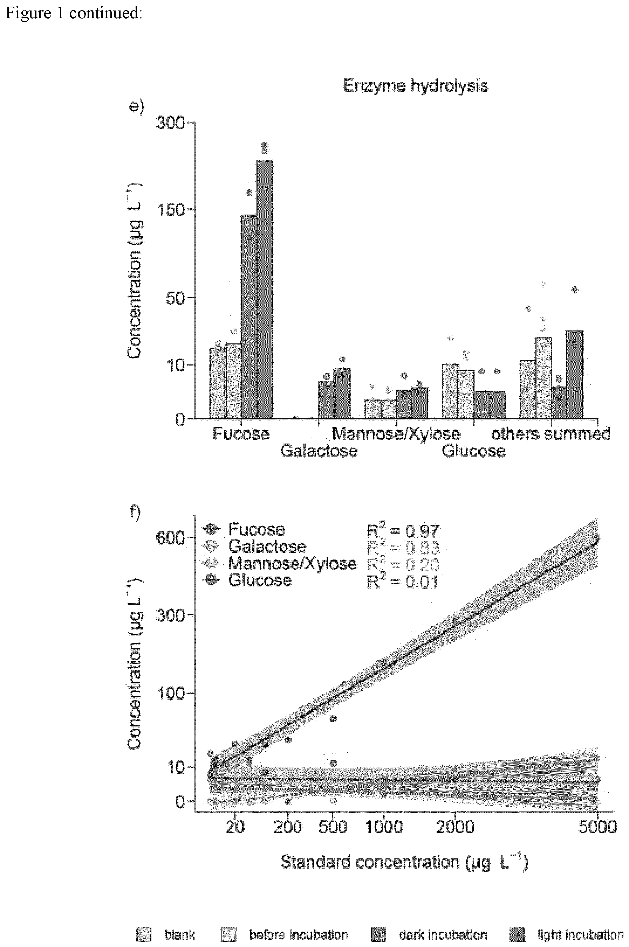

**Figure 1****: Polysaccharide-targeting techniques enabled specific quantification of dissolved fucoidan.** a) Acid hydrolyzed samples from algae incubations (inc.) are plotted in a principal component analysis of monosaccharide concentrations with eigenvalue lengths of dominant monosaccharides. b) Monosaccharide concentrations after acid hydrolysis are plotted against the summed signal absorbance at 450 nm, measured by ELISA, of two fucoidan-specific antibodies. c) Washing with 2 M bicarbonate buffer removed considerable amounts of monosaccharides (striped bars) prior to elution with 5 M NaCl (solid bars). d) Monosaccharide concentrations in acid hydrolyzed samples (solid bars) exceeded monosaccharides in the flow-through of AEX (striped) several-fold. e) Monosaccharides in samples, released using enzymes in the soluble cell fraction of fucoidan-metabolizing *Lentimonas* sp. f) Calibration curves for monosaccharides from enzymatic digestion of fucoidan from *F. vesiculosus.* Abbreviations: arbitrary units (a.u.), fucose (Fuc), galactose (Gal), mannose/xylose (Man/Xyl), glucose (Glc) and glucuronic acid (GlcA).
**Figure 2****: Fucose is the best predictor of dissolved fucoidan concentration** in seawater after dialysis and acid hydrolysis. Standards were prepared using >95% pure *F. vesiculosus* fucoidan in 6 g/kg artificial seawater.

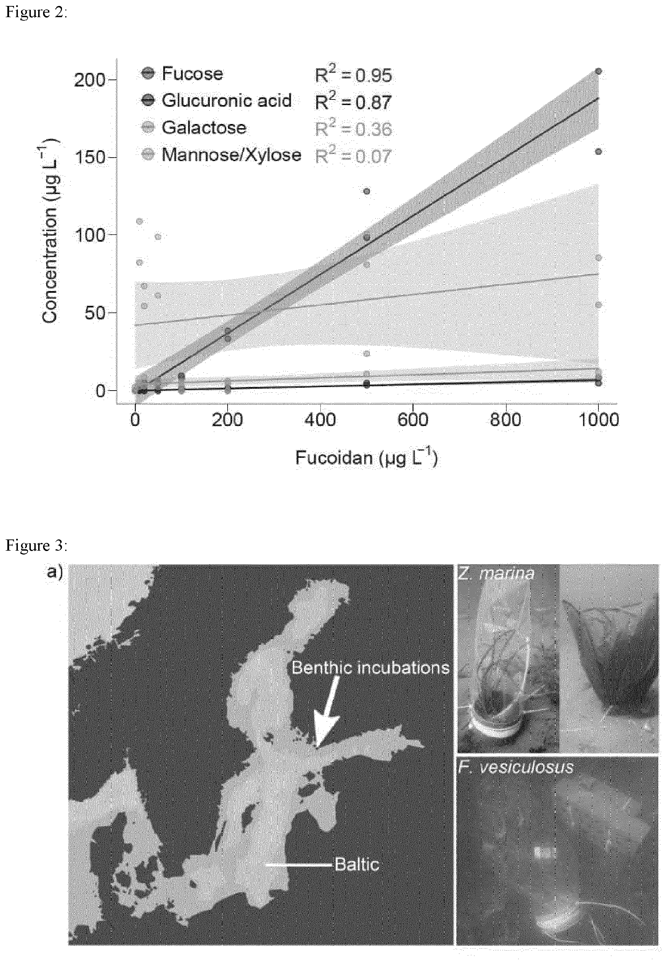

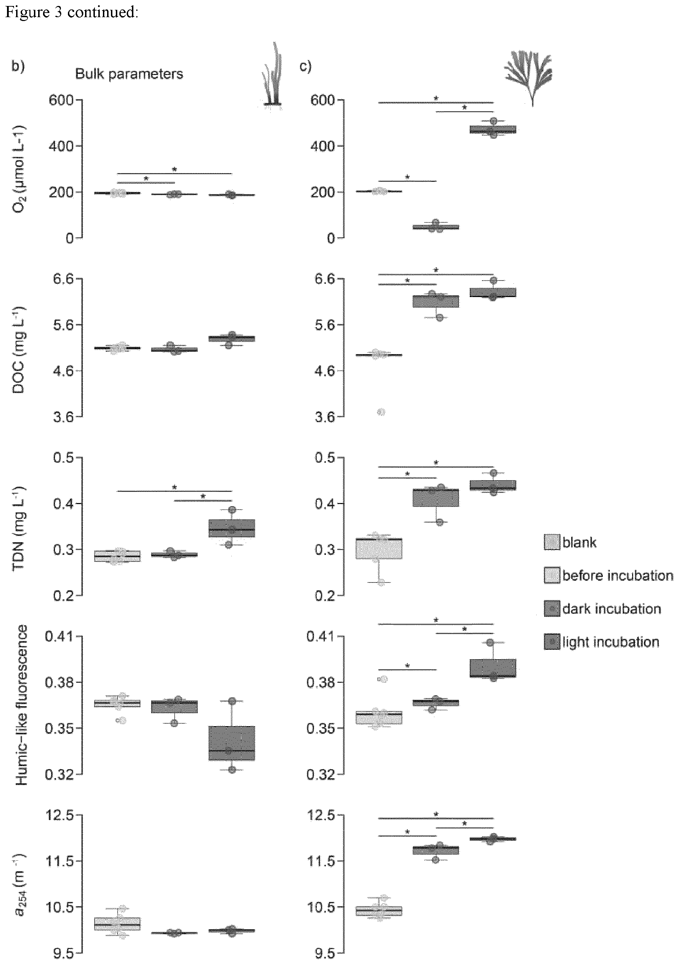

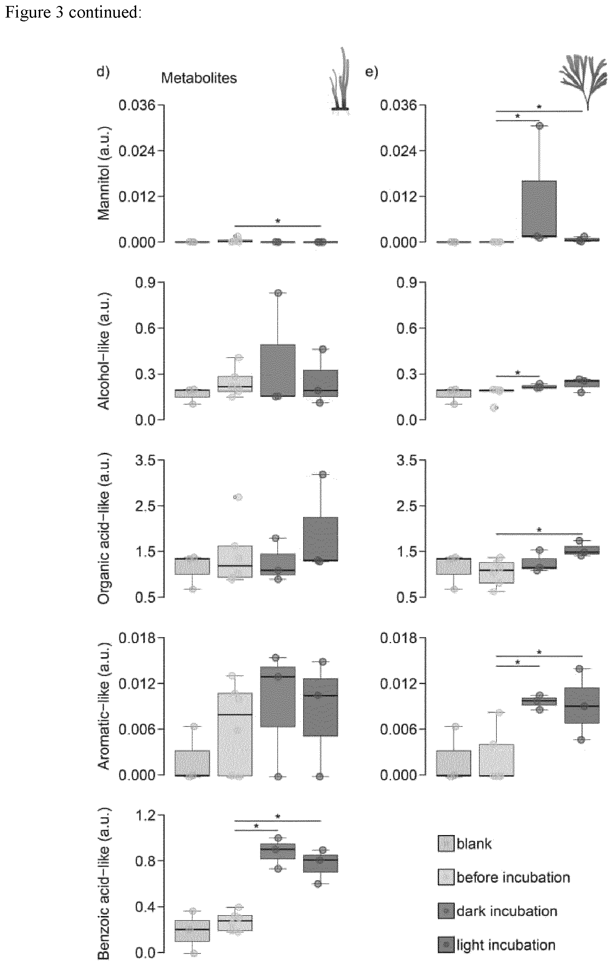

**Figure 3****: Dissolved organic carbon accumulated during brown algae incubations in the Baltic Sea in south-west Finland** (a, white arrow in left panel). Eelgrass, Zostera marina (a, top right panel), and bladderwrack, *Fucus vesiculosus* (a, bottom right panel), were incubated for five hours in transparent bags with and without shading. Before and after incubations, filtered seawater oxygen (O₂), dissolved organic carbon (DOC) and total dissolved nitrogen (TDN) concentrations along with humic-like fluorescence (peak c) and chromophoric dissolved organic matter (CDOM) absorption coefficient a₍₂₅₄₎ were determined for *Z. marina* (b) and *F. vesiculosus* (c). Accumulation of mannitol and four unidentified metabolites (in arbitrary units, a.u.) in incubations could be observed using SeaMet, a derivatization and gas chromatography-mass spectrometry approach for marine samples (d and e). Asterisks indicate statistical significance (Benjamini-Hochberg adjusted p < 0.05) according to Conover-Iman test (b and c) and Wilcoxon signed rank test (d and e).
**Figure 4****: A *F. vesiculosus* thallus used in the algae incubations.** The red circle indicates the location on the stipe that was cut when removing the algae from the bedrock. Every square on the mat is 1 cm².
**Figure 5****: Similar temperature** but **contrasting light conditions** between light and dark incubations of *Zostera marina* on August 5, 2020 (left panel) and *Fucus vesiculosus* on August 18, 2020 (right panel). Light intensity of PAR is given in µmol photons m⁻² s⁻¹.
**Figure 6****: Enzymes and AEX improve the specificity of fucoidan quantification** in a Bray-Curtis dissimilarity analysis on monosaccharide composition of algae incubation samples.
**Figure 7****: Monosaccharides in AEX extracts correlate with antibody signal linking glycan composition, charge and structure.** Carbohydrate microarrays containing the 5 M NaCl elutions were probed with monoclonal antibodies resulting in significant correlations for BAM1 (left panel) and BAM2 (middle panel) signal intensities in algae samples and BAM7 signal intensities (right panel) in seagrass samples. Fucose concentration in 5 M NaCl elutions correlated with BAM1 and BAM2 signal in algae samples, while fucose and galactose concentrations correlated with BAM7 signal in seagrass samples.
**Figure 8****: Mainly fucoidan, but also alginate monosaccharides increase during incubations** of *F. vesiculosus.* The figure shows monosaccharide concentrations after acid hydrolysis of filtered, dialyzed seawater samples.
**Figure 9****: Fucose produced by enzymatic hydrolysis of dialyzed AEX extracts.**
**Figure 10****: Up to 50% of *F. vesiculosus* secreted dissolved organic carbon was fucoidan.** a) Fucoidan obtained via AEX accounted for 13%, enzyme-confirmed fucoidan for 22% and fucoidan calculated from acid hydrolysis for 47% of secreted DOC. b) Elemental composition of fucoidans from various brown algae shows carbon and sulfur content along with near absence of nitrogen. Abbreviations: anion exchange chromatography (AEX), *Fucus* (*F*.), *Cladosiphon* (*C*.), *Durvillea (D.), Ecklonia* (*E*.).
**Figure 11****: CDOM absorbance and fluorescence excitation-emission matrices from nine fucoidan standards.** Crude fucoidans from various brown algae species systematically show higher absorbance and fluorescence in the 220/350 (Excitation/Emission) nm region, than their purified forms.
**Figure 12****: Quantification of fucoidan in seawater using anion exchange chromatography** was achieved. The two plots show results for fucoidan from two species of seaweed. Monosaccharide signals (points) in different colors for each monosaccharide reflect fucoidan concentrations enabling quantification.

The invention is illustrated in the following examples.

### Example 1: Fucoid brown algae inject fucoidan carbon into the ocean

### Materials and methods

Samples were collected in August 2020 at the coastal Ångbåtsbryggan monitoring site (62-64) in the Baltic Sea (59° 50' 28.284" N, 23° 14' 58.308" E). 800 mL ambient seawater from 1 and 3 m depth and from 5-hour incubations of subtidally growing *Zostera marina* and *Fucus vesiculosus* was filtered at 0.7 µm. Dissolved organic carbon, total dissolved nitrogen and CDOM/FDOM were measured at Tvärminne Zoological Station, Hanko, Finland. The experimental setup had a strong effect on oxygen concentrations in the incubations, beyond accurate measurability. Therefore, we do not report primary productivity rates. For the identification of small molecules, water samples were derivatized and analyzed by gas chromatography mass spectrometry (GC-MS) following a protocol for seawater samples (60). Anionic polysaccharides were extracted and purified from 400 mL of the filtered seawater (ambient and incubations) using anion exchange chromatography (AEX) and eluted with 5 M NaCl. 5 M NaCl elutions and remaining filtered seawater were transported frozen to the Max Planck Institute for Marine Microbiology in Bremen, Germany, for further purifications, monosaccharide quantification, GC-MS, antibody binding and enzymatic digestion.

Filtered seawater and 5 M NaCl elutions were dialyzed through 1 kDa membranes against MilliQ-water, dried and resuspended. Monosaccharides were quantified after acid hydrolysis (1 M HCl, 100°C, 24h) using anion exchange chromatography with pulsed amperometric detection (HPAEC-PAD). Filtered seawater was analyzed by ELISA adding aliquots into polymer-binding 96-well plates and measuring antibody binding via spectrophotometric signal, while the 5 M NaCl elutions were analyzed with carbohydrate microarrays by printing aliquots onto nitrocellulose membrane and measuring antibody binding via colorimetric signal (35). Crude and purified fucoidan from seven brown algae species were subjected to elemental analysis for carbon, nitrogen and sulfur content. To calculate daily fucoidan exudation rates, five of the daylight hours were considered at 100% light levels (exudation rates from light incubations), nine were considered to be in the dark (exudation rates from dark incubations), whilst we estimated 10 hours operating at 50% (averaged exudation rates from light and dark incubations) based on the average relative solar altitude.

### Polysaccharide extraction

Anionic polysaccharides were extracted from seawater using anion exchange columns. To this end, 100 mL of filtered seawater samples passed through 5 mL HiTrap ANX (high sub) FF cartridges (Cytiva, USA), which had been conditioned with 40 mL MilliQ-water, at 4 mL min⁻¹ flow rate. For washing, 20 mL MilliQ-water were applied after the sample and combined with the sample flow through. To elute weakly bound anionic molecules, 12 mL of a 2:1 mixture of 2 M ammonium bicarbonate and 2 M ammonium carbonate were passed over the cartridges at 2 mL min⁻¹ and collected in a 15 mL tube. To elute strongly anionic molecules, 30 mL 5 M NaCl were applied and collected in a 50 mL tube. The 15 mL and 50 mL tubes were frozen at -20°C until further processing.

### Enzymatic digestion

Enzymes were obtained from a fucoidan-degrading bacterial culture. A *Lentimonas* sp. CC4 culture (42) was grown on 0.2% fucoidan from *Fucus vesiculosus* (Merck/SigmaAldrich, Germany) as the only carbon source in 200 mL of MOPS-buffered minimal medium (42). 14 days after inoculation, the culture had reached an optical density of 0.714 at 600 nm wavelength. Cells were pelleted by centrifugation at 30 000 × g for 20 min at 4°C. After centrifugation, the supernatant was decanted and the cell pellet stored at -20°C. Cell pellet was resuspended in lysis buffer composed of 5 mL Bug Buster protein extraction kit (Merck/SigmaAldrich), 0.5 mg mL⁻¹ lysozyme from chicken egg white (Merck/SigmaAldrich), 0.1 mg mL⁻¹ DNAse (Merck/SigmaAldrich), and 1X cOmplete protease inhibitors (Merck/SigmaAldrich). Cell suspension was incubated for 15 min on ice with pipetting up and down and centrifuged at 24 000 × g for 30 min at 4°C to remove cell debris. The supernatant containing the soluble cytosolic proteins was transferred into clean tubes and kept on ice until digestion assays. The total protein concentration was measured using a bicinchoninic acid protein assay kit (Merck/SigmaAldrich) following the manufacturer's guidelines.

Dialyzed samples were combined with cell lysate containing catabolic enzymes specific to sulfated fucan to break down the polysaccharide into quantifiable monosaccharides. A 100 µL reaction mixture included 80 µL of dialyzed sample, 0.7% sea salt, 10 mM CaCl₂, and 10 µg of total proteins. A negative control contained heat-inactivated cell lysate to account for residual monosaccharides endogenous to bacterial cells. Samples were incubated for 3 h at 37°C. The reaction was stopped by heating the samples to 99°C for 15 min. Digests were then spun down to remove precipitates. Supernatant containing digestion products was collected, frozen and dried under vacuum. For monosaccharide quantification, dried samples were dissolved in 80 µL MilliQ-water, vortexed and centrifuged at 21 000 × g for 3 min to remove insoluble. For HPAEC-PAD analysis, samples were further diluted 10 times with MilliQ-water.

Fucose was the monosaccharide product of which concentration was significantly correlated with fucoidan concentration of the input (**Fig. If,** **Fig. 2**). Thus, fucose concentration was used to estimate fucoidan concentrations in samples. Even though galactose and glucuronic acid have strong correlation, their constitution was low. Thus, galactose and glucuronic acid are less sensitive estimators for fucoidan especially in environmental samples and in enzymatic digestion assays. Therefore, fucoidan concentration is calculated based on fucose content only.

### Study site

We chose a study site in the central Baltic for which long-term monitoring data is available and exhibits abundant populations of key algal and plant species. Rocky shores with abundant bladderwrack, *Fucus vesiculosus,* and vegetated soft sediments with eelgrass, *Zostera marina*, and a variety of aquatic plants with limnic origin dominate the shallow coastal region here (83). The area can be characterized as being microtidal with the salinity commonly ranging between 5-6. The benthic vegetation shows strong seasonal patterns and both Z. *marina* and *F*. *vesiculosus* tend to have lower biomass in winter and spring with a maximum biomass in late summer/early fall (84, 85). CTD environmental conditions temperature, salinity, oxygen concentration, pH and turbidity in hourly intervals at 4 m depth at Ångbåtsbryggan (DATA AVAILABLE https://www.helsinki.fi/en/ research-stations/tvarminne-zoological-station/research/monicoast/online-data). We used a sparse, monospecific seagrass bed of Z. *marina* in the vicinity of the CTD mooring at 4 meters depth (59°50'30.6"N 23°14'56.4"E) for incubations along with *F. vesiculosus,* lining the shore approx. 50 meters north-westward (59°50'30.8"N 23°14'53.7"E).

### Incubations

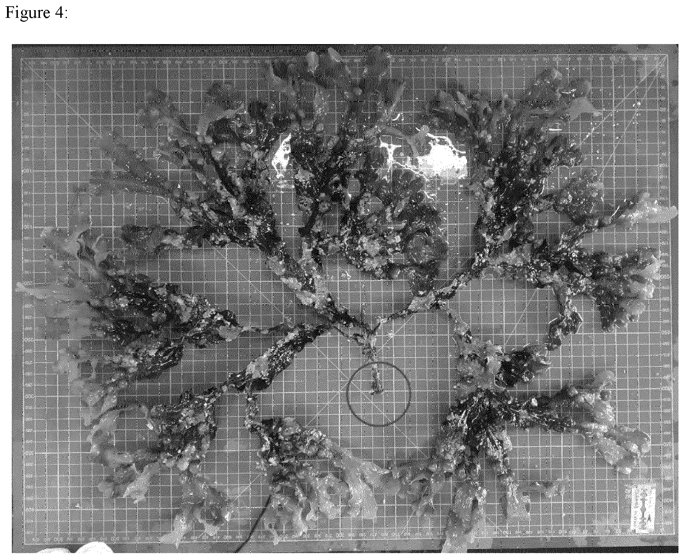

Field *in situ* incubations aimed at capturing organic molecules released from seagrass and algae under light and dark conditions. In total, six seagrass incubations and six algae incubations were set up. On the day of the incubations, August 5, 2020, seagrass shoots were enclosed in transparent gas impermeable plastic vacuum bags (Packing24, PA/PE 20/70, 90my) with approx. 6 L of the surrounding water. In the case of benthic seagrass incubations, the bags were sealed onto the previously installed PVC tubes (**Fig. 3a****, top right panel**). In the case of algae incubations on August 18, 2020, transparent gas impermeable plastic vacuum bags with a single *F. vesiculosus* thallus, were closed with rubber bands around PVC end cap fitting of 12 cm diameter and attached to a bottom-anchored line at ~1 m depth (**Fig. 3a****, bottom right panel**). Thalli of ~50 cm length were detached close to the base by cutting the few millimeters thick, old stipe (**Fig. 4**). The incubations of the algae could not be conducted without removing them from the bedrock, as it was not possible to attach benthic chambers on the bedrock surface with a gas tight construction. However, the exudation values were within range of other studies where holdfasts were not cut, and we did not find evidence of major cell leakage of intracellular metabolites, such as mannose (86). Half of the incubations were covered with black bags to simulate dark conditions. Immediately prior to starting the incubations, water was sampled from the ambient water to determine initial conditions.

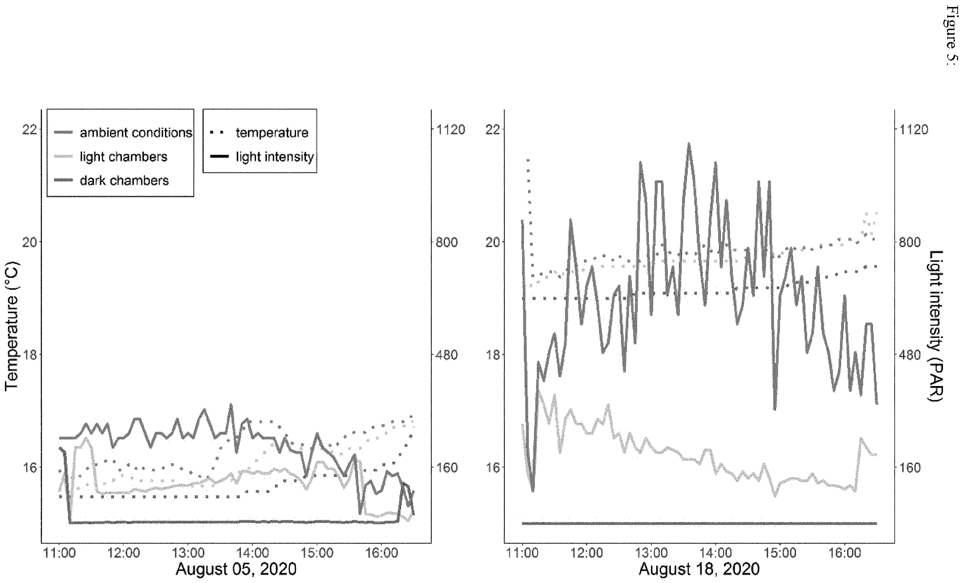

We monitored temperature and irradiation in light-exposed and dark bags as well as in the ambient environment over the course of the incubations (**Fig. 5**). Light loggers (HOBO Pendant^{®} Temperature/Light Data Logger 64 K; Onset Computer Corporation, USA) were programmed to record temperature and irradiation in 5 min intervals. Three loggers were deployed attached to metal rods and stuck into the sediment for monitoring during seagrass incubations or attached to plastic disks during algae incubations. One rod with logger measured inside a light-exposed incubation, a second inside a dark incubation, and a third adjacent to the incubations. The latter logger was complemented with a photosynthetically active radiation (PAR) sensor (RBRsolo Single Channel Logger with a Li-Cor^{®} -192, RBR Ltd., Canada) also recording in 5 min intervals to calibrate the logger data to PAR expressed in Photosynthetic Photon Flux Density (µmol m⁻² s⁻¹).

At the end of the incubation, all seagrass (above- and belowground) and algal tissue was collected. In the laboratory, the length and width of each seagrass shoot was measured, while the algal thallus was spread out across a cutting mat with a grid for surface area estimation. Epiphytes and epifauna were scraped off shoots and thalli using a razor blade, and collected separately from the plant/algal biomass. All biomass was frozen and subsequently freeze-dried for 24 hours, and weighed to measure dry weight.

### Water sample collection

After five hours, we sampled water from the incubations and collected the incubated seagrass, sediment and algae. Water column samples from seagrass incubations were collected into acid washed single use, sterile, 60 mL syringes (Thermo Fisher Scientific, USA) via water column ports on the PVC tubes. Water column samples from algae incubations were collected into acid washed 60 mL syringes after opening incubation bags on board. Water column samples were filtered immediately through a precombusted 45 mm diameter glass microfiber GF/F filter (Whatman, UK). For each sample, 30 mL were pushed through the filter for rinsing before starting collection. To determine the concentration of dissolved organic carbon (DOC), 15 mL sample were filtered into a precombusted 24 mL glass vial containing 60 µL 25% hydrochloric acid and the vial sealed with an acid washed teflon-lined cap. Another 10 mL of sample were filtered into a precombusted 12 mL glass vial and the vial sealed with an acid washed teflon-lined cap for colored dissolved organic matter (CDOM) and fluorescent dissolved organic matter (FDOM) analysis. Subsamples for DOC were frozen at -20°C and samples for CDOM/FDOM were stored at 4°C until analysis within two weeks. For gas chromatography-mass spectrometry (GC-MS), 2 mL samples were filtered into 2 mL microtubes. About 600 mL of filtered water column samples and 30-60 mL porewater samples were collected in an acid washed PP or HDPE bottle (Thermo Fisher Scientific, USA) for extractions.

The chamber volume of the algal incubations was measured directly with a graduated cylinder and adding the amount of water that was sampled. We attempted to measure the volume of the seagrass incubations by injecting a saline solution and measure the change in salinity. However, the conductivity meter used was not sensitive enough to accurately measure the change, and the benthic chamber volume estimation was not possible.

### Water sample analyses

Optical FireSting O₂ sensors (Pyroscience GmbH, Germany) determined oxygen concentrations in water samples. Calibration of the sensors was achieved using MilliQ water. Oxygen concentrations were measured in 600 mL subsamples from water column within two hours of sample collection. For algae incubations, oxygen concentrations could also be determined in water from incubations without prior filtration.

To determine the total dissolved nitrogen (TDN), the samples were spectrophotometrically analyzed using an autoanalyzer (Aquakem 250). In addition, DOC-concentrations were measured using a Shimadzu TOC-V_{CPH}-analyzer.

Colored DOM (CDOM) absorption was measured using a Shimadzu 2401PC spectrophotometer with 5 cm quartz cuvette over the spectral range from 200 to 800 nm with 1 nm resolution. Ultrapure water was used as the blank for all samples. Excitation-emission matrices (EEMs) of fluorescent DOM (FDOM) were measured with a Varian Cary Eclipse fluorometer (Agilent). Processing of the EEMs was done using the *eemR* package for R software (87). A blank sample of ultrapure water was subtracted from the EEMs, and the Rayleigh and Raman scattering bands were removed from the spectra after calibration. EEMs were calibrated by normalizing to the area under the Raman water scatter peak (excitation wavelength of 350 nm) of an ultrapure water sample run on the same session as the samples, and were corrected for inner filter effects with absorbance spectra (88).

### GC-MS analysis

For metabolomics on seawater, 2 mL samples for GC-MS were thawed and 0.5 mL subsamples taken. Processing blanks consisted of artificial seawater (ASW) prepared at a salinity of 3.6 % (w/v) and diluted 1:5 to match Baltic Sea salinity. For a liter of ASW, 26.37 g sodium, 6.8 g magnesium sulfate heptahydrate, 5.67 g magnesium chloride hexahydrate, 1.47 g calcium chloride, 0.6 g potassium chloride and 0.09 g potassium bromide were dissolved in MilliQ-water and autoclaved. Heat-sensitive elements were added to the following final concentrations: 2.5 mM sodium bicarbonate, 150 µM monopotassium phosphate, 500 µM ammonium chloride, 7.55 nM iron sulfate, 4.85 nM boric acid, 0.51 nM manganese chloride, 0.8 nM cobalt chloride, 0.1 nM nickel chloride, 0.06 nM copper chloride, 0.5 nM zinc sulfate, 3.72 nM sodium permanganate, 0.11 nM sodium selenite, 0.02 nM sodium tungstate, 10 nM 4-aminobenzoic acid, 10 nM D-biotin, 100 nM nicotinic acid, 50 nM calcium D-pantothenate, thiamine, 0.8 nM thiamine chloride dihydrochloride, and 36.9 pM cyanocobalamin. For standards, 100 µL of a mixture with 50 metabolites at 0.4 mM concentration each **(Table 5**) were added to diluted ASW. Subsamples, processing blanks and standards were spiked with 100 µL cholestane at 1 mM in ethanol and 40 µL ribitol at 0.2 mg mL⁻¹, dried at 45°C for 6 h in a Concentrator plus speedvac (Eppendorf, Germany) and frozen until further processing. Immediately prior to the derivatization, samples were again dried for 20 min to remove any moisture. After drying, 250 µL anhydrous toluene were added and samples ultrasonicated for 10 min at 100 % intensity in an ultrasonication bath. In a chamber with dry air (humidity <5 %), samples were dried under constant nitrogen gas stream. To each sample, 80 mL of 20 mg mL⁻¹ methoxamine in pyridine were added, samples ultrasonicated for 10 min at 100 % intensity and subsequently incubated for 90 min at 37°C shaking at 1350 rpm. Samples were dried again under constant nitrogen gas stream and 100 µL of N,O-Bis(trimethylsilyl)trifluoroacetamide (BSTFA) added. After ultrasonication for 10 min at 100 % intensity and brief vortexing, samples were incubated for 30 min at 37°C shaking at 1350 rpm. Finally, the derivatized samples were ultrasonicated for 10 min at 100% intensity, centrifuged for 2 min at 21 100 × g and 100 µL of clear supernatant transferred to a GC-vial for analysis.

Samples were analyzed within 24 h after derivatization on an Agilent 7890B gas chromatograph coupled to an Agilent 5977A single quadrupole mass selective detector. To this end, an Agilent 7693 autosampler injected 1 µL of sample in splitless mode through the inlet liner onto an Agilent DB-5MS column (30 m length, 0.25 mm diameter, 0.25 µm film thickness) with Agilent DuraGuard guard column (10 m length). Injector temperature was set to 290°C. Chromatographic separation was achieved with helium as carrier gas at a constant flow of 1 mL min⁻¹. The column oven temperature was increased from 60°C at injection to 325°C over 13.25 min and maintained at 325°C for 2 min. The mass detector acquired spectra from electron ionization at 70eV in a mass range of 50-600 m/z at a scan rate of 3 s⁻¹.

### Dialysis

To desalt eluates collected from polysaccharide extractions, we dialyzed eluate subsamples against MilliQ-water. The eluates in 2 M ammonium bicarbonate/carbonate or 5 M NaCl were thawed and vortexed rigorously. Subsamples of 4 up to 10 mL were transferred into prerinsed 1 kDa membrane (SpectraPor^{®} Biotech CE Dialysis Membrane, Carl Roth, Germany) with MilliQ-water controls in separate dialysis tubing to account for processing artifacts. Salt and small molecules were removed by dialyzing three times against 10 L MilliQ-water at 4°C. Dialyzed samples were transferred to 15 mL centrifuge tubes, freeze dried and resuspended in MilliQ-water.

### Acid hydrolysis

Dialyzed and freeze dried samples were resuspended in MilliQ-water, of which 200 µL were combined with 200 µL 2 M HCl in a precombusted glass ampule. Ampules were sealed and incubated for 24 h at 100°C to achieve complete acid hydrolysis of polysaccharides in the sample. After acid hydrolysis, samples were transferred from glass ampules to microtubes and dried in an acid proof vacuum concentrator (Martin Christ Gefriertrocknungsanlagen GmbH, Germany). Dried, hydrolyzed samples were reconstituted in MilliQ-water, diluted if necessary and transferred to 100 µL glass inlets in HPLC vials for HPAEC-PAD analysis.

### Monosaccharide quantification

Monosaccharides in samples were separated via anionic interaction chromatography and individually quantified using electrochemical detection, as reported previously (89). For analysis, 25 µL of sample were autosampler-injected into a DIONEX ICS 5000+ equipped with a 2 × 250 mm PA10 column with corresponding guard (all Thermo Fisher Scientific, USA). Separation of neutral monosaccharides was achieved during an isocratic phase with 18 mM NaOH, followed by separation of acidic monosaccharides during a gradient up to 200 mM NaCH₃COO. Monosaccharides were detected using pulsed amperometric detection. Based on quantitative standards, monosaccharides were identified by retention times and peak areas fitted to the standard series, which consisted of arabinose, fucose, galactosamine, galactose, galacturonic acid, gluconic acid, glucose, glucuronic acid, iduronic acid, mannose, mannuronic acid, rhamnose, and xylose. Mannose and xylose peaks eluted too close to confidently distinguish between and peak areas are reported combined.

### Antibody-based methods

Enzyme-linked immunosorbent assay (ELISA) was used to detect fucoidan epitopes in the dialyzed samples from algae incubations. 50 µL of sample and 50 µL of phosphate buffered saline (PBS, ×1) were added in the wells of a microtitre plate (NUNC Maxisorp, Thermo Fisher Scientific). Each sample was added in triplicate and plates were incubated at 4 °C overnight. Plate contents were discarded and wells were washed six times with 200 µL deionized water. Wells were blocked with 200 µL PBS with 5% (w/v) low-fat milk powder (MPBS) for 2 h and washed nine times with 200 µL deionized water. Wells were incubated for 1.5 h with 100 µL of BAM1 or BAM2 (SeaProbes, Roscoff, France) antibody solution diluted 1:10 in MPBS. After antibody probing, wells were washed nine times with 200 µL deionized water. Wells were incubated 1.5 h with 100 µL of secondary antibody conjugated to horseradish peroxidase (A9037, Sigma-Aldrich) diluted 1:1000 in MPBS. Wells were washed nine times with 200 µL deionized water. The plate was developed with 100 µL ELISA tetramethylbenzidine substrate per well. Development reaction was stopped with 100 µL 1 M HCl and absorbance in wells read at 450 nm using a SpectrostarNano absorbance plate reader and MARS software (BMGlabtech).

AEX elutions were analyzed by carbohydrate microarray analysis as described previously (90). In brief, aliquots of the dialyzed 5 M NaCl elutions from AEX were added into wells of 384-microwell plates and the content of the plates printed onto nitrocellulose membrane with a pore size of 0.45 µm (Whatman) using a microarray robot (Sprint, Arrayjet, Roslin, UK). Each sample was printed in duplicates. The printed microarrays were blocked in MPBS for 1 h. Next, each microarray was individually incubated for 2 h with the monoclonal antibodies BAM1, BAM2 or BAM7 (SeaProbes, Roscoff, France) diluted 1:10 in MPBS. After probing, arrays were thoroughly washed in PBS and incubated for 2 h with a secondary antibody conjugated to alkaline phosphatase (A8438, Sigma-Aldrich) diluted 1:5000 in MPBS. Arrays were washed in PBS and deionized water and developed in a solution containing 5-bromo-4-chloro-3-indolylphosphate and nitro blue tetrazolium in alkaline phosphatase buffer (100 mM NaCl, 5 mM MgCl₂, 100 mM Tris-HCl, pH 9.5). Developed arrays were analyzed as described previously (90) using Array-Pro Analyzer 6.3 (Media Cybernetics). The highest mean antibody signal intensity detected in the data set (obtained with alginate from brown algae - A1112, Sigma Aldrich - probed with BAM7, which was included as a positive control) was set to 100 and all other values were normalized accordingly.

### Statistical analysis

All statistical analyses and calculations were performed in R version 4.0.3 in RStudio version 1.3.1093. Due to the small sample size and violation of assumptions of parametric tests, we used non-parametric tests to compare dissolved compounds in the benthic chambers experiment. For the bulk parameters (dissolved oxygen, dissolved organic carbon and total nitrogen, humic-like fluorescence and absorption at 254 nm of dissolved compounds), a Kruskal-Wallis rank sum test (*R Base*), followed by a *post hoc* Conover-Iman test *(conover. test* package (91)) for pairwise comparisons, was used to test for significant differences between filtered seawater (n = 6), dark incubations (n = 3) and light incubations (n = 3) (**Fig. 3b****, c**). The peak picking of the metabolomics data was performed with the R package *xcms* (92). Each sample was normalized by the intensity of the internal standard, ribitol, quantification ion. A Wilcoxon rank sum test (*rstatix* package (93)) was used for pairwise comparisons between filtered seawater and either dark incubations or light incubations to find five metabolites which differed significantly between the sampling points (**Fig. 3d****, e**). Comparing with reference spectra and standards from the MassBank of North America (MoNA) and analytical standards **(Table 5**), one of the five metabolites was identified as mannitol, while the other four were labeled unidentified alcohol-like, unidentified small organic acid-like, unidentified small aromatic-like and unidentified benzoic acid-like compounds.

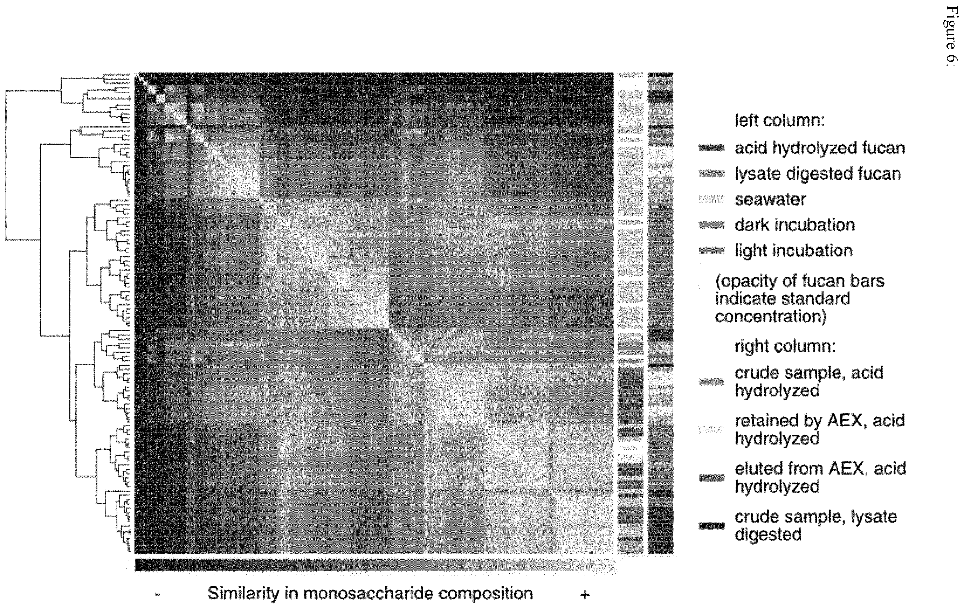

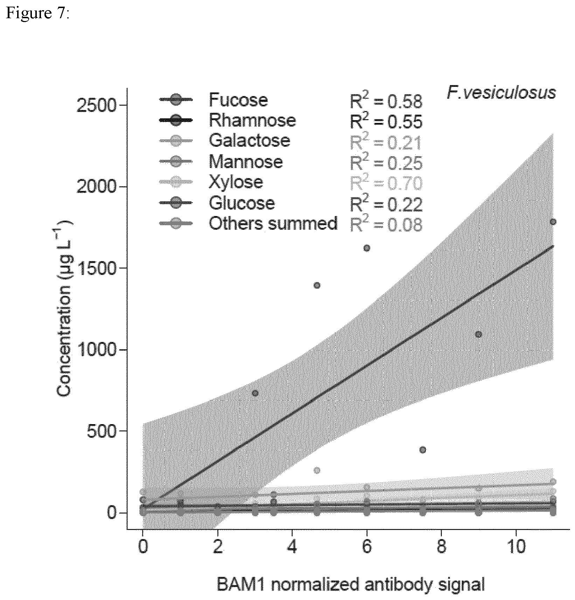

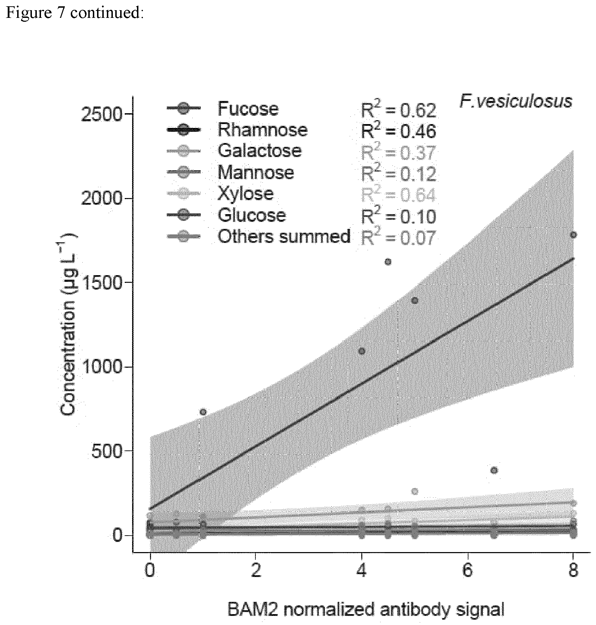

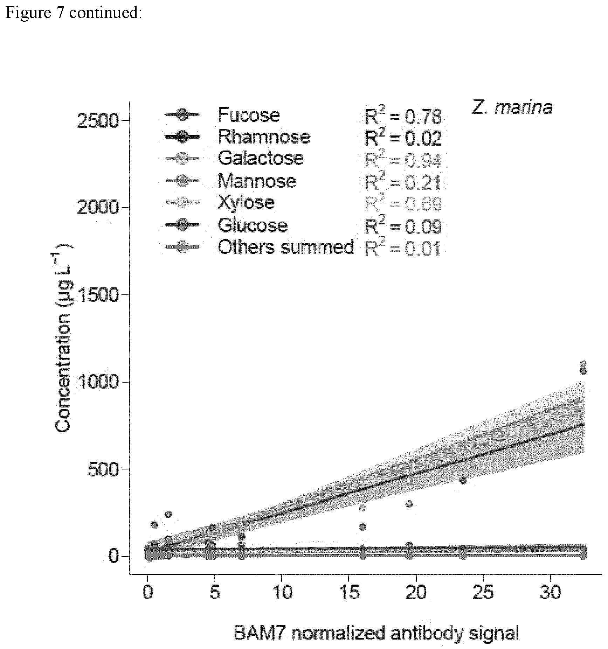

Concentrations of the complete monosaccharide composition in raw samples were compared using multivariate statistics (*vegan* package(94)). Eigenvalues of monosaccharides were computed from a redundancy analysis on all monosaccharide concentrations. A Bray-Curtis dissimilarity analysis (*vegan* package) was used to compare monosaccharide composition between samples. To this end, abundances were normalized to the most abundant monosaccharide in a sample. Relative abundances were used to compute a distance matrix based on the Bray-Curtis method. Samples were clustered hierarchically using the generated distance matrix with complete linkage. Similarities in monosaccharide composition between samples were visualized in a heatmap (**Fig. 6**). Variance structure-corrected generalized linear models were explored to assess significant differences between blanks, seawater and incubation samples for individual monosaccharides with a focus on fucose, the main monomer of fucoidan. However, the data did not fulfill model assumptions of variance heterogeneity. Log-transformation evened the spread to some extent, but not sufficiently to apply parametric tests. Linear regression models (*R Base*) were used to quantify the correlations between 1) monosaccharide concentrations from acid hydrolysis with the summed signal intensities of fucoidan antibodies BAM1 and BAM2 (**Fig. 1b**); 2) monosaccharide concentrations from AEX extracts and the signal intensities of fucoidan antibodies BAM1 and BAM2, and the alginate antibody BAM7 (**Fig. 7**); 3) enzyme-produced monosaccharides and standard concentrations of a fucoidan standard (**Fig. 1**); and 4) acid-produced monosaccharides and standard concentrations of a fucoidan standard (**SFig. 2**).

Linear mixed models (*lme4* package (95)) were used to compare the groups of blanks (n = 9), filtered seawater before incubations (n = 18), dark incubations (n = 9) and light incubations (n = 9) for antibodies BAM1 and BAM2 (**Table 8**). Analytical triplicates were used for each sample, and thus, a random effect of sample number was used. To compare between fucoidan concentrations the groups quantified by acid hydrolyzed filtered seawater, acid hydrolyzed AEX extracts, and enzyme hydrolyzed filtered seawater, a non-parametric Kruskal-Wallis rank sum test, followed by a *post hoc* Conover-Iman test for pairwise comparisons (**Table 8**).

**Table 1. Results from Kruskal-Wallis rank sum tests and post-hoc Conover-Iman pairwise comparisons of the concentrations of parameters dissolved oxygen (O2), dissolved organic carbon (DOC), total dissolved nitrogen (TDN), Peak c (humic-like fluorescence), and absorption coefficient a₍₂₅₄₎ between initial seawater, end of light algae incubations, and end of dark algae incubations. Abbreviations: degrees of freedom (df), p-value (p), T-statistic (T) and chi-square statistic (χ²).**

| *Parameter* | *O₂* | | | *DOC* | | | *TON* | | | *Peak c* | | | *a₍₂₅₄)* | | |
|---|---|---|---|---|---|---|---|---|---|---|---|---|---|---|---|
| *Kruskal-Wallis rank sum test* | | | | | | | | | | | | | | | |
| | χ² | df | *p* | χ² | df | *p* | χ² | df | *p* | χ² | df | *p* | χ² | df | *p* |
| | 9.3 | 2 | 0.01 | 7.6 | 2 | 0.02 | 7.6 | 2 | 0.02 | 7.6 | 2 | 0.02 | 9.4 | 2 | 0.01 |

| *Conover-Iman pairwise comparison* | | | | | | | | | | | | | | | |
|---|---|---|---|---|---|---|---|---|---|---|---|---|---|---|---|
| | T | | *p* | T | | *p* | T | | *p* | T | | *p* | T | | *p* |
| *initial-dark* | 4.1 | | 0.001 | -3.8 | | 0.004 | -3.8 | | 0.004 | -1.9 | | 0.044 | -4.2 | | 0.002 |
| *initial-light* | -4.1 | | 0.002 | -4.6 | | 0.003 | -4.6 | | 0.003 | -4.5 | | 0.002 | -6.9 | | <0.001 |
| *dark-light* | -7.1 | | <0.001 | -0.7 | | 0.258 | -0.7 | | 0.258 | -2.2 | | 0.040 | -2.4 | | 0.020 |

**Table 2. Results from Kruskal-Wallis rank sum tests and post-hoc Conover-Iman pairwise comparisons of the concentrations of parameters dissolved oxygen (O2), dissolved organic carbon (DOC), total dissolved nitrogen (TDN), Peak c (humic-like fluorescence), and absorption coefficient a₍₂₅₄₎ between initial seawater, end of light seagrass incubations, and end of dark seagrass incubations. Abbreviations: degrees of freedom (df), p-value (p), T-statistic (T) and chi-square statistic (χ²).**

| *Parameter* | *O₂* | | | *DOC* | | | *TON* | | | *Peak c* | | | *a₍₂₅₄)* | | |
|---|---|---|---|---|---|---|---|---|---|---|---|---|---|---|---|
| *Kruskal-Wallis rank sum test* | | | | | | | | | | | | | | | |
| | χ² | df | *p* | χ² | df | *p* | χ² | df | *p* | χ² | df | *p* | χ² | df | *p* |
| | 7.1 | 2 | 0.029 | 5.3 | 2 | 0.07 | 6.5 | 2 | 0.039 | 2.0 | 2 | 0.375 | 3.2 | 2 | 0.197 |

| *Conover-Iman pairwise comparison* | | | | | | | | | | | | | | | |
|---|---|---|---|---|---|---|---|---|---|---|---|---|---|---|---|
| | T | | *p* | T | | *p* | T | | *p* | T | | *p* | T | | *p* |
| *initial-dark* | 2.2 | | 0.039 | ns | | ns | -0.7 | | 0.253 | ns | | ns | ns | | ns |
| *initial-light* | 3.9 | | 0.005 | ns | | ns | -3.6 | | 0.009 | ns | | ns | ns | | ns |
| *dark-light* | 1.5 | | 0.088 | ns | | ns | -2.5 | | 0.026 | ns | | ns | ns | | ns |

**Table 3. Results from Wilcoxon rank sum tests of the signal of metabolites mannitol, alcohol-like, organic acid-like, and aromatic-like metabolites between initial seawater, and end of light and dark algae incubations. Abbreviations: W-statistic (W), p-value (p)**

| *Parameter* | *Mannitol* | | *Alcohol-like* | | *Organic acid-like* | | *Aromatic-like* | |
|---|---|---|---|---|---|---|---|---|
| | W | *p* | W | *p* | W | *p* | W | *p* |
| *initial-dark* | 0 | 0.024 | 0 | 0.024 | 5 | 0.381 | 0 | 0.024 |
| *initial-light* | 0 | 0.024 | 5 | 0.381 | 0 | 0.024 | 1 | 0.048 |

**Table 4. Results from Wilcoxon rank sum tests of the concentrations of metabolites mannitol, alcohol-like, organic acid-like, and aromatic-like and benzoic-like metabolites between initial seawater, end of light algae incubations, and end of dark algae incubations. Abbreviations: W-statistic (W), p-value (p).**

| *Parameter* | *Mannitol* | | *Alcohol-like* | | *Organic acid-like* | | *Aromatic-like* | | *Benzoic acid-like* | |
|---|---|---|---|---|---|---|---|---|---|---|
| | W | *p* | W | *p* | W | *p* | W | *p* | W | *p* |
| *initial-dark* | 16 | 0.095 | 10 | 0.905 | 9 | 1 | 6 | 0.548 | 0 | 0.024 |
| *initial-light* | 18 | 0.024 | 10 | 0.905 | 6 | 0.548 | 7 | 0.714 | 0 | 0.024 |

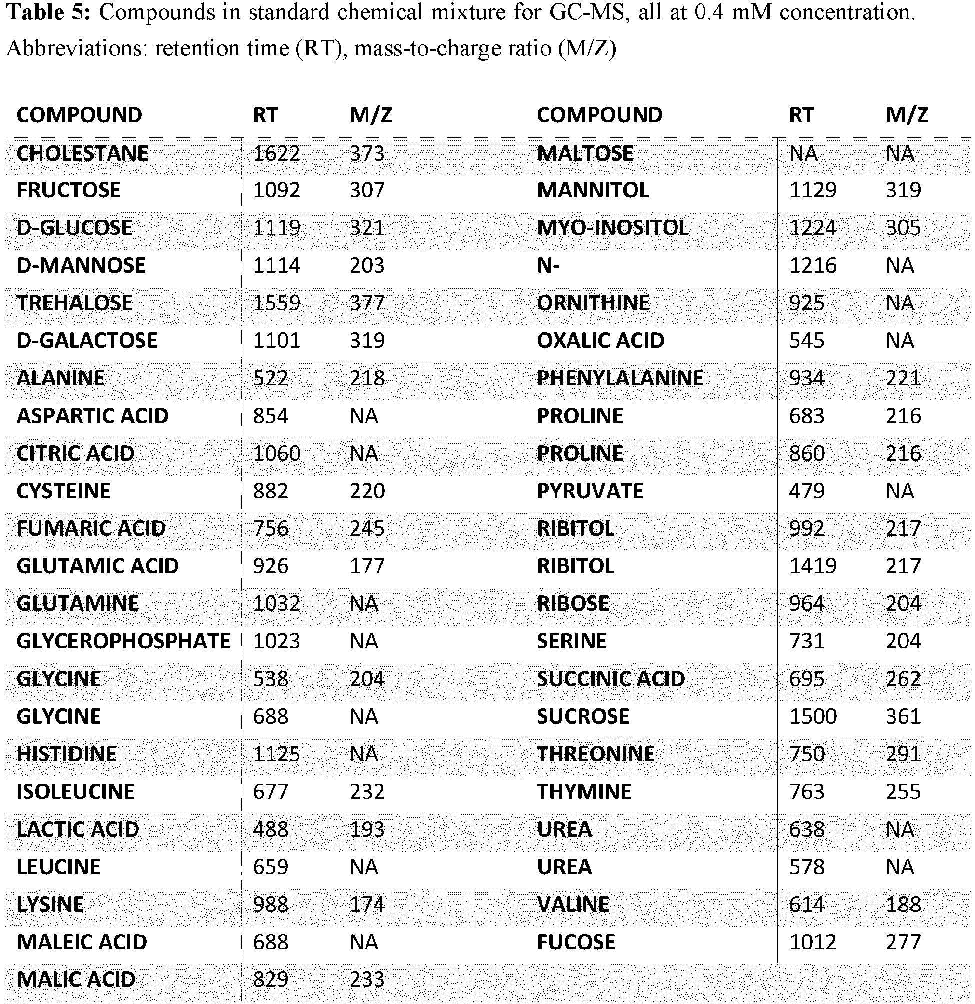

**Table 6. Results from a three-way analysis of variance (ANOVA) of fucoidan signal between blanks, initial seawater, end of light and dark seagrass incubations for the antibody-based technique ELISA (BAM1 and BAM2), and a Kruskal-Wallis rank sum test for fucoidan estimates from acid hydrolysis of filtered seawater, acid hydrolysis of anionic exchange (AEX) extracts, and enzyme digestions. Abbreviations: degrees of freedom (df), p-value (p), F-statistic (F) and chi-square statistic (χ²).**

| *Three-way Analysis of Variance* | | | | | | |
|---|---|---|---|---|---|---|
| | *Sums of squares* | *Mean square* | *Numerator df* | *Denominator df* | *F* | *p* |
| *BAM1* | 1.7 | 0.6 | 3 | 8.2 | 63.2 | <0.001 |
| *BAM2* | 12.4 | 4.1 | 3 | 3.5 | 751.7 | <0.001 |

| *Kruskal-Wallis rank sum test* | | | | | | |
|---|---|---|---|---|---|---|
| | | | df | | χ² | *p* |
| *acid* | | | 3 | | 12.6 | 0.010 |
| *AEX* | | | 3 | | 10.3 | 0.016 |
| *enzyme* | | | 3 | | 11.2 | 0.011 |

**Table 7. Results from post-hoc tests Tukey's Honest Significant Difference (HSD) following the three-way ANOVA, and a Conover-Iman pairwise comparison, following the Kruskal-Wallis rank sum test in Table 5. Abbreviations: p-value (p), T-statistic (t-ratio or T).**

| *Analytical technique* | *BAM1* | | *BAM2* | | *acid* | | *AEX* | | *enzyme* | |
|---|---|---|---|---|---|---|---|---|---|---|
| | Tukey's HSD test | | | | Conover-Iman pairwise comparison | | | | | |
| | t-ratio | *p* | t-ratio | *p* | T | *p* | T | *p* | T | *p* |
| *blank-dark* | 12.1 | <0.001 | 29.6 | <0.001 | -7.1 | <0.001 | -4.2 | 0.002 | -3.2 | 0.006 |
| *blank-initial* | -9.2 | <0.001 | -1.7 | 0.463 | -3.9 | 0.001 | -1.5 | 0.086 | 0 | 0.500 |
| *blank-light* | -12.6 | <0.001 | -28.4 | <0.001 | -8.8 | <0.001 | -6.2 | <0.001 | -4.6 | <0.001 |
| *initial-dark* | 5.5 | 0.002 | 36.6 | <0.001 | -4.2 | 0.001 | -3.6 | 0.005 | -3.4 | 0.005 |
| *initial-light* | -6.2 | <0.001 | -35 | <0.001 | -6.3 | <0.001 | -6 | <0.001 | -5 | <0.001 |
| *dark-light* | -0.6 | 0.934 | 1.4 | 0.551 | -1.8 | 0.053 | -2.2 | 0.035 | -1.4 | 0.118 |

### RESULTS

Brown algae annually convert gigatons of carbon dioxide into carbohydrates, including the complex extracellular matrix polysaccharide fucoidan. Due to its persistence in the environment, fucoidan is potentially a pathway for marine carbon sequestration. Rates of fucoidan secretion by brown algae remain unknown due to the challenge of identifying and quantifying complex polysaccharides in seawater. We adapted the techniques of anion exchange chromatography, enzyme-linked immunosorbent assay and biocatalytic enzyme-based assay for detection and quantification of fucoidan. We found the brown alga *Fucus vesiculosus* at the Baltic Sea coast of south-west Finland to secrete 0.3% of their biomass as fucoidan per day. Dissolved fucoidan concentrations in seawater adjacent to algae reached up to 0.48 mg L⁻¹. Fucoidan accumulated during incubations of *F. vesiculosus,* significantly more in light than in darkness. Maximum estimation by acid hydrolysis indicated fucoidan secretion at a rate of 28-40 mg C kg⁻¹ h⁻¹, accounting for 44-50% of all exuded dissolved organic carbon. Composed only of carbon, oxygen, hydrogen and sulfur, fucoidan secretion does not consume nutrients enabling carbon sequestration independent of algal growth. Extrapolated over a year, the algae sequester more carbon into secreted fucoidan than their biomass. The global utility of fucoidan secretion is an alternative pathway for carbon dioxide removal by brown algae without the need to harvest or bury algal biomass.

Combining rapid carbon dioxide fixation and growth, brown algae shape atmosphere chemistry that regulates climate. Brown algae exude dissolved organic molecules. Some of these molecules provide long-term carbon storage in the dissolved organic carbon pool, which holds substantial amounts of carbon. Accounting the carbon that comes from exudates requires analytic techniques to identify and quantify its molecules in the ocean. In this article, we show how to count carbon of the extracellular matrix polysaccharide fucoidan and found it is a substantial amount. This quantification of fucoidan can assess its contribution to carbon sequestration. *Fucus vesiculosus* contributes to carbon sequestration through the secretion of fucoidan without concomitant nutrient removal, expanding algal carbon dioxide removal to include secreted molecules.

Brown algae fix more carbon per area than terrestrial forests (1-4) and brown algal biomass is considered a potential carbon sink (5-9). However, algae release 14-35% of net primary production as dissolved molecules (10-15). Whether these exudates fuel microbial remineralization or contribute to carbon sequestration through recalcitrance or export to the deep sea remains under debate. Dissolved organic carbon concentrations decrease offshore with distance to kelp forests, but the high molecular weight polysaccharide fraction has shown considerable persistence (12, 16, 17). Remineralization rates of carbohydrates depend on molecular structure (18). There is a need to constrain the molecular composition of brown algal exudates to understand their role in the ecosystem and in the global carbon cycle.

Primary producers, including algae, fix carbon into carbohydrates (19), which serve a multitude of functions. Brown algae mainly synthesize the polysaccharides laminarin, cellulose, alginate and fucoidan: laminarin and cellulose from the immediate photosynthesis product glucose, alginate from uronic acids, and fucoidan from fucose. Fucoidan can be decorated with additional monosaccharides, as well as acetyl and sulfate groups giving fucoidan a net negative charge (20-22). While laminarin stores energy within cells (21), cellulose and alginate fulfill structural functions as cell wall constituents (20). Fucoidan builds the extracellular matrix or mucilage in brown algae (23, 24), increases in response to salinity (24, 25), and acts as an antimicrobial agent (26).

Fucoidan is delivered to the extracellular matrix by vesicle cells that secrete fucoidan into mucilage ducts (25, 27, 28). Thereby, brown algae maintain a mucilage barrier primarily consisting of fucoidan (20, 22, 27). Secretion of carbohydrates is a feature of epithelia and can mediate substantial carbon flux (29, 30). The high solubility of fucoidan and its location at the algae-seawater interface suggest that fucoidan has to be constantly resupplied.

Fucoidan shows considerable recalcitrance and has been found to persist for centuries in sediments. Dissolved molecules can sequester carbon if they are not degraded due to recalcitrance or if they are exported to below 1,000 m depth (31, 32), for example after aggregation (33-35). Dissolved energy storage polysaccharides like laminarin are preferentially targeted by bacteria of different classes (36-38) that need only three enzymes for complete degradation within hours (37, 39-41). In contrast, fucoidan can only be degraded by specialized bacteria that require dozens of different enzymes (35, 42). Fucoidan-targeting antibodies revealed fucose-containing sulfated polysaccharides from diatoms assemble and form particles (34, 35) that transport carbon to depth. Consistent antibody signals along sediment cores indicate that fucoidan persists during transport through the water column and in sediments for hundreds of years (43, 44). Thus, mucus secretion by brown algae could mediate carbon sequestration through the recalcitrance and assembly of fucoidan.

We hypothesized that brown algae secrete fucoidan into seawater based on reports of high carbohydrate content in dissolved organic carbon from brown algae (10, 12). Without methods for dissolved polysaccharide quantification, fucoidan concentrations in seawater remain unknown (35). We analyzed seawater from seagrass and brown algae incubations with approaches dedicated to specific and quantitative carbohydrate detection. Monosaccharide quantification, antibody binding, anion exchange chromatography and enzymatic hydrolysis conclusively point toward the accumulation of dissolved fucoidan during algae incubations. Fucoidan carbon contributed substantially to the algae-released organic carbon. Our results suggest secreted fucoidan to sequester carbon at the same rate as carbon is stored in biomass.

### Accumulating dissolved organic carbon from brown algae

We incubated subtidal bladderwrack, *Fucus vesiculosus*, and co-occurring eelgrass, *Zostera marina,* during August 2020 in the coastal Baltic Sea over five hours in transparent or dark bags (**Fig. 3a**) and analyzed seawater from incubations. *Z. marina* served as a control species that secretes organic carbon, but not fucoidan. We found differing impacts on seawater chemistry (**Fig. 3b, c****, Table 1, 2).** In response to light and dark treatments (**Fig. 5**), oxygen concentrations in algae incubations surpassed solubility in light and decreased to ~50 µmol L⁻¹ in dark (**Fig. 3c****, first panel**). Dissolved organic carbon (mean ± SE) increased from 4.71 ± 0.23 mg C L⁻¹ to 6.32 ± 0.12 mg C L⁻¹ in light incubations of algae and to 6.08 ± 0.16 mg C L⁻¹ in the dark (**Fig. 3c****, second panel**). Concurrently, total dissolved nitrogen concentrations, humic-like fluorescence (FDOM) and absorption at 254 nm (CDOM) of dissolved compounds increased (**Fig. 3c****, third to fifth panel**). Dissolved oxygen, FDOM and CDOM in light incubations exceeded dark incubations. The statistical results are summarized in **Table 1**. In contrast to *F*. *vesiculosus,* dissolved oxygen concentrations decreased in both *Z. marina* incubations. Dissolved organic carbon concentrations increased slightly in light, while dissolved nitrogen increased significantly in the light incubations and compared to dark incubations (**Table 2**). Overall, the incubations of *F. vesiculosus* reproduced previously observed effects of brown algae on bulk seawater organic chemistry (10-12, 15, 45, 46)

Metabolomics on seawater samples from incubations showed that small molecules increased in concentration but did not drive the increase in dissolved organic carbon observed in algae incubations (**Fig. 3e****, Table 3, 4**). Signals of derivatized metabolites on a gas chromatography coupled mass spectrometer were minimal compared to signals from a standard chemical mixture at 0.4 mM (**Table 5**). Mannitol was the only metabolite out of four significantly accumulating compounds that could be identified by matching to the standard chemical mixture (**Fig. 3e****, first panel**). It showed the highest accumulation in algal incubations, with average signal increases of ~1190-fold and ~70-fold after the dark and light incubations, respectively. Previous studies have described mannitol accumulation up to 400 mg L⁻¹ during brown algae fragmentation (17) and mannitol release in response to osmotic shock of up to 6 g kg⁻¹ dry weight within two minutes (47). Mannitol from brown algae is completely consumed in seawater within days (17). Metabolomics also detected significant signal increases of an alcohol-like, an organic acid-like and an aromatic-like metabolite from -1.5-fold to ~10-fold after incubation of algae (**Fig. 3e****, second to fifth panel**). In seagrass incubations, a benzoic acid-like metabolite accumulated that could not be found in algae incubations (**Fig. 3d****, fifth panel**). In terms of carbon mass, mannitol and the three unidentified metabolites contributed minor quantities to the increase of dissolved organic carbon during algae incubations.

### Monosaccharide composition, antibody binding and enzymatic digestion identify dissolved fucoidan

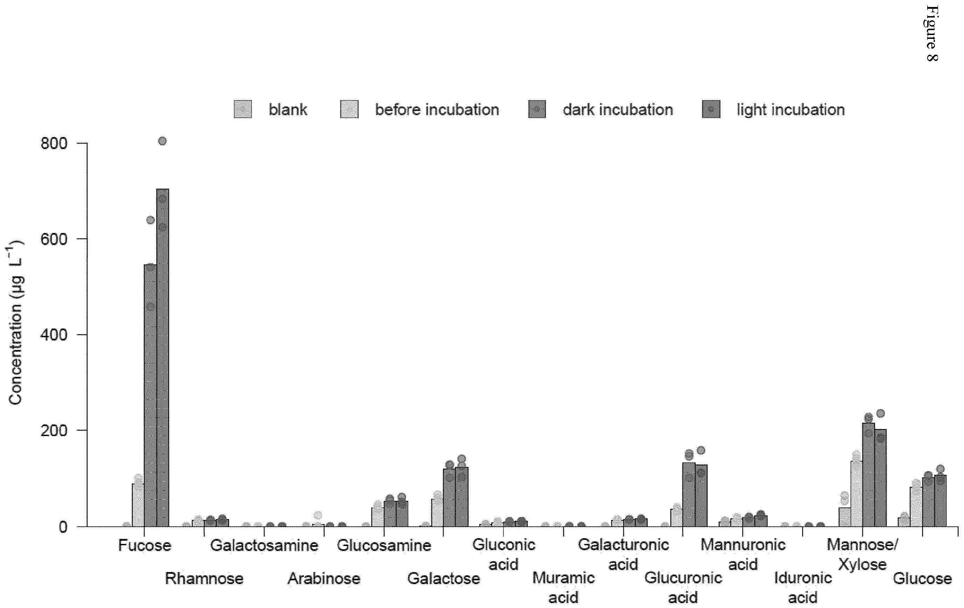

We identified dissolved fucoidan in ambient and incubation seawater via two complementary approaches, monosaccharide composition and antibody-based techniques. After acid hydrolysis, the monosaccharides fucose, glucuronic acid, mannose and xylose, and galactose separated sample groups of blank samples, ambient seawater, and light and dark incubations of *F. vesiculosus* in a principal component analysis (**Fig. 1a**). These monosaccharides compose *F*. *vesiculosus* fucoidan (42). Additionally, the concentrations of fucose, glucuronic acid, mannose and xylose, and galactose correlated with the summed signal intensity of fucoidan-specific antibodies BAM1 and BAM2 (24)(analyzed by ELISA) with adjusted R² ~0.9 or higher (**Fig. 1b**). Mannuronic acid in the range of 5 to 20 µg L⁻¹ indicated the presence of dissolved alginate, however ~30-fold lower concentrated than fucoidan (**Fig. 8**). Monosaccharide composition and antibody binding supported a major contribution of fucoidan to carbohydrates in seawater from algae incubations.

A fucose-dominated fraction from filtered seawater eluted after application of 5 M NaCl to anion exchange chromatography (AEX) columns (**Fig. 1c**). Prior washing with 2 M bicarbonate eluted a fraction with a lower relative abundance of fucose, and monosaccharides that are not present in *F. vesiculosus* fucoidan. (**Fig. 1c****, striped bars**). Binding to the AEX column showed that 70-80% of fucose, galactose and glucuronic acid were contained in anionic polymers (**Fig. 1d**). As in seawater samples, the signal from fucoidan-specific antibodies BAM1 and BAM2 (analyzed by carbohydrate microarrays) correlated with fucose concentration in AEX elutions from brown algae incubations and ambient seawater (**Fig. 7**). For comparison, samples from Z. *marina* incubations showed no binding of BAM1 and BAM2 fucoidan-specific antibodies, but in some replicates binding of BAM7 antibody that recognizes alginate, and has reported cross-reactivity with pectin and to a lesser extent with fucoidan (48) (**Fig. 7**). BAM7 antibody signal on *Z. marina* correlated linearly with galactose (*p* <0.001, R² = 0.98), fucose (*p* <0.001, R² = 0.91) and xylose (*p* <0.001, R² = 0.66). This correlation may indicate the presence of anionic polysaccharides of seagrass origin, including sulfated galactans, which also contain xylose (49), or arabinogalactan-proteins (50). AEX elutions obtained with 5 M NaCl contained strongly anionic fucoidan, while the washing step eluted some fucose-containing and other anionic carbohydrates.

Detection of fucoidan monosaccharides as products of enzymatic activities confirmed the presence of *F. vesiculosus* fucoidan in seawater. Digests were performed with enzymes extracted from the fucoidan-metabolizing *Lentimonas* sp. CC4 grown on *F. vesiculosus* fucoidan. Enzymatic digestion of *F. vesiculosus* fucoidan from standards and environmental samples yielded quantitative amounts of fucose and galactose (**Fig. 1e****, f**). In a Bray-Curtis dissimilarity analysis, enzymatic hydrolysis of filtered seawater and acid hydrolysis of AEX elutions showed higher specificity than acid hydrolysis of filtered seawater (**Fig. 6**). We used this enzymatic approach for fucoidan-specific hydrolysis to produce the most specific quantitative assessment of fucoidan. Enzymatic hydrolysis of filtered and dialyzed seawater confirmed the accumulation of *F. vesiculosus* fucoidan during incubations.

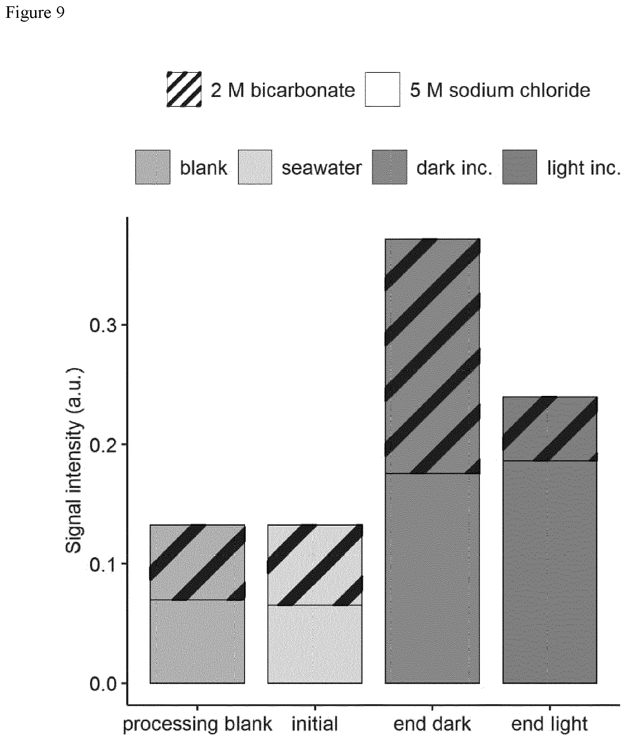

**Table 8: Combined polysaccharide-specific quantification confirms fucoidan accumulation during brown algae incubations. Relative signal intensities (mean ± SE) of two fucoidan-binding monoclonal antibodies (BAM1 and BAM2) from ELISA on filtered seawater samples on algae incubations. Calculated concentrations (µg L⁻¹, mean ± SE) of dissolved fucoidan in algae incubation samples based on acid hydrolysis, AEX followed by acid hydrolysis and enzymatic hydrolysis. Fucose from hydrolyzed fucoidan standard was the best quantitative predictor (Fig. 9), and used to calculate sample fucoidan concentrations. Superscripted letters indicate significant differences between groups (blank, initial, dark inc. and light inc.) within each method (Table 6 and 7). How specific and quantitative approaches are is indicated from low (o) to high (++) by symbols. Abbreviation: incubation (inc.).**

| **Method** | **Blank** | **Initial** | **Dark inc.** | **Light inc.** | **Specific** | **Quantita-tive** |
|---|---|---|---|---|---|---|
| **Antibody BAM1** | 0.07 ± 0.00^{a} | 0.23 ± 0.01^{b} | 0.38 ± 0.01^{c} | 0.41 ± 0.03^{c} | ++ | o |
| **Antibody BAM2** | 0.07 ± 0.00^{a} | 0.08 ± 0.00^{a} | 0.28 ± 0.01^{b} | 0.27 ± 0.01^{b} | ++ | o |
| **Acid hydrolysis** | 0.5 ± 0.3^{a} | 476 ± 15.5^{b} | 2913 ± 279^{c} | 3755 ± 282^{c} | o | ++ |
| **Acid hydrolysis of 5 M NaCl elution** | 42.6 ± 8.0^{a} | 61.9 ± 9.35^{a} | 614 t 170^{b} | 1333 ± 94^{c} | + | + |
| **Enzymatic hydrolysis** | 136 ± 8.9^{a} | 154 ± 19.5^{a} | 1135 ± 145^{b} | 1832 ± 182^{b} | ++ | + |

### Specific quantification reveals the accumulation of dissolved fucoidan in brown algae incubations

The seawater collected from the *F. vesiculosus* incubations after five hours was enriched in dissolved fucoidan (**Table 8, Table 6**). Fucan-specific monoclonal antibody binding qualitatively verified the presence of fucoidan in all seawater samples, and showed accumulation during algae incubations compared to control samples. Quantitative estimates of fucoidan in seawater from algae incubations range between 1333 and 3755 µg L⁻¹ in light and between 614 and 2913 µg L⁻¹ in dark. These concentrations exceed fucoidan levels prior to incubations (62-476 µg L⁻¹) in ambient seawater. Fucoidan accumulated more under light conditions compared to dark conditions, a trend that was consistent across methods.

The employed approaches differed in how specific and how quantitative polysaccharide detection is achieved **(Table 8**). Acid hydrolysis reported the highest values, followed by enzymatic hydrolysis and finally acid hydrolysis of 5 M NaCl elutions. Using acid hydrolysis of filtered seawater to estimate dissolved fucoidan yielded the lowest levels of contamination and fucoidan concentrations in ambient seawater distinct from blanks. Acid hydrolysis of 5 M NaCl elutions produced fucoidan estimates lower than estimates from direct acid hydrolysis of filtered, dialyzed seawater due to losses during AEX. Yet, selectivity and the concentration factor associated with this method revealed a significant influence of treatment **(Table 7**; light vs. dark, *p* = 0.035) on fucoidan estimates. Enzymatic hydrolysis of filtered, dialyzed seawater produced estimates in-between acid hydrolysis and AEX values. Specificity and quantitative signal of enzymatic hydrolysis suggest this to be a potential stand-alone method for fucoidan quantification. The three quantitative approaches converge on a clear pattern of fucoidan content in samples, with comprehendible differences in absolute values.

### Fucoidan secretion drives dissolved carbon flux

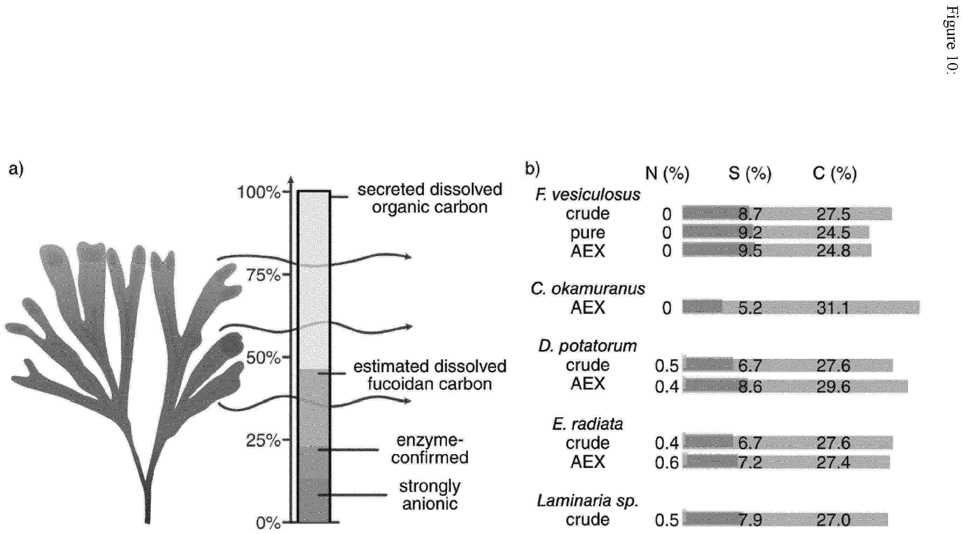

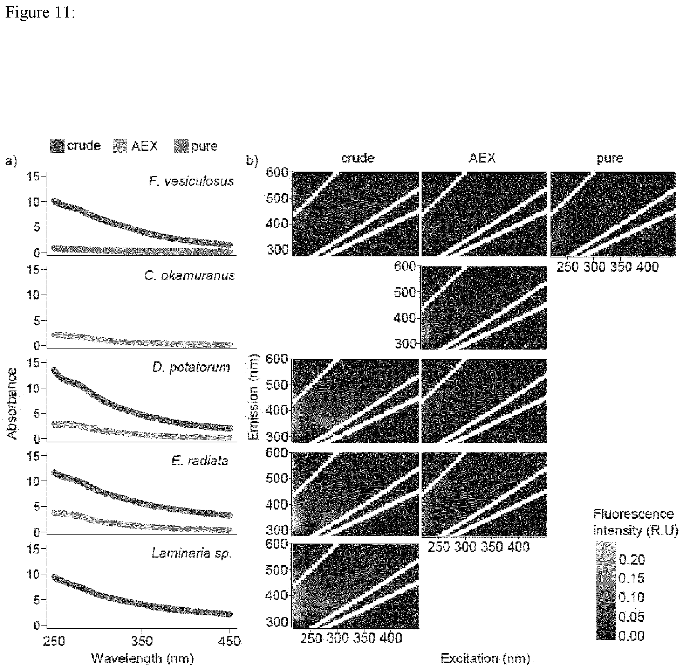

Fucoidan contributed substantially to the accumulation of dissolved organic carbon, but not to the accumulation of dissolved nitrogen (**Fig. 10a**). Enzyme-confirmed fucoidan quantification accounted for 25.4 ± 3.2% of organic carbon exudation in light incubations and 18.0 ± 4.5% in dark incubations. Based on acid hydrolysis, fucoidan secretion accounted for 49.5 ± 1.8% of the organic carbon exudation in light, and 43.8 ± 4.4% in dark. Carbon content of fucoidan was based on elemental analysis of pure *F. vesiculosus* fucoidan. Brown algal fucoidans consisted of 25-30% carbon, 5-10% sulfur and 0-0.6% nitrogen (**Fig. 10b**). Absorbance of fucoidans showed a distinct difference between crude and pure or AEX-purified standards, with crude standards showing a higher absorbance of light between 250 and 450 nm in all cases (**Fig. 11**). Molecules associated with fucoidan such as phlorotannins likely contributed to the increased CDOM and FDOM signals, but may fall short of explaining nitrogen accumulation (15). Fucoidan explained 18-25% or 44-50% of the dissolved organic carbon that accumulated during algae incubations, based on enzymatic hydrolysis and acid hydrolysis respectively.

Secretion rates could be calculated by direct assessment of dissolved fucoidan. Secretion rates of fucoidan carbon per kg dry biomass under light conditions amounted to 19.9 ± 5.08 mg C kg⁻¹ h⁻¹ based on enzyme hydrolysis and 39.2 ± 10.37 mg C kg⁻¹ h⁻¹ based on acid hydrolysis. Under dark conditions, algae secreted 11.5 ± 3.46 and 28.3 ± 7.10 mg C kg⁻¹ h⁻¹ based on enzyme and acid hydrolysis respectively. Dry algal thalli biomass, excluding epibiota, varied between 16.5 and 29.7 g, while bag volumes ranged between 4.8 and 6.8 L. Rates of organic carbon exudation of 45.6-106.8 mg C kg⁻¹ h⁻¹ as determined here are lower than previously reported rates for *F. vesiculosus* of 127.2 and 298.8 mg C kg⁻¹ h⁻¹, but within the range of values reported for brown algae (summarized in (51)). There was no significant correlation between fucoidan accumulation and algal biomass or algal surface area. For a day with 15-hour daylight we estimated a rate of fucoidan secreted per dry biomass at 360 mg C kg⁻¹ d⁻¹ based on enzymatic hydrolysis and at 789 mg C kg⁻¹ d⁻¹ based on acid hydrolysis. This compares to a mean biomass accumulation of 240 mg C kg⁻¹ d⁻¹ for a four-year-old algal stock (52, 53). Carbon removal by fucoidan secretion is on the same order of magnitude as carbon accumulation in biomass.

### Discussion

Brown algae secrete a substantial fraction of fixed carbon as organic material, of which we found up to 50% to be in the form of fucoidan. Algae incubations led to an accumulation of dissolved organic carbon from 4.7 mg L⁻¹ to 6.2 mg L⁻¹. We performed acid hydrolysis, anion exchange chromatography, enzyme-linked immunosorbent assay and a biocatalytic enzyme-based assay on seawater samples. The combination of methods enabled identification and quantification of complex fucoidan, accounting for up to half of the accumulated organic matter. Despite different analytical windows, the techniques converged on a consistent pattern comparing treatments, with higher accumulation in light **(Table 8**). The findings reported relieve the major analytical constraint that has until now prohibited quantification of rates of fucoidan release by brown algae. The substantial secretion rates suggest that fucoidan production and release contribute to carbon sequestration by brown algae.

A combination of techniques is required to quantify complex polysaccharides such as fucoidan. We examined three approaches for fucoidan quantification. 1) Acid hydrolysis combined with antibody identification showed fucoidan release by *F. vesiculosus.* Acid indiscriminately hydrolyzed glycosidic and ester bonds, yielding ~90% of quantifiable monosaccharide constituents (37). Monoclonal antibodies provided specificity with a semi-quantitative assessment (24, 35, 43). Backed by identification on a structural level by antibodies, acid hydrolysis provides an upper boundary of fucoidan concentrations. 2) AEX narrowed the analytical window by selectively extracting anionic polysaccharides from seawater. As binding strength increases with fucoidan length, fucoidan molecules of very high molecular weight may bind irreversibly to the AEX resin. AEX underestimated fucoidan as low molecular weight compounds were washed away (**Fig. 1c**) and elution was potentially incomplete. 3) Enzymatic hydrolysis specifically yielded monosaccharides from *F. vesiculosus* fucoidan, providing confidence in the molecular identity. The soluble cell fraction of *Lentimonas* sp. CC4 grown on *F. vesiculosus* fucoidan showed activity on cellulose, alginate and *F. vesiculosus* fucoidan, and lower to no activity on galactomannan, pectin and *Laminaria hyperborea* fucoidanin line with previous results (42). Species-specific fucoidan quantification is thus theoretically possible with enzymatic tools. Incomplete hydrolysis of fucoidan and possible conversion of fucose by downstream enzymes likely decrease the yield of this method. Lower molecular weight of the fucoidan standard compared to samples may have led to an additional underestimation of fucoidan concentrations. Technical noise likely caused by fucoidan substrate remaining in the soluble cell fraction impaired quantification below 0.1 mg L⁻¹ fucoidan. It is possible to concentrate fucoidan from seawater by AEX and detect fucoidan in extracts by enzymatic hydrolysis (**Fig. 9**). The combination of carbohydrate-targeting techniques affirmed the identification and quantification of dissolved *F. vesiculosus* fucoidan, paving the way for fucoidan tracing in marine environments.

The molecular composition of brown algae exudates reported elsewhere suggests fucoidan secretion to be a common feature. Brown algae release fucoidan into mucilage ducts, which deliver the polysaccharide to the thallus surface (23, 24, 28) from where it dissolves (24, 35, 54). In comparison to alginate, fucoidan undergoes less bridging with divalent cations and exhibits lower viscosity (20, 22, 54). Dedicated vesicle cells for fucoidan production and secretion support the notion of fucoidan loss to the surrounding seawater (23, 27, 28). Green and red algae lack this dedicated machinery of fucoidan production (22). In direct comparison, brown, green and red algae show similar exudation rates, but widely different molecular compositions (55). Compared to green and red algae, brown algae-exuded organics contain four times as much carbohydrate carbon, of which 40% is in fucose (55). With vesicle cells and mucilage constituting a widespread feature of brown algae and reports of carbohydrate-rich, fucose-dominated exudates, fucoidan secretion appears to occur across this cosmopolitan taxon. Fucoidan secretion in combination with constrained microbial degradation indicates sequestration of brown algal exudates in the ocean. In the laboratory with replete nutrients and no competition or pathogens, adapted bacteria degrade about 40% of fucoidan within days (56). In nature under suboptimal conditions, fucoidan degradation takes more time (57). About 10-30% of organic matter moved and stored in the ocean is glycan and within this glycan mixture there is about 10% fucose, the major building block of fucoidan (57). Fucose-containing sulfated polysaccharides are dissolved, present in particles (34, 58) and exported showing it is stable enough to reach the deep sea and underlying sediment (43, 44). How much carbon fucoidan removes becomes measurable only with these or other techniques that provide sufficient molecular resolution. Several studies established resistance of carbohydrate-rich brown algae exudates to microbial degradation, either due to intrinsic recalcitrance or inhibition of microbes. Bacteria grown on exudates of *Turbinaria ornata* exhibit lower growth and growth efficiency than bacteria growing on exudates of green or red algae from the same habitat (55). In degradation studies, between 30 and 85% of organic carbon exuded by *Ecklonia cava* remained after 30 days (16), whilst almost 40% of *Saccharina japonica* exudates and 78% of *Sargassum horneri* exudates persisted for 150 days (3, 45).

This study focused on fucoidan secretion of *F*. *vesiculosus.* The dissolved organic carbon exudation rates and abundant carbohydrates reported here, particularly fucose, are consistent with previous studies (10, 12, 55). This study measured fucoidan secretion for an interval during one day in August when exudation rates are lower compared to other months (10, 12). The incubated algae potentially experienced stress due to experimental manipulation, but stress also occurs in nature and neither stress molecules nor indicators of decay were detected. Our sampling location in the Baltic Sea differs from most marine environments with a lower salinity of approximately 6 g/kg, which may have affected the degree of fucoidan sulfation (22) and secretion rates (14). However, we could also show, that purification and quantification work with samples having a higher salinity (see **Example 2**). Fucoidan secretion as a protection may change with tidal changes, desiccation and other stressors. The examined techniques allow quantification of fucoidan secretion rates across the cosmopolitan taxon of brown algae under different environmental conditions.

Fucoidan secretion by *F. vesiculosus* expands conventional carbon dioxide removal valuation, which considers algal biomass carbon, to include secreted molecules. While organic carbon secretion by brown algae has been known for decades (17, 28, 59), the lack of analytics for marine samples has constrained molecular quantification (35, 37, 39, 60). Natural release of dissolved fucoidan by brown algae eases the necessity to dump biomass in current approaches. Farmed algae biomass could supply applications in agriculture, pharmaceutics and materials. Harvesting of farmed algal biomass benefits eutrophied systems, but would reduce primary productivity in oligotrophic waters including most of the open ocean due to nutrient depletion (61). In turn, brown algae likely continue to secrete fucoidan despite low nutrient concentrations as long as light and carbon dioxide are available. Secreted fucoidan contributes to carbon sequestration by brown algae and does not induce significant nutrient removal (**Fig. 10b**) (52).

### Conclusion

We adapted and compared techniques to quantify dissolved fucoidan, a complex polysaccharide that is difficult to degrade for microbes and accumulates in the ocean. Fucoidan quantification was enabled by using acid hydrolysis, anion exchange chromatography and/or enzymatic digestion, which yielded in converging results from samples, and the presence of fucoidan was confirmed by monoclonal antibody binding. Fucoidan comprised 18-50% of dissolved organic carbon exuded by the brown algae *Fucus vesiculosus.* Dissolved fucoidan likely stems from dissipation of mucilage. Fucoidan does not contain relevant amounts of nitrogen nor other growth-limiting elements, enabling nutrient-independent release of fucoidan carbon. As brown algae secrete 14-35% of their net primary production, they inject substantial amounts of fucoidan carbon into the 660 Gt dissolved organic carbon pool in the ocean. Fucoidan can accumulate in particles and could, through the biological carbon pump, reach the deep ocean where carbon is sequestered. We conclude that dissolved fucoidan secretion is likely an overlooked contribution of brown algae to carbon dioxide removal.

### Example 2: Quantification of fucoidan standard/reference sample in artificial seawater

Fucoidan was extracted from *Lessonia trabeculata* and *Ecklonia maxima* yielding colorless lumps of material after final purification steps and freeze drying. For both, *Z. trabeculata* and *E. maxima* fucoidan, a stock solution of 5 mg mL⁻¹ was prepared. Artificial seawater was prepared from seasalts (Sigma) and MilliQ water (Ω>18.2 µS) at a concentration of 38 g L⁻¹ in prerinsed 1 L wide-mouth polypropylene bottles (Nalgene). The artificial seawater was used to prepare 12 extraction samples of 1 L volume with either *Z. trabeculata* or *E. maxima* fucoidan at concentrations of 0.01, 0.02, 0.05, 0.1, 0.5, and 1 mg L⁻¹. Of each extraction sample, 10 mL were taken for direct analysis (see below). The remainder was split into two technical replicates of approx. 450 mL each, of which 400 mL were used for fucoidan purification in the following way:
Extraction columns consisted of High Trap fast flow ANX anion exchange columns with 5 mL bed volume (Cytiva, USA). Solutions were degassed before use. Final concentrations in buffer A were 20 mM Tris-Cl and 0.5 MNaCl with pH set to 8. Final concentrations in buffer B were 20 mM Tris-Cl and 4 M NaCl with pH set to 8. Flow rate was set to 5 mL min⁻¹ unless stated otherwise. The column was prepared by washing for 5 min with buffer A, followed by 5 min with buffer B, and another 5 min with buffer A for conditioning. The sample of 400 mL was passed over the column during 80 min. After sample application, any unbound residuals were rinsed off the column with buffer A applied for 6 min. Buffer B was passed over the column for 30 s to replace buffer A. After the 30 s corresponding to a little less than the column volume, 12.5 mL of eluate were collected in a clean 15 mL polypropylene tube as buffer B was applied to the column for 2.5 min to unbind fucoidan. The eluted fraction was stored at 4°C or at -20°C until further processing (see below). Residual compounds on the column were removed by washing the column for 5 min with buffer B followed by 5 min of MilliQ-water. For 20 min, 1 M NaOH was applied to the column at reduced velocity of 1 mL min⁻¹. NaOH was removed afterwards by applying MilliQ-water again for 5 min at 5 mL min⁻¹. For column storage, 20% ethanol was applied to the column for 5 min. The flow was stopped and the column inlet and outlet closed to prevent drying. The column containing 20% ethanol was stored at 4°C until further use.

### Quantification

Directly collected samples of 10 mL and 10 mL of eluates were dialyzed against MilliQ-water in 1 kDa membrane (SpectraPor^{®} Biotech CE Dialysis Membrane, Carl Roth, Germany) with MilliQ-water controls in separate dialysis tubing to account for processing artifacts. Salt and small molecules were removed by dialyzing three times against 10 L MilliQ-water at 4°C. Dialyzed samples were transferred to 15 mL polypropylene tubes, freeze dried and resuspended in 2 mL of MilliQ-water.

Of the dialyzed and freeze dried samples and eluates, 500 µL were combined with 500 µL 2 M HCl in a precombusted glass ampule. Ampules were sealed and incubated for 24 h at 100°C to achieve complete acid hydrolysis of polysaccharides in the sample. After acid hydrolysis, 900 µL were transferred from glass ampules to 1.5 mL microtubes and dried in an acid proof vacuum concentrator (Martin Christ Gefriertrocknungsanlagen GmbH, Germany). Dried, hydrolyzed samples were reconstituted in 900 µL MilliQ-water, diluted if necessary and 100 µL transferred to 200 µL glass inlets in HPLC vials for High Performance Anion Exchange Chromatoraphy with online Pulsed Amperometric Detector (HPAEC-PAD) quantification of monosaccharides.

Monosaccharides in samples were separated via anionic interaction chromatography and individually quantified using electrochemical detection. For analysis, 25 µL of sample were autosampler-injected into a DIONEX ICS 5000+ equipped with a 2 × 250 mm PA10 column with corresponding guard (all Thermo Fisher Scientific, USA). Separation of neutral monosaccharides was achieved during an isocratic phase with 18 mM NaOH, followed by separation of acidic monosaccharides during a gradient up to 200 mM NaCH₃COO. Monosaccharides were detected using pulsed amperometric detection. Based on quantitative standards, monosaccharides were identified by retention times and peak areas fitted to the standard series, which consisted of arabinose, fucose, galactosamine, galactose, glucose, mannose, rhamnose, and xylose.

### Results

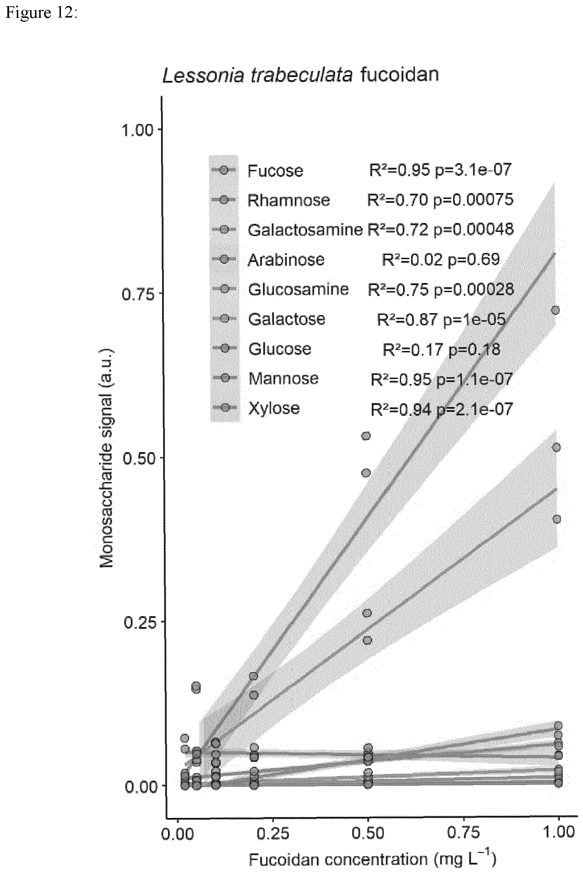

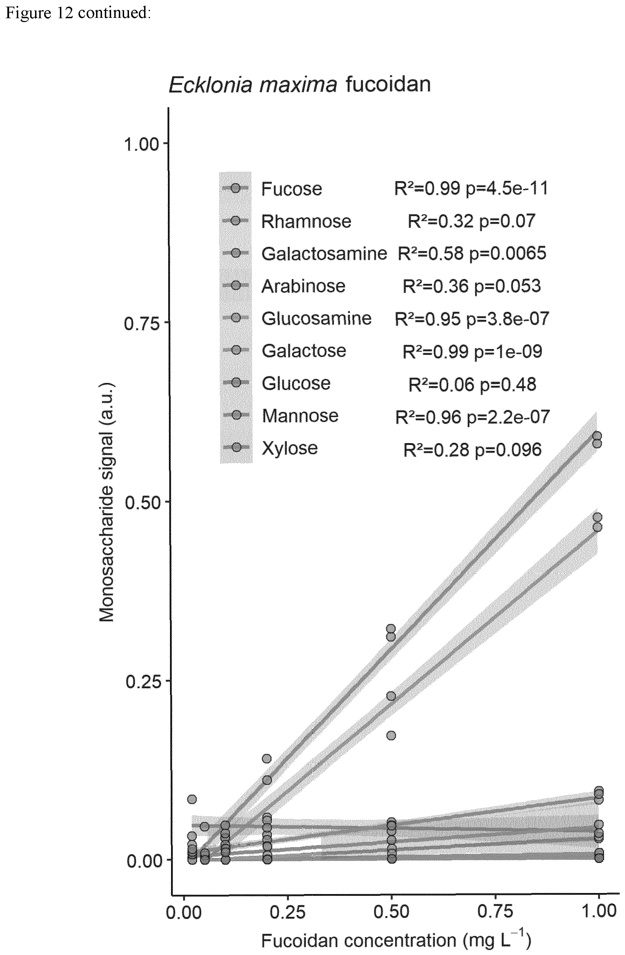

Fucoidan was successfully quantified in seawater using anion exchange chromatography. The signals of fucoidan-constituting monosaccharides correlated with fucoidan concentrations in extracted seawater (Fig. 12). For *Z. trabeculata* fucoidan, the correlation was >0.90 for fucose, mannose and xylose. For *E. maxima* fucoidan, fucose, glucosamine, galactose and mannose correlated >0.95 with fucoidan concentrations. In comparison to analyzed seawater, extraction enhanced signal intensities, highlighting the advantage of the purification method of the present invention. In addition, polysaccharides other than fucoidan would appear in monosaccharide profiles of seawater analyses but are removed during the fucoidan extraction procedure. Thus, the purification method of the present invention results in improved fucoidan quantification.

### List of references:

1 Green, E. R. & Mecsas, J. Bacterial Secretion Systems: An Overview. Microbiol Spectr 4, doi:10.1128/microbiolspec. VMBF-0012-2015 (2016).
2 Macion, A., Wyszynska, A. & Godlewska, R. Delivery of Toxins and Effectors by Bacterial Membrane Vesicles. Toxins (Basel) 13, doi:10.3390/toxins13120845 (2021).
3 Bumba, L. et al. Calcium-Driven Folding of RTX Domain beta-Rolls Ratchets Translocation of RTX Proteins through Type I Secretion Ducts. Mol Cell 62, 47-62, doi:10.1016/j.molcel.2016.03.018 (2016).
4 Roderer, D. & Raunser, S. Tc Toxin Complexes: Assembly, Membrane Permeation, and Protein Translocation. Annu Rev Microbiol 73, 247-265, doi:10.1146/annurev-micro-102215-095531 (2019).
5 McQuade, R. & Stock, S. P. Secretion Systems and Secreted Proteins in Gram-Negative Entomopathogenic Bacteria: Their Roles in Insect Virulence and Beyond. Insects 9, doi:10.3390/insects9020068 (2018).
6 Blackburn, M., Golubeva, E., Bowen, D. & Ffrench-Constant, R. H. A novel insecticidal toxin from photorhabdus luminescens, toxin complex a (Tca), and its histopathological effects on the midgut of manduca sexta. Appl Environ Microbiol 64, 3036-3041, doi:10.1128/AEM.64.8.3036-3041.1998 (1998).
7 Song, N. et al. Genome-wide dissection reveals diverse pathogenic roles of bacterial Tc toxins. PLoSPathog 17, e1009102, doi:10.1371/journal.ppat.1009102 (2021).
8 Roderer, D., Hofnagel, O., Benz, R. & Raunser, S. Structure of a Tc holotoxin pore provides insights into the translocation mechanism. Proc Natl Acad Sci U S A 116, 23083-23090, doi:10.1073/pnas. 1909821116 (2019).
9 Leidreiter, F. et al. Common architecture of Tc toxins from human and insect pathogenic bacteria. Sci Adv 5, eaax6497, doi:10.1126/sciadv.aax6497 (2019).
10 Gatsogiannis, C. et al. Tc toxin activation requires unfolding and refolding of a beta-propeller. Nature 563, 209-213, doi:10.1038/s41586-018-0556-6 (2018).
11 Gatsogiannis, C. et al. Membrane insertion of a Tc toxin in near-atomic detail. Nat Struct Mol Biol 23, 884-890, doi:10.1038/nsmb.3281 (2016).
12 Meusch, D. et al. Mechanism of Tc toxin action revealed in molecular detail. Nature 508, 61-65, doi:10.1038/nature13015 (2014).
13 Gatsogiannis, C. et al. A syringe-like injection mechanism in Photorhabdus luminescens toxins. Nature 495, 520-523, doi:10.1038/nature11987 (2013).
14 Piper, S. J. et al. Cryo-EM structures of the pore-forming A subunit from the Yersinia entomophaga ABC toxin. Nat Commun 10, 1952, doi:10.1038/s41467-019-09890-8 (2019).
15 Roderer, D. et al. Glycan-dependent cell adhesion mechanism of Tc toxins. Nat Commun 11, 2694, doi:10.1038/s41467-020-16536-7 (2020).
16 Song, N. et al. N-Glycans and sulfated glycosaminoglycans contribute to the action of diverse Tc toxins on mammalian cells. PLoS Pathog 17, e1009244, doi:10.1371/journal.ppat.1009244 (2021).
17 Ng'ang'a, P. N. et al. Involvement of N-glycans in binding of Photorhabdus luminescens Tc toxin. Cell Microbiol 23, e13326, doi:10.1111/cmi.13326 (2021).
18 Lang, A. E. et al. Photorhabdus luminescens toxins ADP-ribosylate actin and RhoA to force actin clustering. Science 327, 1139-1142, doi:10.1126/science.1184557 (2010).
19 Yang, G. et al. Pdl1 is a putative lipase that enhances Photorhabdus toxin complex secretion. PLoSPathog 8, e1002692, doi:10.1371/journal.ppat.1002692 (2012).
20 Yang, G. & Waterfield, N. R. The role of TcdB and TccC subunits in secretion of the Photorhabdus Tcd toxin complex. PLoS Pathog 9, e1003644, doi:10.1371/journal.ppat.1003644 (2013).
21 Silva, C. P. et al. Bacterial infection of a model insect: Photorhabdus luminescens and Manduca sexta. Cell Microbiol 4, 329-339, doi:10.1046/j.1462-5822.2002.00194.x (2002).
22 Gendlina, I. et al. Identification and type III-dependent secretion of the Yersinia pestis insecticidal-like proteins. Mol Microbiol 64, 1214-1227, doi:10.1111/j.1365-2958.2007.05729.x (2007).
23 Hurst, M. R. et al. The main virulence determinant of Yersinia entomophaga MH96 is a broad-host-range toxin complex active against insects. J Bacteriol 193, 1966-1980, doi:10.1128/JB.01044-10 (2011).
24 Grayson, J. M. Digestive Tract pH of Six Species of Coleoptera. Annals of the Entomological Society of America 51, 403-405, doi:10.1093/aesa/51.4.403 (1958).
25 Terra, W. R. & Ferreira, C. Biochemistry and Molecular Biology of Digestion. Insect Molecular Biology and Biochemistry, 365-418, doi:10.1016/B978-0-12-384747-8.10011-X (2012).
26 Hurst, M. R., van Koten, C. & Jackson, T. A. Pathology of Yersinia entomophaga MH96 towards Costelytra zealandica (Coleoptera; Scarabaeidae) larvae. J Invertebr Pathol 115, 102-107, doi:10.1016/j.jip.2013.11.004 (2014).
27 Palmer, T., Finney, A. J., Saha, C. K., Atkinson, G. C. & Sargent, F. A holin/peptidoglycan hydrolase-dependent protein secretion system. Mol Microbiol 115, 345-355, doi:10.1111/mmi. 14599 (2021).
28 Young, R. Phage lysis: three steps, three choices, one outcome. J Microbiol 52, 243-258, doi:10.1007/s12275-014-4087-z (2014).
29 Hamilton, J. J. et al. A holin and an endopeptidase are essential for chitinolytic protein secretion in Serratia marcescens. J Cell Biol 207, 615-626, doi:10.1083/jcb.201404127 (2014).
30 Owen, R. A. et al. Structure and activity of ChiX: a peptidoglycan hydrolase required for chitinase secretion by Serratia marcescens. Biochem J 475, 415-428, doi:10.1042/BCJ20170633 (2018).
31 Schoof, M., O'Callaghan, M., Sheen, C. R., Glare, T. R. & Hurst, M. R. H. Identification of genes involved in exoprotein release using a high-throughput exoproteome screening assay in Yersinia entomophaga. PLoS One 17, e0263019, doi:10.1371/journal.pone.0263019 (2022).
32 Berry, J., Rajaure, M., Pang, T. & Young, R. The spanin complex is essential for lambda lysis. J Bacteriol 194, 5667-5674, doi:10.1128/JB.01245-12 (2012).
33 Starke, M., Richter, M. & Fuchs, T. M. The insecticidal toxin genes of Yersinia enterocolitica are activated by the thermolabile LTTR-like regulator TcaR2 at low temperatures. Molecular Microbiology 89, 596-611, doi:10.1111/mmi.12296 (2013).
34 Cornelis, G. R. et al. ymoA, a Yersinia enterocolitica chromosomal gene modulating the expression of virulence functions. Mol Microbiol 5, 1023-1034, doi:10.1111/j.1365-2958.1991.tb01875.x (1991).
35 Starke, M. & Fuchs, T. M. YmoA negatively controls the expression of insecticidal genes in Yersinia enterocolitica. Mol Microbiol 92, 287-301, doi:10.1111/mmi.12554 (2014).
36 Bohme, K. et al. The Small Protein YmoA Controls the Csr System and Adjusts Expression of Virulence-Relevant Traits of Yersinia pseudotuberculosis. Front Microbiol 12, 706934, doi:10.3389/fmicb.2021.706934 (2021).
37 Ellison, D. W., Young, B., Nelson, K. & Miller, V. L. YmoA negatively regulates expression of invasin from Yersinia enterocolitica. J Bacteriol 185, 7153-7159, doi:10.1128/JB.185.24.7153-7159.2003 (2003).
38 Jackson, M. W., Silva-Herzog, E. & Plano, G. V. The ATP-dependent ClpXP and Lon proteases regulate expression of the Yersinia pestis type III secretion system via regulated proteolysis of YmoA, a small histone-like protein. Mol Microbiol 54, 1364-1378, doi:10.1111/j.1365-2958.2004.04353.x (2004).
39 Dewey, J. S. et al. Micron-scale holes terminate the phage infection cycle. Proc Natl Acad Sci USA 107, 2219-2223, doi:10.1073/pnas.0914030107 (2010).
40 Grundling, A., Manson, M. D. & Young, R. Holins kill without warning. Proc Natl Acad Sci USA 98, 9348-9352, doi:10.1073/pnas.151247598 (2001).
41 White, R. et al. Holin triggering in real time. Proc Natl Acad Sci USA 108, 798-803, doi:10.1073/pnas. 1011921108 (2011).
42 Kashket, E. R. The proton motive force in bacteria: a critical assessment of methods. Annu Rev Microbiol 39, 219-242, doi:10.1146/annurev.mi.39.100185.001251 (1985).
43 Michels, M. & Bakker, E. P. Generation of a large, protonophore-sensitive proton motive force and pH difference in the acidophilic bacteria Thermoplasma acidophilum and Bacillus acidocaldarius. J Bacteriol 161, 231-237, doi:10.1128/jb.161.1.231-237.1985 (1985).
44 Tacke, S. et al. A streamlined workflow for automated cryo focused ion beam milling. J Struct Biol 213, 107743, doi:10.1016/j.jsb.2021.107743 (2021).
45 Vollmer, W. & Bertsche, U. Murein (peptidoglycan) structure, architecture and biosynthesis in Escherichia coli. Biochim Biophys Acta 1778, 1714-1734, doi:10.1016/j.bbamem.2007.06.007 (2008).
46 Saier, M. H., Jr. Microcompartments and protein machines in prokaryotes. J Mol Microbiol Biotechnol 23, 243-269, doi:10.1159/000351625 (2013).
47 Frederiksen, R. F. et al. Bacterial chitinases and chitin-binding proteins as virulence factors. Microbiology (Reading) 159, 833-847, doi:10.1099/mic.0.051839-0 (2013).
48 Springer, K. et al. Activity of a Holin-Endolysin System in the Insecticidal Pathogenicity Island of Yersinia enterocolitica. J Bacteriol 200, doi:10.1128/JB.00180-18 (2018).
49 Sänger, P.-A., Wagner, S., Liebler-Tenorio, E. & Fuchs, T. M. Dissecting the invasion of Galleria mellonella by Yersinia enterocolitica reveals metabolic adaptations and a role of a phage lysis cassette in insect killing. bioRxiv, 2022.2006.2027.497489, doi:10.1101/2022.06.27.497489 (2022).
50 Bos, K. I. et al. A draft genome of Yersinia pestis from victims of the Black Death. Nature 478, 506-510, doi:10.1038/nature10549 (2011).
51 Haensch, S. et al. Distinct clones of Yersinia pestis caused the black death. PLoS Pathog 6, e1001134, doi:10.1371/journal.ppat. 1001134 (2010).
52 Govind, R. & Dupuy, B. Secretion of Clostridium difficile toxins A and B requires the holin-like protein TcdE. PLoS Pathog 8, e1002727, doi:10.1371/journal.ppat.1002727 (2012).
53 Wydau-Dematteis, S. et al. Cwp19 Is a Novel Lytic Transglycosylase Involved in Stationary-Phase Autolysis Resulting in Toxin Release in Clostridium difficile. mBio 9, doi:10.1128/mBio.00648-18 (2018).
54 Mehner-Breitfeld, D. et al. Evidence for an Adaptation of a Phage-Derived Holin/Endolysin System to Toxin Transport in Clostridioides difficile. Front Microbiol 9, 2446, doi:10.3389/fmicb.2018.02446 (2018).
55 Vidor, C. J. et al. A Highly Specific Holin-Mediated Mechanism Facilitates the Secretion of Lethal Toxin TcsL in Paeniclostridiumsordellii. Toxins (Basel) 14, doi:10.3390/toxins14020124 (2022).
56 Saadat, A. & Melville, S. B. Holin-Dependent Secretion of the Large Clostridial Toxin TpeL by Clostridium perfringens. J Bacteriol 203, doi:10.1128/JB.00580-20 (2021).
57 Geiger, T., Pazos, M., Lara-Tejero, M., Vollmer, W. & Galan, J. E. Peptidoglycan editing by a specific LD-transpeptidase controls the muramidase-dependent secretion of typhoid toxin. Nat Microbiol 3, 1243-1254, doi:10.1038/s41564-018-0248-x (2018).
58 Hurst, M. R. H., Becher, S. A., Young, S. D., Nelson, T. L. & Glare, T. R. Yersinia entomophaga sp. nov., isolated from the New Zealand grass grub Costelytra zealandica. Int J Syst Evol Microbiol 61, 844-849, doi:10.1099/ij s.0.024406-0 (2011).
59 Bae, S., Park, J. & Kim, J. S. Cas-OFFinder: a fast and versatile algorithm that searches for potential off-target sites of Cas9 RNA-guided endonucleases. Bioinformatics 30, 1473-1475, doi:10.1093/bioinformatics/btu048 (2014).
60 Zhao, D. et al. CRISPR/Cas9-assisted gRNA-free one-step genome editing with no sequence limitations and improved targeting efficiency. Sci Rep 7, 16624, doi:10.1038/s41598-017-16998-8 (2017).
61 Cox, J. & Mann, M. MaxQuant enables high peptide identification rates, individualized p.p.b.-range mass accuracies and proteome-wide protein quantification. Nat Biotechnol 26, 1367-1372, doi:10.1038/nbt.1511 (2008).
62 Tyanova, S. et al. The Perseus computational platform for comprehensive analysis of (prote)omics data. Nat Methods 13, 731-740, doi:10.1038/nmeth.3901 (2016).
63 Goedhart, J. & Luijsterburg, M. S. VolcaNoseR is a web app for creating, exploring, labeling and sharing volcano plots. Sci Rep 10, 20560, doi:10.1038/s41598-020-76603-3 (2020).
64 Teufel, F. et al. SignalP 6.0 predicts all five types of signal peptides using protein language models. Nat Biotechnol, doi:10.1038/s41587-021-01156-3 (2022).
65 Boisvert, S., Raymond, F., Godzaridis, E., Laviolette, F. & Corbeil, J. Ray Meta: scalable de novo metagenome assembly and profiling. Genome Biol 13, R122, doi:10.1186/gb-2012-13-12-r122 (2012).
66 Chen, K. T. & Lu, C. L. CSAR-web: a web server of contig scaffolding using algebraic rearrangements. Nucleic Acids Res 46, W55-W59, doi:10.1093/nar/gky337 (2018).
67 Wang, Z. et al. The molecular basis for sarcomere organization in vertebrate skeletal muscle. Cell 184, 2135-2150 e2113, doi:10.1016/j.cell.2021.02.047 (2021).
68 Mastronarde, D. N. Automated electron microscope tomography using robust prediction of specimen movements. J Struct Biol 152, 36-51, doi:10.1016/j.jsb.2005.07.007 (2005).
69 Hagen, W. J. H., Wan, W. & Briggs, J. A. G. Implementation of a cryo-electron tomography tilt-scheme optimized for high resolution subtomogram averaging. J Struct Biol 197, 191-198, doi:10.1016/j.jsb.2016.06.007 (2017).
70 Tegunov, D. & Cramer, P. Real-time cryo-electron microscopy data preprocessing with Warp. Nat Methods 16, 1146-1152, doi:10.1038/s41592-019-0580-y (2019).
71 Kremer, J. R., Mastronarde, D. N. & McIntosh, J. R. Computer visualization of three-dimensional image data using IMOD. J Struct Biol 116, 71-76, doi:10.1006/jsbi.1996.0013 (1996).
72 Tang, G. et al. EMAN2: an extensible image processing suite for electron microscopy. J Struct Biol 157, 38-46, doi:10.1016/j.jsb.2006.05.009 (2007).
73 Buchholz, T. O. et al. Content-aware image restoration for electron microscopy. Methods Cell Biol 152, 277-289, doi:10.1016/bs.mcb.2019.05.001 (2019).
74 Bharat, T. A. & Scheres, S. H. Resolving macromolecular structures from electron cryo-tomography data using subtomogram averaging in RELION. Nat Protoc 11, 2054-2065, doi:10.1038/nprot.2016.124 (2016).
75 Pettersen, E. F. et al. UCSF ChimeraX: Structure visualization for researchers, educators, and developers. Protein Sci 30, 70-82, doi:10.1002/pro.3943 (2021).
76 Cui, J. et al. Design, Synthesis and Evaluation of Triazole-Pyrimidine Analogues as SecA Inhibitors. ChemMedChem 11, 43-56, doi:10.1002/cmdc.201500447 (2016).
77 Kulp, A. J. et al. Genome-Wide Assessment of Outer Membrane Vesicle Production in Escherichia coli. PLoS One 10, e0139200, doi:10.1371/journal.pone.0139200 (2015).
78 Sedlacek JD, Vail PV. Application and Evaluation of Entomopathogens for Managing Lepidoptera in Stored Products. InField Manual of Techniques in Invertebrate Pathology 2000 (pp. 707-720). Springer, Dordrecht.
79 Roh JY, Choi JY, Li MS, Jin BR, Je YH. Bacillus thuringiensis as a specific, safe, and effective tool for insect pest control. Journal of microbiology and biotechnology. 2007;17(4):547-59.
80 Tabashnik BE, Brévault T, Carrière Y. Insect resistance to Bt crops: lessons from the first billion acres. Nature biotechnology. 2013 Jun;31(6):510-21.
81 Bundesanstalt für Arbeitsschutz und Arbeitsmedizin. TRBA 466 "Einstufung von Prokaryonten (Bacteria und Archaea) in Risikogruppen". Ausgabe 2015, GMBI. 2015, Nr. 46-50 vom 25.8.2015, 9. Änderung vom 21.12.2020, GMBl. Nr. 51.
82 Jones SA, Hurst MR. Purification of the Yersinia entomophaga Yen-TC toxin complex using size exclusion chromatography. In: Glare, T., Moran-Diez, M. (eds) Microbial-Based Biopesticides. Methods Mol Biol. 1477, 39-48, doi: 10.1007/978-1-4939-6367-6_4 (2016)
83 C. Gustafsson, A. Norkko, Not all plants are the same: Exploring metabolism and nitrogen fluxes in a benthic community composed of different aquatic plant species. Limnology and Oceanography 61, 1787-1799 (2016).
84 K. M. Attard et al., Seasonal metabolism and carbon export potential of a key coastal habitat: The perennial canopy-forming macroalga Fucus vesiculosus. Limnology and Oceanography 64, 149-164 (2019).
85 I. F. Rodil, K. M. Attard, C. Gustafsson, A. Norkko, Variable contributions of seafloor communities to ecosystem metabolism across a gradient of habitat-forming species. Marine Environmental Research 167, 105321 (2021).
86 S. Wada et al., Quantitative and qualitative analyses of dissolved organic matter released from Ecklonia cava Kjellman, in Oura Bay, Shimoda, Izu Peninsula, Japan. Journal of Experimental Marine Biology and Ecology 349, 344-358 (2007).
87 P. Massicotte (2017) PMassicotte/eemR: eemR 0.1.5 (V0.1.5). (Zenodo).
88 K. R. Murphy et al., Measurement of dissolved organic matter fluorescence in aquatic environments: an interlaboratory comparison. Environmental Science & Technology 44, 9405-9412 (2010).
89 A. Engel, N. Händel, A novel protocol for determining the concentration and composition of sugars in particulate and in high molecular weight dissolved organic matter (HMW-DOM) in seawater. Marine Chemistry 127, 180-191 (2011).
90 S. Vidal-Melgosa et al., Diatom fucan polysaccharide precipitates carbon during algal blooms. Nature Communications 12, 1150 (2021).
91 A. Dinno (2017) conover.test: Conover-Iman test of multiple comparisons using rank sums.
92 C. A. Smith, E. J. Want, G. O'Maille, R. Abagyan, G. Siuzdak, XCMS: processing mass spectrometry data for metabolite profiling using nonlinear peak alignment, matching, and identification. Analytical Chemistry 78, 779-787 (2006).
93 A. Kassambara (2021) rstatix: pipe-friendly framework for basic statistical tests.
94 J. Oksanen et al. (2020) vegan: community ecology package.
95 D. Bates, M. Mächler, B. Bolker, S. Walker, Fitting linear mixed-effects models using lme4. Journal of Statistical Software 67 (2015).
96 Deniaud-Bouet, E., N. Kervarec, G. Michel, T. Tonon, B. Kloareg, and C. Herve. 2014. 'Chemical and enzymatic fractionation of cell walls from Fucales: insights into the structure of the extracellular matrix of brown algae', Ann Bot, 114: 1203-16.
97 Dittmar, Thorsten, Boris Koch, Norbert Hertkorn, and Gerhard Kattner. 2008. 'A simple and efficient method for the solid-phase extraction of dissolved organic matter (SPE-DOM) from seawater', Limnology and Oceanography: Methods, 6: 230-35.
98 Dittmar, Thorsten, Sinikka T Lennartz, Hagen Buck-Wiese, Dennis A Hansell, Chiara Santinelli, Chiara Vanni, Bernd Blasius, and Jan-Hendrik Hehemann. 2021. 'Enigmatic persistence of dissolved organic matter in the ocean', Nature Reviews Earth & Environment, 2: 570-83.
99 Huang, Guoyin, Silvia Vidal-Melgosa, Andreas Sichert, Stefan Becker, Yang Fang, Jutta Niggemann, Morten Hvitfeldt Iversen, Yi Cao, and Jan-Hendrik Hehemann. 2021. 'Secretion of sulfated fucans by diatoms may contribute to marine aggregate formation', Limnology and Oceanography, 66: 3768-82.
100 Sogin, Emilia M., Erik Puskás, Nicole Dubilier, Manuel Liebeke, and Julie A. Huber. 2019. 'Marine Metabolomics: a Method for Nontargeted Measurement of Metabolites in Seawater by Gas Chromatography-Mass Spectrometry', mSystems, 4: e00638-19.
101 Vidal-Melgosa, S., A. Sichert, T. B. Francis, D. Bartosik, J. Niggemann, A. Wichels, W. G. T. Willats, B. M. Fuchs, H. Teeling, D. Becher, T. Schweder, R. Amann, and J. H. Hehemann. 2021. 'Diatom fucan polysaccharide precipitates carbon during algal blooms', Nat Commun, 12: 1150.
102 Xu, Gege, et al. "Revisiting monosaccharide analysis-quantitation of a comprehensive set of monosaccharides using dynamic multiple reaction monitoring." Analyst 143.1 (2018): 200-207.
103 Dean, Gillian H., et al. "Analysis of monosaccharides from Arabidopsis seed mucilage and whole seeds using HPAEC-PAD." Bio-protocol 9.24 (2019): e3464-e3464.
104 Deniaud-Bouët, Estelle, et al. "Chemical and enzymatic fractionation of cell walls from Fucales: insights into the structure of the extracellular matrix of brown algae." Annals of Botany 114.6 (2014): 1203-1216.

## Claims

1. A method for purifying fucoidan, comprising:
(i) obtaining a sample of seawater, optionally comprising a prefiltration step, and
(ii) purifying fucoidan from the sample.

2. The method of claim 1, comprising purifying fucoidan via an anion exchange chromatography column.

3. The method of claim 2, wherein the column is conditioned with a buffer A and/or a buffer B, optionally, wherein buffer A comprises a same or similar salinity/salt concentration as the sample of seawater,
optionally, comprising conditioning the column with about 1-5 column volumes of buffer A, optionally followed by about 1-5 column volumes of buffer B, optionally followed by about 1-5 column volumes of buffer A.

4. The method of claim 3, wherein buffer A comprises about 20 mM Tris-HCl, about 0.4-0.7 M NaCl at about pH 8, and wherein buffer B comprises about 20 mM Tris-HCl, about 4-5 M NaCl at about pH 8.

5. The method of any one of claims 2-4, further comprising applying the sample of seawater onto the conditioned column.

6. The method of any one of claims 1-5, further comprising a washing step which comprises applying a volume of wash buffer to the column,
optionally, wherein the salinity/salt concentration of the wash buffer resembles the salinity/salt concentration of the sample of seawater,
optionally, wherein the wash buffer comprises about 20 mM Tris-HCl, about 0.4-0.7 M NaCl at about pH 8.

7. The method of any one of claims 1-6, further comprising an elution step which comprises applying a volume of elution buffer to the column,
optionally, wherein the elution buffer comprises about 20 mM Tris-HCl, about 4-5 M NaCl at about pH 8.

8. The method of claim 1, comprising a precipitation step, wherein fucoidan in the sample of seawater is precipitated, wherein the sample of seawater is diluted with ultrapure water, comprising:
(I) adding metal ions, alcohols, low pH acids, or an amphiphilic compound to the sample of seawater,
(II) a filtration step, wherein the filtration step comprises filtrating the sample of seawater through a filter, e.g. a combusted glass fiber filter,
(III) an elution or resuspension step, wherein the precipitated fucoidan is eluted or resuspended from the filter.

9. The method of any one of claims 1-8, further comprising step (iii) quantifying fucoidan in the sample.

10. The method of claim 9, wherein the quantifying step comprises processing of fucoidan into monosaccharides.

11. A method for quantifying fucoidan, comprising:
(i) obtaining a sample of seawater, and
(ii) quantifying fucoidan in the sample.

12. The method of any one of claims 7, 8 or 11, comprising contacting a sample with enzymes capable of digesting fucoidan, e.g. fucoidanases, sulfatases and/or fucosidases.

13. The method of claim 11 or 12, further comprising
(a) contacting a sample comprising a known amount/quantity of fucoidan (fucoidan standard/reference sample) with enzymes capable of digesting fucoidan,
(b) quantifying monosaccharides obtained in (a), and
(c) generating a fucoidan standard curve by correlating the yield of monosaccharides in (b) with the known amount/quantity of fucoidan in the sample of (a).

14. The method of claim 12 or 13, comprising comparing the yield of monosaccharides, such as fucose, obtained from the fucoidan digestion in the sample of seawater with a fucoidan standard curve, thereby quantifying the amount of fucoidan in the sample of seawater.

15. A composition comprising fucoidan produced by the method of any one of claims 1-8.
